# EUROPEAN PATENT SPECIFICATION

(11) **EP 1 991 542 B1**
(45) Date of publication and mention of the grant of the patent: **16.03.2011**
(21) Application number: 07704963.3
(22) Date of filing: 22.01.2007
(51) Int. Cl.: C07D 413/12, A01N 43/80, C07D 261/04

(54) **HERBICIDAL ISOXAZOLINE COMPOUNDS**
HERBIZIDE ISOXAZOLINVERBINDUNGEN
ISOXAZOLINES HERBICIDES

(30) Priority: 27.02.2006 GB 0603891
(43) Date of publication of application: 19.11.2008
(73) Proprietor: Syngenta Limited, Guildford Surrey GU2 7YH (GB)
(72) Inventor: BOEHMER, Jutta, Elisabeth, Berkshire RG42 6EY (GB); MCLACHLAN, Matthew, Murdoch, Woodhead, Berkshire RG42 6EY (GB)
(74) Representative: Osborn, Martin Keith
(86) International application number: PCT/GB2007/000184
(87) International publication number: WO 2007/096576

(56) References cited:
- EP-A1- 1 203 768
- EP-A1- 1 364 946
- EP-A1- 1 405 853
- WO-A-2005/104848
- WO-A-2006/024820
- JP-A- 2005 035 924
- US-A1- 2005 256 004

## Description

The present invention relates to novel, herbicidal isoxazoline compounds, to processes for their preparation, to compositions comprising those compounds, and to their use in controlling plants or in inhibiting plant growth.

Isoxazoline compounds which display a herbicidal action are described, for example, in WO 01/012613, WO 02/062770, WO 03/000686, WO 04/on0165, JP 20051035924, JP 2005/213168 and WO 06/024820. The preparation of these compounds is also described in WO 04/013106 and WO 05/095352.

Novel isoxazoline compound which display herbicidal and growth-inhibiting properties have now been found.

The present invention accordingly relates to compounds of formula I 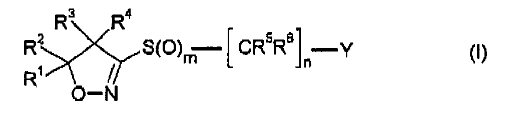 wherein
R¹ and R² are each independently of the other hydrogen, C₁-C₁₀alkyl, C₁-C₁₀haloalkyl, C₃-C₈cycloalkyl or C₃-C₈cycloalkyl-C₁-C₃alkyl, or
R¹ and R² together with the carbon atom to which they are bonded form a C₃-C₇riuag,
R³ and R⁴ are each independently of the other hydrogen, C₁-C₁₀alkyl, C₁-C₁₀haloalkyl, C₃-C₈cycloalkyl, C₃-C₈cycloalkyl-C₁-C₁₀alkyl or C₁-C₆alkoxy-C₁-C₁₀alkyl, or
R³ and R⁴ together with the carbon atom to which they are bonded form a C₃-C₇ring, or
R¹ with R³ or R⁴ and together with the carbon atoms to which they are bonded form a C₅-C₈ring, or
R² with R² or R⁴ and together with the carbon atoms to which they are bonded form a C₅-C₈ring;
R⁵ is halogen;
R⁶ is halogen;
m is 0, I or 2;
n is 1, 2 or 3; and
Y is one of the following groups 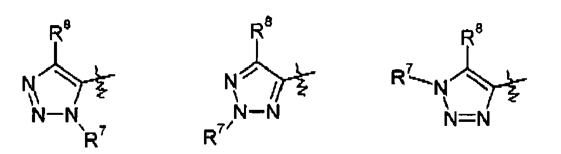 wherein
R⁷ is hydrogen, C₁-C₁₀alkyl, C₁-C₁₀alkylcarbonyl, formyl, C₁-C₁₀haloalkylcarbonyl, C₁-C₁₀alkoxycarbonyl, C₁-C₁₀haloalkyl, cyano, C₂-C₁₀alkenyl, C₂-C₁₀alkynyl, C₃-C₁₀cycloalkyl, C₃-C₁₀cycloalkyl-C₁-C₁₀alkyl, C₁-C₁₀alkylcarbonyl-C₁-C₁₀alkyl, C₁-C₁₀alkylsulfonyl, C₁-C₁₀haloalkylsulfonyl, C₁-C₁₀alkoxy-C₁-C₁₀alkyl or phenyl which is optionally substituted by one to five substituents selected from cyano, C₁-C₁₀alkyl, C₁-C₁₀haloalkyl or halogen; or
R⁷ is -CONR¹³R¹⁴ or -SO₂NR¹³R¹⁴ wherein R¹³ and R¹⁴ are independently of each other hydrogen, C₁-C₆alkyl, C₁-C₆haloalkyl, C₁-C₆cycloalkyl, C₁-C₆alkylcarbonyl, C₁-C₆halo-alkylcarbonyl, C₁-C₆alkylsulfonyl, C₁-C₆haloalkylsulfonyl, or R¹³ and R¹⁴ together form a C₃-C₈alkylene group which optionally contains one oxygen, sulfur, amino or C₁-C₆alkylamino group;
R⁸ is hydrogen, C₁-C₁₀alkyl, C₁-C₁₀alkylcarbonyl, formyl, C₁-C₁₀haloalkylcarbonyl, C₁-C₁₀alkoxycarbonyl, C₁-C₁₀haloalkyl, C₂-C₁₀alkenyl, C₂-C₁₀alkynyl, C₃-C₁₀cycloalkyl, C₃-C₁₀cycloalkyl-C₁-C₁₀alkyl, halogen, cyano, C₁-C₁₀alkoxy or C₁-C₁₀haloalkoxy, or
R⁸ is -CONR¹⁵R¹⁶, -SO₂NR¹⁵R¹⁶ or -NR¹⁵R¹⁶ wherein R¹⁵ and R¹⁶ are independently of each other hydrogen, C₁-C₆alkyl, C₁-C₆haloalkyl, C₁-C₆cycloalkyl, C₁-C₆alkylcarbonyl, C₁-C₆haloalkylcarbonyl, C₁-C₆alkylsulfonyl, C₁-C₆haloalkylsulfonyl, or R¹⁵ and R¹⁶ together form a C₃-C₈alkylene group which optionally contains one oxygen, sulfur, amino or C₁-C₆alkylamino group;
and to N-oxides, salts and optical isomers of compounds of formula I.

Preferably R¹ and R² are independently C₁-C₁₀alkyl or C₁-C₁₀haloalkyl, more preferably C₁-C₆alkyl or C₁-C₆haloalkyl, most preferably methyl.

Preferably R³ and R⁴ are independently hydrogen, C₁-C₁₀alkyl or C₁-C₁₀haloalkyl, more preferably hydrogen, C₁-C₆alkyl or C₁-C₆haloalkyl, most preferably hydrogen.

Preferably R⁵ is fluoro or chloro.

Preferably R⁶ is fluoro or chloro, most preferably fluoro. Preferably m is 1 or 2, more preferably 2.

Preferably n is 1.

Preferably Y is one of the following groups

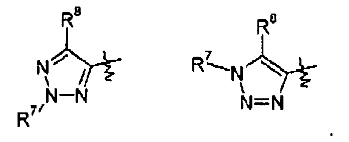

Preferably R⁷ is hydrogen, C₁-C₁₀alkyl, C₁-C₁₀haloalkyl, C₂-C₁₀alkenyl, C₂-C₁₀alkynyl, C₃-C₁₀cycloalkyl, C₃-C₁₀cycloalkyl-C₁-C₁₀alkyl, C₁-C₁₀alkoxy-C₁-C₁₀alkyl or phenyl which is optionally substituted by one to five substituents selected from cyano, C₁-C₁₀alkyl, C₁-C₁₀haloalkyl or halogen, more preferably hydrogen, methyl, ethyl, *iso-*propyl, *tert-*butyl, monofluoromethyl, difluoromethyl, trifluoromethyl, allyl, propargyl, cyclopropyl, cyclopentyl, cyclopropylmethyl, cyclobutylmethyl. methoxymethyl, 2-methoxy-ethyl or phenyl, even more preferably methyl, ethyl, *iso-*propyl, *tert-*butyl, difluoromethyl, allyl, cyclopentyl, cyclobutylmethyl, 2-methoxy-ethyl or phenyl, most preferably methyl, ethyl, ico-propyl, *tert-*butyl, difluoromethyl, allyl, cyclopentyl, cyclobutylmethyl or 2-methoxy-ethyl.

Preferably R⁸ is hydrogen, C₁-C₁₀alkyl, C₁-C₁₀alkylcarbonyl, formyl, C₁-C₁₀alkoxycarbonyl, C₁-C₁₀haloalkyl, C₃-C₁₀cycloalkyl, halogen, cyano, C₁-C₁₀alkoxy or C₁-C₁₀haloalkoxy, more preferably hydrogen, methyl, ethyl, acetyl, formyl, methoxycarbonyl, monotluoromethyl. difluoromethyl, trifluoromethyl, bromodifluoromethyl, 1-fluoroethyl, cyclopropyl, fluoro, chloro, bromo, cyano, methoxy, difluoromethoxy, trifluoromethoxy or 2,2,2-trifluoroethoxy, even more preferably hydrogen, methyl, ethyl, acetyl, methoxycarbonyl, monofluoromethyl, difluoromethyl, trifluoromethyl, bromodifluoromethyl, 1-fluoro-ethyl, cyclopropyl, bromo, methoxy or difluoromethoxy, most preferably hydrogen, methyl, ethyl, monofluoromethyl, trifluoromethyl, bromo or methoxy.

A group of preferred compounds of formula I comprises those wherein Y is 1,2,3-triazolyl which is optionally substituted by one to two substituents independently selected from C₁-C₆alkyl, C₁-C₆haloalkyl, C₁-C₆alkylcarbonyl, formyl, C₁-C₆alkoxycarbonyl, C₂-C₆alkenyl, C₁-C₆cycloalkyl, C₃-C₆cycloalkyl-C₁-C₆-alkyl, C₁-C₆alkoxy-C₁-C₆alkyl, halogen, cyano, C₁-C₆alkoxy, C₁-C₆haloalkoxy or phenyl which is optionally substituted by one to five substituents selected from cyano, C₁-C₁₀alkyl, C₁-C₁₀haloalkyl or halogen, more preferably wherein Y is 1,2,3-triazolyl which is optionally substituted by one to two substituents independently selected from methyl, ethyl, *iso*-propyl, *tert*-butyl, monofluoromethyl, difluoromethyl, trifluoromethyl, bromodifluoromethyl, 1-fluoro-ethyl, acetyl, formyl, methoxycarbonyl, allyl, cyclopropyl, cyclopentyl, cyclopropylmethyl, cyclobutylmethyl, 2-inethoxy-ethyl, fluoro, chloro, bromo, cyano, methoxy, ethoxy, monofluoromethoxy, difluoromethoxy, trifluoromethoxy or phenyl, even more preferably wherein Y is 1,2,3-triazolyl which is optionally substituted by one to two substituents independently selected from methyl, ethyl, *iso-*propyl, *tert-*butyl, monofluoromethyl, difluoromethyl, trifluoromethyl, bromodifluoromethyl, 1-fluoro-ethyl, acetyl, methoxycarbonyl, allyl, cyclopropyl, cyclopentyl, cyclobutylmethyl, 2-methoxy-ethyl, bromo, methoxy, difluoromethoxy or phenyl, most preferably wherein Y is 1,2,3-triazolyl which is optionally substituted by one to two substituents independently selected from methyl, ethyl, *iso*-propyl, *tert*-butyl, monofluoromethyl, difluoromethyl, trifluoromethyl, allyl, cyclopentyl, cyclobutylmethyl, 2-methoxy-ethyl or bromo.

A group of especially preferred compounds of formula I comprises those wherein Y is 1,2,3-triazol-4-yl which is optionally substituted by one to two substituents independently selected from C₁-C₆alkyl, C₁-C₆haloalkyl, C₁-C₆alkylcarbonyl, formyl, C₁-C₆alkoxycarbonyl, C₂-C₆alkenyl, C₃-C₆cycloalkyl, C₃-C₆cycloalkyl-C₁-C₆-alkyl, C₁-C₆alkoxy-C₁-C₆alkyl, halogen, cyano, C₁-C₆alkoxy, C₁-C₆haloalkoxy or phenyl which is optionally substituted by one to five substituents selected from cyano, C₁-C₁₀alkyl, C₁-C₁₀haloalkyl or halogen, more preferably wherein Y is 1,2,3-triazol-4-yl which is optionally substituted by one to two substituents independently selected from methyl, ethyl, *iso-*propyl, *tert-*butyl, monofluoromethyl, difluoromethyl, trifluoromethyl, bromodifluoromethyl, 1-fluoro-ethyl, acetyl, formyl, methoxycarbonyl, allyl, cyclopropyl, cyclopentyl, cyclopropylmethyl, cyclobutylmethyl, 2-methoxy-ethyl, fluoro, chloro, bromo, cyano, methoxy, ethoxy, monofluoromethoxy, difluoromethoxy, trifluoromethoxy or phenyl, even more preferably wherein Y is 1,2,3-triazol-4-yl which is optionally substituted by one to two substituents independently selected from methyl, ethyl, *iso*-propyl, *tert*-butyl, monofluoromethyl, difluoromethyl, trifluoromethyl, bromodifluoromethyl, 1-fluoro-ethyl, acetyl, methoxycarbonyl, allyl, cyclopropyl, cyclopentyl, cyclobutylmethyl, 2-methoxy-ethyl, bromo, methoxy, difluoromethoxy or phenyl, most preferably wherein Y is 1,2,3-triazol-4-yl which is optionally substituted by one to two substituents independently selected from methyl, ethyl, *iso-*propyl, *tert-*butyl, monofluoromethyl, difluoromethyl, trifluoromethyl, allyl, cyclopentyl, cyclobutylmethyl, 2-methoxy-ethyl or bromo.

A group of especially preferred compounds of formula I comprises those wherein Y is

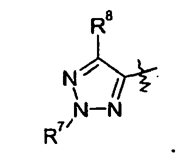

A group of especially preferred compounds of formula I comprises those wherein Y is 5-monofluoromethyl-2-methyl-1,2,3-triazol-4-yl.

A group of especially preferred compounds of formula I comprises those wherein Y is 2,5-dimethyl-1,2,3-triazol-4-yl.

A group of especially preferred compounds of formula I comprises those wherein Y is 5-cycloproyl-2-methyl-1,2,3-triazol-4-yl.

A group of especially preferred compounds of formula I comprises those wherein Y is 2,5-diethyl-l,2,3-triazol-4-yl.

A group of especially preferred compounds of formula I comprises those wherein Y is 2-difluoiomethyl-5-methyl-1,2,3-triazol-4-yl.

A group of especially preferred compounds of formula I comprises those wherein Y is 2-allyl-5-trifluoromethyl-1,2,3-triazol-4-yl.

A group of especially preferred compounds of formula I comprises those wherein Y is 2-cyclopentyl-5-trifluoromethyl-1,2,3-triazol-4-yl.

A group of especially preferred compounds of formula I comprises those wherein Y is 2-cyclobutylmethyl-5-trifluoromethyl-1,2,3-triazol-4-yl.

A group of especially preferred compounds of formula I comprises those wherein Y is 2-(2-methoxy-ethyl)-5-trifluoromethyl-1,2,3-triazol-4-yl.

A group of especially preferred compounds of formula I comprises those wherein Y is 2-methyl-1,2,3-triazol-4-yl.

A group of especially preferred compounds of formula I comprises those wherein Y is 2-*iso-*propyl-5-trifluoromethyl- 1 ,2,3-triazol.4.yl.

A group of especially preferred compounds of formula I comprises those wherein Y is 2-ethyl-5-trifluoromethyl-1,2,3-triazol-4-yl.

A group of especially preferred compounds of formula I comprises those wherein Y is 5-ethyl-2-methyl-1,2,3-triazol-4-yl.

A group of especially preferred compounds of formula I comprises those wherein Y is 2-ethyl-1,2,3-triazol-4-yl.

A group of especially preferred compounds of formula I comprises those wherein Y is 2-*iso-*propyl-5-methyl-1,2,3-triazol-4-yl.

A group of especially preferred compounds of formula I comprises those wherein Y is 2-methyl-5-trifluoromethyl-1,2,3-triazol-4-yl.

A group of especially preferred compounds of formula I comprises those wherein Y is 5-methoxy-2-methyl-1,2,3-triazol-4-yl.

A group of especially preferred compounds of formula I comprises those wherein Y is 5-bromo-2-methyl-1,2,3-triazol-4-yl.

A group of especially preferred compounds of formula I comprises those wherein Y is 2-ethyl-5-methyl-1,2,3-triazol-4-yl.

A group of especially preferred compounds of formula I comprises those wherein Y is 5-difluoromethoxy-2-methyl-1,2,3-triazol-4-yl.

A group of especially preferred compounds of formula I comprises those wherein Y is S-methoxycarbonyl-2-methyl-1,2,3-triazol-4-yl.

A group of especially preferred compounds of formula I comprises those wherein Y is 5-acetyl-2-methyl-1,2,3-triazol-4-yl.

A group of especially preferred compounds of formula I comprises those wherein Y is 5-bromodifluoromethyl-2-methyl-1,2,3-triazol-4-yl..

A group of especially preferred compounds of formula I comprises those wherein Y is 5-difluoromethyl-2-methyl-1,2,3-triazol-4-yl.

A group of especially preferred compounds of formula I comprises those wherein Y is 5-(1-fluoro-ethyl)-2-methyl-1,2,3-triazol-4-yl.

A group of especially preferred compounds of formula I comprises those wherein Y is 5-methyl-2-phenyl-1,2,3-triazol-4-yl.

A group of especially preferred compounds of formula I comprises those wherein Y is

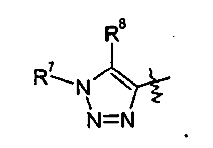

A group of especially preferred compounds of formula I comprises those wherein Y is 1-*tert-*butyl-1,2,3-triazol-4-yl.

A group of especially preferred compounds of formula I comprises those wherein Y is 1,5-dimethyl-1,2,3-triazol-4-yl.

A group of especially preferred compounds of formula I comprises those wherein Y is 5-cyclopropyl-1-methyl-1,2,3-triazol-4-yl.

A group of especially preferred compounds of formula I comprises those wherein Y is 1,2,3-triazol-5-yl which is optionally substituted by one to two substituents independently selected from C₁-C₆alkyl, C₁-C₆haloalkyl, C₁-C₆alkylcarbonyl, formyl, C₁-C₆alkoxycarbonyl, C₂-C₆alkenyl, C₃-C₆cycloalkyl, C₃-C₆cycloalkyl-C₁-C₆-alkyl, C₁-C₆alkoxy-C₁-C₆alkyl, halogen, cyano, C₁-C₆alkoxy, C₁-C₆haloalkoxy or phenyl which is optionally substituted by one to five substituents selected from cyano, C₁-C₁₀alkyl, C₁-C₁₀haloalkyl or halogen, more preferably wherein Y is 1,2,3-triazol-5-yl which is optionally substituted by one to two substituents independently selected from methyl, ethyl, *iso-*propyl, *tert-*butyl, monofluoromethyl, difluoromethyl, trifluoromethyl, bromodifluoromethyl, 1-fluoro-ethyl, acetyl, formyl, methoxycarbonyl, allyl, cyclopropyl, cyclopentyl, cyclopropylmethyl, cyclobutylmethyl, 2-methoxy-ethyl, fluoro, chloro, bromo, cyano, methoxy, ethoxy, monofluoromethoxy, difluoromethoxy, trifluoromethoxy or phenyl, even more preferably wherein Y is 1,2,3-triazol-5-yl which is optionally substituted by one to two substituents independently selected from methyl, *iso-*propyl, trifluoromethyl, cyclopropyl or bromo, most preferably wherein Y is 1,2,3-triazol-5-yl which is optionally substituted by one to two substituents independently selected from methyl, trifluoromethyl or bromo.

A group of especially preferred compounds of formula I comprises those wherein Y is

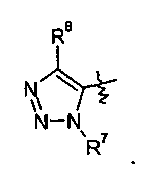

A group of especially preferred compounds of formula I comprises those wherein Y is 4-bromo-1-methyl-1,2,3-triazol-5-yl.

A group of especially preferred compounds of formula I comprises those wherein Y is 4-methyl-1-*iso-*propyl-1,2,3-triazol-5-yl.

A group of especially preferred compounds of formula I comprises those wherein Y is 1-methyl-4-trifluoromethyl-1,2,3-triazol-5-yl.

A group of especially preferred compounds of formula I comprises those wherein Y is 4-cyclopropyl-1-methyl-1,2,3-triazol-5-yl.

A group of especially preferred compounds of formula I comprises those wherein Y is 1,2,3-triazol-4-yl-1-oxide which is optionally substituted by one to two substituents independently selected from C₁-C₆alkyl, C₁-C₆haloalkyl, C₁-C₆alkylcarbonyl, formyl, C₁-C₆alkoxycarbonyl, C₂-C₆alkenyl, C₃-C₆cycloalkyl, C₃-C₆cycloalkyl-C₁-C₆-alkyl, C₁-C₆alkoxy-C₁-C₆alkyl, halogen, cyano, C₁-C₆alkoxy, C₁-C₆haloalkoxy or phenyl which is optionally substituted by one to five substituents selected from cyano, C₁-C₁₀alkyl, C₁-C₁₀haloalkyl or halogen, more preferably wherein Y is 1,2,3-triazol-4-yl-1-oxide which is optionally substituted by one to two substituents independently selected from methyl, ethyl, *iso-*propyl, *tert-*butyl, monofluoromethyl, difluoromethyl, trifluoromethyl, bromodifluoromethyl, 1-fluoro-ethyl, acetyl, formyl, methoxycarbonyl, allyl, cyclopropyl, cyclopentyl, cyclopropylmethyl, cyclobutylmethyl, 2-methoxy-ethyl, fluoro, chloro, bromo, cyano, methoxy, ethoxy, monofluoromethoxy, difluoromethoxy, trifluoromethoxy or phenyl, most preferably wherein Y is 1,2,3-triazol-4-yl-1-oxide which is optionally substituted by one or two methyl groups.

A group of especially preferred compounds of formula I comprises those wherein Y is

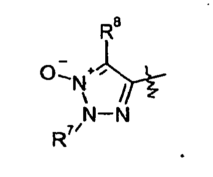

A group of especially preferred compounds of formula I comprises those wherein Y is 2,5-dimethyl-1.,2,3-triazol-4-yl-1-oxide.

In one embodiment the invention relates to a compound of formula I wherein
R¹ and R² are each independently of the other hydrogen, C₁-C₁₀alkyl, C₁-C₁₀haloalkyl, C₃-C₈cycloalkyl or C₃-C₈cycloalkyl-C₁-C₃alkyl, or
R¹ and R² together with the carbon atom to which they are bonded form a C₃-C₇ring,
R³ and R⁴ are each independently of the other hydrogen, C₁-C₁₀alkyl, C₁-C₁₀haloalkyl, C₃-C₈cycloalkyl, C₃-C₈cycloalkyl-C₁-C₁₀alkyl or C₁-C₆alkoxy-C₁-C₁₀alkyl, or
R³ and R⁴ together with the carbon atom to which they are bonded form a C₃-C₇ring, or
R¹ with R³ or R⁴ and together with the carbon atoms to which they are bonded form a C₅-C₈ring, or
R² with R³ or R⁴ and together with the carbon atoms to which they are bonded form a C₅-C₈ring;
R⁵ is halogen;
R⁶ is halogen;
m is 0, 1 or 2;
n is 1, 2 or 3; and
Y is one of the following groups 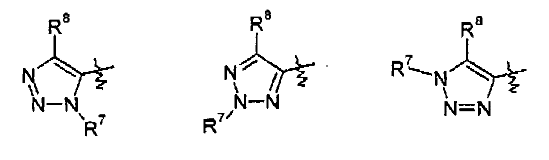 wherein
R⁷ is hydrogen, C₁-C₁₀alkyl, C₁-C₁₀alkylcarbonyl, C₁-C₁₀haloalkylcarbonyl, C₁-C₁₀alkoxycarbonyl, C₁-C₁₀haloalkyl, C₂-C₁₀alkenyl, C₂-C₁₀alkynyl, C₃-C₁₀cycloalkyl, C₃-C₁₀cycloalkyl-C₁-C₁₀alkyl, C₁-C₁₀alkylcarbonyl-C₁-C₁₀alkyl, C₁-C₁₀alkylsulfonyl, C₁-C₁₀haloalkylsulfonyl or C₁-C₁₀alkoxy-C₁-C₁₀alkyl;
R⁸ is hydrogen, C₁-C₁₀alkyl, C₁-C₁₀alkylcarbonyl, C₁-C₁₀haloalkylcarbonyl, C₁-C₁₀alkoxycarbonyl, C₁-C₁₀haloalkyl, C₂-C₁₀alkenyl, C₂-C₁₀alkynyl, C₃-C₁₀cycloalkyl, C₃-C₁₀cycloalkyl-C₁-C₁₀alkyl, halogen, C₁-C₁₀alkoxy or C₁-C₁₀haloalkoxy;
and to N-oxides, salts and optical isomers of compounds of formula I.

Preferably R⁷ is hydrogen, C₁-C₁₀alkyl, C₁-C₁₀haloalkyl, C₂-C₁₀alkenyl, C₂-C₁₀alkynyl, C₃-C₁₀cycloalkyl, C₃-C₁₀cycloalkyl-C₁-C₁₀alkyl or C₁-C₁₀alkoxy-C₁-C₁₀alkyl, more preferably hydrogen, methyl, ethyl, allyl, propargyl, cyclopropyl, cyclopropylmethyl, methoxymethyl, methoxymethyl, difluoromethyl or trifluoromethyl, most preferably methyl.

Preferably R⁸ is hydrogen, C₁-C₁₀alkyl, C₁-C₁₀haloalkyl, C₃-C₁₀cycloalkyl, halogen, C₁-C₁₀alkoxy or C₁-C₁₀haloalkoxy, more preferably hydrogen, methyl, ethyl, trifluoromethyl, difluoromethyl, monofluoromethyl, cyclopropyl, fluoro, chloro, trifluoromethoxy or 2,2,2-trifluoroethoxy, most preferably methyl, trifluoromethyl or monofluoromethyl.

The compounds of the invention may contain one or more asymmetric carbon atoms, for example, in the -CR⁵R⁶- group or in the -CR³R⁴-group and may exist as enantiomers (or as pairs of diastereomers) or as mixtures of such. Further, when m is 1, the compounds of the invention are sulfoxides, which can exists in two enantiomeric forms, the adjacent carbon can also exists in two enantiomeric forms and the -CR³R⁴-group can also exist in two enantiomeric forms. Compounds of general formula I can therefore exist as racemates, diastereoisomers, or single enantiomers, and the invention includes all possible isomers or isomer mixtures in all proportions. It is to be expected that for any given compound, one isomer may be more herbicidal than another.

Alkyl groups, haloalkyl groups, hydroxyalkyl groups, alkoxy groups, haloalkoxy groups and alkylene groups can be straight or branched chain. Preferred alkyl groups, haloalkyl groups, hydroxyalkyl groups, alkoxy groups, haloalkoxy groups and alkylene groups each independently contain 1 to 4 carbons. Examples of alkyl groups are methyl, ethyl, *n-*and *iso-*propyl and *n-, sec-, iso-* and *tert-*butyl, hexyl, nonyl and decyl. Examples of haloalkyl groups are difluoromethyl and 2,2,2-trifluoroethyl. Examples of hydroxyalkyl groups are 1,2-dihydroxyethyl and 3-hydroxypropyl. Examples of alkoxy groups are methoxy, ethoxy, propoxy, butoxy, hexyloxy, nonyloxy and decyloxy. Examples of haloalkoxy groups are difluoromethoxy and 2,2,2-trifluoroethoxy. Examples of alkylene groups are methylene, ethylene, *n-*and *iso-*propylene and *n-, sec-, iso-* and *tert-*butylehe.

Cycloalkyl groups can be in mono-, bi- or tri-cyclic form. Preferred cycloalkyl groups independently contain 3 to 8 carbons. Examples of monocyclic cycloalkyl groups are cyclopropyl, cyclobutyl, cyclopentyl, cyclohexyl, cycloheptyl and cyclooctyl.

Alkenyl and alkynyl groups and haloalkenyl groups and haloalkynyl groups can be straight or branched chain. Examples of alkenyl and alkynyl groups are allyl, but-2-enyl, 3-methylbut-2-enyl, ethynyl, propargyl and but-2-ynyl. Examples of haloalkenyl and haloalkynyl groups are trifluoroallyl and 1-chloroprop-1-yn-3-yl.

Halogen means fluoro, chloro, bromo and iodo, preferably fluoro, chloro or bromo, more preferably fluoro or chloro.

The invention relates likewise to the salts which the compounds of formula I are able to form with amines, alkali metal and alkaline earth metal bases and quartemary ammonium bases.

Among the alkali metal and alkaline earth metal hydroxides as salt formers, special mention should be made of the hydroxides of lithium, sodium, potassium, magnesium and calcium, but especially the hydroxides of sodium and potassium. The compounds of formula I according to the invention also include hydrates which may be formed during the salt formation.

Examples of amines suitable for ammonium salt formation include ammonia as well as primary, secondary and tertiary C₁-C₁₈alkylamines, C₁-C₄hydroxyalkylamines and C₂-C₄alkoxyalkylamines, for example methylamine, ethylamine, *n-*propylamine, isopropylamine, the four butylamine isomers, *n-*amylamine, isoamylamine, hexylamine, heptylamine, octylamine, nonylamine, decylamine, pentadecylamine, hexadecylamine, heptadecylamine, octadecylamine, methylethylamine, methylisopropylanfne, methylhexylamine, methylnonylamine, methylpentadecylamine, methyloctadecylamine, ethylbutylamine, ethylheptylamine, ethyloctylamine, hexylheptylamine, hexyloctylamine, dimethylamine, diethylamine, di-*n*-propylamine, diisopropylamine, di*n*-butylamine, di-*n*-amylamine, diisoamylamine, dihexylamine, diheptylamine, dioctylamine, ethanolamine, ll-propanolamine, isopropanolamine, N,N-diethanolamine, N-ethylpropanolamine, N-butylethanolamine, allylamine, *n-*butenyl-2-amine, *n-*pentenyl-2-amine, 2,3-dimethylbutenyl-2-amine, dibutenyl-2-amine, *n-*hexenyl-2-amine, propylenediamine, trimethylamine, triethylamine, tri-*n-*propylamine, triisopropylamine, tri-*n*-butylamine, triisobutylamine, tri-*sec-*butylamine, tri-*n-*amylamine, methoxyethylamine and ethoxyethylamine; heterocyclic amines such as, for example, pyridine, quinoline, isoquinoline, morpholine, piperidine, pyrrolidine, indoline, quinuclidine and azepine; primary arylamines such as, for example, anilines, methoxyanilines, ethoxyanilines, o-, m- and p-toluidines, phenylenediamines, benzidines, naphthylamines and o-, m- and p-chloroanilines; but especially triethylamine, isopropylamine and diisopropylamine.

Preferred quarternary ammonium bases suitable for salt formation correspond, for example, to the formula [N(Rₐ R_{b}R_{c}R_{d} )]OH wherein Rₐ, R_{b}, R_{c} and R_{d} are each independently of the others C₁-C₄alkyl. Other suitable tetraalkylammonium bases with other anions can be obtained, for example, by anion exchange reactions.

The term "herbicide" as used herein means a compound that controls or modifies the growth of plants. The term "herbicidally effective amount" means the quantity of such a compound or combination of such compounds that is capable of producing a controlling or modifying effect on the growth of plants. Controlling or modifying effects include all deviation from natural development, for example: killing, retardation, leaf bum, albinism, dwarfing and the like. The term "plants" refers to all physical parts of a plant, including seeds, seedlings, saplings, roots, tubers, stems, stalks, foliage, and fruits. The term "locus" is intended to include soil, seeds, and seedlings, as well as established vegetation.

The compounds in Table A below illustrate the compounds of the invention.

**Table A: Compounds of formula L1**

| | | | | |
|---|---|---|---|---|
| 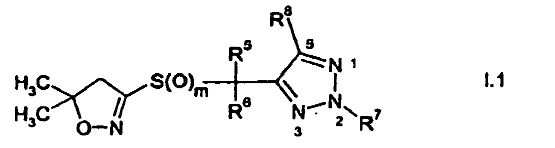 | | | | |

| **m** | **R⁵** | **R⁶** | **R⁷** | **R⁸** |
|---|---|---|---|---|
| 2 | F | Cl | Me | OMe |
| 2 | F | F | Me | CF₃ |
| 1 | F | F | Me | OCHF₂ |
| 2 | F | F | Et | COMe |
| 1 | F | Cl | Et | CH₂F |
| 2 | F | Cl | Et | CHFMe |
| 1 | F | F | Me | CHFMe |
| 1 | F | F | Et | OCHF₂ |
| 1 | F | Cl | Me | CH₂F |
| 2 | F | F | Et | ^{c}Pr |
| 1 | F | Cl | Me | Me |
| 2 | F | Cl | Me | CH₂F |
| 2 | F | Cl | Me | ^{c}Pr |
| 1 | F | Cl | Et | OCHF₂ |
| 1 | F | F | Et | CHFMe |
| 2 | F | Cl | Et | CF₃ |
| 2 | F | Cl | Et | CH₂F |
| 2 | F | Cl | Et | ^{c}Pr |
| 1 | F | Cl | Et | Et |
| 2 | F | F | Me | Me |
| 1 | F | Cl | Me | CHFMe |
| 2 | F | Cl | Me | Et |
| 1 | F | Cl | Et | ^{c}Pr |
| 2 | F | Cl | Me | CF₃ |
| 2 | F | Cl | Me | CHF₂ |
| 2 | F | F | Et | OCHF₂ |
| 1 | F | Cl | Et | OMe |
| 1 | F | F | Et | Et |
| 2 | F | F | Et | CHF₂ |
| 1 | F | F | Et | CH₂F |
| 1 | F | F | Et | OMe |
| 1 | F | Cl | Me | CHF₂ |
| 2 | F | F | Me | OMe |
| 1 | F | F | Me | Me |
| 1 | F | F | Me | COMe |
| 2 | F | F | Me | ^{c}Pr |
| 1 | F | Cl | Et | CHF₂ |
| 2 | F | F | Me | CH₂F |
| 2 | F | F | Et | Me |
| 1 | F | F | Me | ^{c}Pr |
| 2 | F | Cl | Et | Et |
| 1 | F | F | Me | Et |
| 1 | F | F | Et | COMe |
| 2 | F | F | Et | CH₂F |
| 2 | F | Cl | Et | CHF₂ |
| 2 | F | Cl | Et | COMe |
| 2 | F | Cl | Me | COMe |
| 1 | F | F | Me | CH₂F |
| 1 | F | F | Et | CHF₂ |
| 2 | F | F | Me | Et |
| 2 | F | F | Me | CHF₂ |
| 1 | F | F | Et | CF₃ |
| 1 | F | Cl | Me | COMe |
| 2 | F | Cl | Et | OCHF₂ |
| 2 | F | F | Me | OCHF₂ |
| 1 | F | F | Me | CHF₂ |
| 2 | F | Cl | Et | Me |
| 1 | F | Cl | Me | OCHF₂ |
| 1 | F | Cl | Me | OMe |
| 2 | F | F | Et | Et |
| 1 | F | Cl | Me | ^{c}Pr |
| 2 | F | F | Et | CF₃ |
| 2 | F | Cl | Et | OMe |
| 1 | F | Cl | Et | CHFMe |
| 1 | F | Cl | Me | Et |
| 2 | F | F | Et | OMe |
| 1 | F | Cl | Et | Me |
| 1 | F | F | Me | OMe |
| 2 | F | Cl | Me | OCHF₂ |
| 1 | F | F | Et | ^{c}Pr |
| 1 | F | F | Et | Me |
| 2 | F | Cl | Me | Me |
| 2 | F | Cl | Me | CHFMe |
| 1 | F | F | Me | CF₃ |
| 2 | F | F | Et | CHFMe |
| 1 | F | Cl | Me | CF₃ |
| 1 | F | Cl | Et | COMe |
| 2 | F | F | Me | CHFMe |
| 2 | F | F | Me | COMe |
| 1 | F | Cl | Et | CF₃ |

The compounds of the invention may be made by a variety of methods.

### Methods of halogenation, alkylation and oxidation

1) The compounds of formula I wherein R¹, R², R³, R⁴, R⁵, R⁶ and Y are as defied above, m is 1 or 2, and n is 1, can be prepared by processes known *per se,* by reacting e.g. the compounds of formula Ia 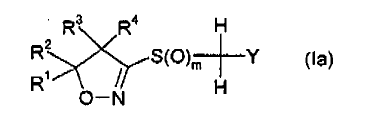 wherein R¹, R², R³, R⁴ and Y are as defined above and m is 1 or 2, in a single step or stepwise in succession with compounds of the formula R⁵-X and/or R⁶-X, wherein R⁵ is halogen and R⁶ is halogen and X is a suitable leaving group e.g. halide, such as bromide or iodide, a carboxylate, such as acetate, an alkylsulfonate, such as methylsulfonate, an arylsulfonate, such as p-toluenesulfonate, a haloalkylsulfonate, such as trifluoromethylsulfonate, an imide, such as succinimide, a sulfonimide, such as bis(phenylsulfonyl)-imide, or an arylsulfinate, such as p-toluenesulfinate, in the presence of a base, e.g. an alkyl-lithium compound, such as methyl-lithium, *n-*butyl-lithium or *tert-*butyl-lithium, a lithium dialkylamide, such as lithium diisopropylamide, a metal hydride, preferably an alkali metal hydride, such as sodium hydride, or an alkali metal amide, such as sodium amide, a metal bis(tri(C₁-C₆alkyl)silyl)amide, such as lithium bis(trimethylsilyl)amide, a metal alkoxide, such as potassium *tert-*butoxide, or a phosphazene base, such as N'-*tert-*butyl-N,N,N',N',N",N"-hexamethylphosphorimidic triamide (P₁-t-Bu), 1-*tert-*butyl-2,2,4,4,4-pentakis(dimethylamino)-2-lambda⁵,4lambda⁵-catenadi(phosphazene) (P₂-*t-*Bu), 1-ethyl-2,2,4,4,4.pentakis(dimethylarnino)-2-lambda⁵,4lambda⁵. catenadi(phosphazene) (P₂-Et) and 2-*tert-*butylimino-2-diethylamino-1,3-dimethyl-perhydro-1,3,2-diazaphosphorine (BEMP), optionally in the presence of a diluent, preferably an inert solvent, e.g. a hydrocarbon, an ether, such as tetrahydrofuran, an amide, such as N,N-dimethylformamide, or a halogenated hydrocarbon, such as dichloromethane, or mixtures thereof and optionally in the presence of a complexing agent, such as hexamethylphosphoramide or tetramethylethylenediamine in a temperature range of from -120°C to 100°C, preferably from -80°C to 50°C. Such processes are known in the literature and are described, for example, in J. Med. Chem., 2003 (46) 3021-3032; J. Org. Chem., 2003 (68) 1443-1446; J. Org. Chem., 2002 (67) 5216-5225 and J. Org. Chem., 2002 (67) 3065-3071, Heterocycles 2003 (59) 161-167 and WO061024820.
2) The compounds of formula I wherein R¹, R², R³, R⁴, R⁵, R⁶ and Y are as defined above, m is 1 or 2, and n is 1, can be prepared by processes known *per se*, by reacting e.g. the compounds of formula Ib 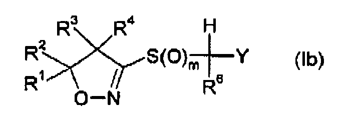 wherein R¹, R², R³, R⁴, R⁶ and Y are as defined above and m is 1 or 2, with compounds of the formula R⁵-X, wherein R⁵ is halogen and X is a suitable leaving group as defined in 1), in the presence of a base as defined in 1), optionally in the presence of a diluent as defined in 1), preferably an inert solvent, and optionally in the presence of a complexing agent as defined in 1) in a temperature range of from -120°C to 100°C, preferably from -80°C to 50°C.
3) The compounds of formula I wherein R¹, R², R³, R⁴, R⁵, R⁶ and Y are as defined above, m is 1 or 2, and n is 1, can be prepared by processes known *per se,* by reacting e.g. the compounds of formula Ic 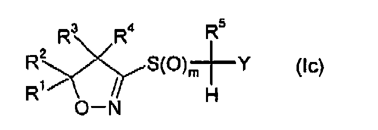 wherein R¹, R², R³, R⁴, R⁵ and Y are as defined above and m is 1 or 2, with compounds of the formula R⁶-X, wherein R⁶ is halogen and X is a suitable leaving group as defined in 1), in the presence of a base as defined in 1), optionally in the presence of a diluent as defined in 1) and optionally in the presence of a complexing agent as defined in 1) in a temperature range of from -120°C to 100°C, preferably from -80°C to 50°C.
4) The compounds of formula I wherein R¹, R², R³, R⁴, R⁶ and Y are as defined above, R^{P} is hydrogen or halogen, m is 1 or 2, and n is 1, can, furthermore, be prepared by processes known *per se* 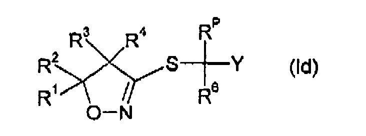 by starting from compounds of formula Id wherein R¹, R², R³, R⁴, R⁶ and Y are as defined above and R^{P} is hydrogen or halogen, and reacting those compounds with a suitable organic or inorganic oxidising agent, e.g. a peroxy acid, such as 3-chloroperoxybenzoic acid, peracetic acid or hydrogen peroxide, an alkoxyperoxide or a periodate, such as sodium periodate, optionally in the presence of a catalyst, such as ruthenium(III) chloride, optionally in the presence of a diluent, such as a halogenated hydrocarbon, e.g. dichloromethane, 1,2-dichloroethane or carbon tetrachloride, an alcohol, e.g. methanol, a polar solvent, e.g. N,N-dimethylformamide, acetonitrile, water or acetic acid, or a mixture thereof. The reactions are usually carried out in a temperature range of from -80°C to 150°C, preferably from -20°C to 120°C. Such processes are known in the literature and are described e.g. in J. Org. Chem., 2003 (68) 3849-3859; J. Med. Chem., 2003 (46) 3021-3032; J. Org. Chem., 2003 (68) 500-511; Bioorg. Med. Chem., 1999 (9) 1837-1844. One equivalent of oxidizing agent is required to convert a sulfide to the corresponding sulfoxide. Two equivalents of oxidizing agent are required to convert a sulfide to the corresponding sulfone.
5) The compounds of formula Ig wherein R¹, R², R³, R⁴, R⁵ and Y are as defined above, can be prepared, for example, by starting from compounds of formula Ie wherein R¹, R², R³, R⁴ and Y are as defined above 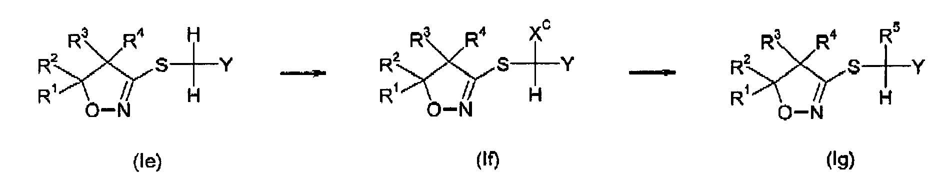 by reacting those compounds with a halogenating agent, e.g. bromine or an N-halo-succinimide, such as N-chlorosuccinimide or N-bromosuccinimide, to form compounds of formula If wherein R¹, R², R³, R⁴ and Y are as defined above, and X^{c} is halogen, optionally in the presence of a diluent, e.g. acetic acid or a halogenated hydrocarbon, such as carbon tetrachloride or dichloromethane, in a temperature rage of from -80°C to 120°C, preferably from -20°C to 60°C. The compounds of formula If wherein R¹, R², R³, R⁴ and Y are as defined above and X^{C} is halogen can then be oxidised directly as described in 4), or optionally in a second step reacted with compounds of formula

   M-R⁵,

   wherein R⁵ is fluoro and M-R⁵ is a suitable salt or an organometal compound in which M is e.g. Li, MgBr, Na, K, Ag or tetraalkylammonium, optionally in the presence of a Lewis acid, e.g. SnCl₄, optionally in the presence of a complexing agent, e.g. hexamethylphosphoramide (HMPA) or 1,3-dimethyl-3,4,5,6-tetrahydro-2(1H)-pyrimidinone (DMPU), and optionally in the presence of a diluent, e.g. acetonitrile, dichloromethane, diethyl ether or tetrahydrofuran, in a temperature range of from -120°C to 100°C, preferably from -80°C to 80°C. Such processes are known in the literature and are described, for example, in J. Org. Chem., 1998 (63) 3706-3716; J. Chem. Soc. Pekin Trans., 1995 (22) 2845-2848; Synthesis 1982 (2), 131-132; Liebigs Annalen, 1993, 49-54 and Synth. Commun., 1990 (20) 1943-1948.
6) The compounds of formula Ii wherein R¹, R², R³, R⁴, R⁵, R⁶ and Y are as defined above, can be prepared, for example, by starting from compounds of formula Ie wherein R¹, R², R³, R⁴ and Y are as defined above 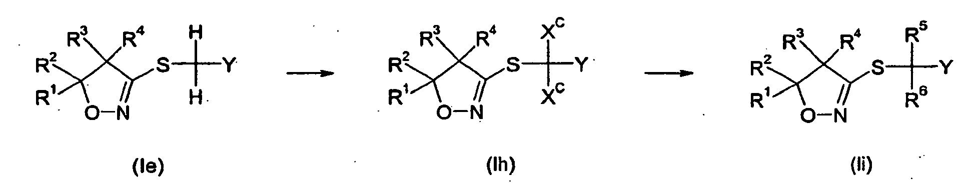 by reacting those compounds with a halogenating agent, e.g. bromine or an N-halo-succinimide, such as N-chlorosuccinimide or N-bromosuccinimide, to form compounds of formula Ih wherein R¹, R², R³, R⁴ and Y are as defined above and X^{C} is halogen, optionally in the presence of a diluent, e.g. acetic acid or a halogenated hydrocarbon such as carbon tetrachloride or dichloromethane, in a temperature range of from -80°C to 120°C, preferably from -20°C to 60°C. The compounds of formula Ih wherein R¹, R², R³, R⁴ and Y are as defined above and X^{c} is halogen can then be oxidised directly as described in 4), or optionally in a second or third step reacted with compounds of formula

   M-R⁵ and / or M-R⁶,

   wherein R⁵ and / or R⁶ are fluoro and M-R⁵ and / or M-R⁶ are a suitable salt or an organometal compound in which M is e.g. Li, MgBr, Na, K, Ag or tetraalkylammonium, optionally in the presence of a Lewis acid, e.g. SnCl₄, optionally in the presence of a complexing agent, e.g. hexamethylphosphoramide (HMPA) or 1,3-dimethyl-3,4,5,6-tetrahydro-2(1H)-pyrimidinone (DMPU), and optionally in the presence of a diluent, e.g. acetonitrile, dichloromethane, diethyl ether or tetrahydrofuran, in a temperature range of from 120°C to 100°C, preferably from -80°C to 80°C. Such processes are known in the literature and are described, for example, in J. Org. Chem., 1998 (63) 3706-3716; J. Chem. Soc. Perkin Trans., 1995 (22) 2845-2848; Synthesis 1982 (2), 131-132; Liebigs Annalen, 1993, 49-54 and Synth. Commun., 1990 (20) 1943-1948.

### Methods for Coupling Reactions

7) The compounds of formula Id as defined in 4) can be prepared by reacting a compound of formula II wherein R⁶ and Y are as defined above, R^{P} is as defined in 4) and X^{A} is a leaving group such as halide e.g. bromide or chloride, an alkylsulfonate, e.g. methylsulfonate, an arylsulfonate, e.g. p-toluenesulfonate, or a haloalkylsulfonate, e.g. trifluoromethylsulfonate, with thiourea, optionally in the presence of a diluent e.g. acetonitrile or an alcohol, e.g. ethanol, optionally in the presence of an alkali iodide, e.g. sodium iodide or potassium iodide, in a temperature range of from -30°C to 100°C, preferably from 0°C to 80°C, to give an isothiourea intermediate of formula IV, which is reacted with a compound of formula V 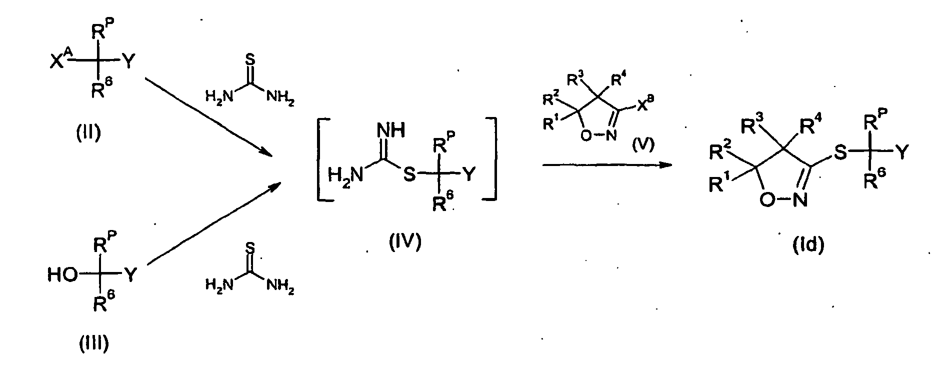 wherein R¹, R², R³ and R⁴ are as defined above, and X^{B} is a suitable leaving group such as halogen, e.g. chloro, an alkylsulfinyl group, an arylsulfinyl group, a haloakylsulfinyl group, an alkylsulfonyl group, e.g. methylsulfonyl, an arylsulfonyl group, e.g. p-toluenesulfonyl, a haloalkylsulfonyl group, e.g. trifluoromethylsulfonyl, or nitro, in the presence of a base, such as a carbonate, e.g. potassium carbonate, sodium carbonate or potassium hydrogencarbonate, or a hydroxide, e.g. potassium hydroxide, or an alkoxide, e.g. sodium alkoxide, optionally in the presence of an alkali iodide, e.g. sodium iodide or sodium bromide, optionally in the presence of a diluent, such as an alcohol, e.g. ethanol, an ether, e.g. 1,4-dioxane or tetrahydrofuran, a polar solvent, e.g. water, acetonitrile or N,N-dimethylformamide, or a mixture of solvents, e.g. a mixture of 1,4-dioxane and water, in a temperature range of from 2C°C to 200°C; preferably from 50°C to 150°C, optionally in the presence of an inert gas e.g. nitrogen, and optionally under microwave irradiation. Such processes are known in the literature and are described, for example, in WO 04/013106 and WO 06/024820.
8) A further method of preparing intermediates of formula IV, wherein R⁶ and Y are as defined above and R^{P} is as defined in 4), is to react a compound of the formula III, wherein R⁶ and Y are as defined above and R^{P} is as defined in 4), with thiourea in the presence of an acid, for example a mineral acid, such as hydrochloric acid or hydrobromic acid, or sulfuric acid, or an organic acid, such as trifluoroacetic acid, and optionally in the presence of a diluent, such as an ether, e.g. 1,4-dioxane or tetrahydrofuran, a polar solvent, e.g. water or NN-dimethylformamide, or a mixture of solvents, e.g. a mixture of 1,4-dioxane and water, in a temperature range of from 20°C to 270°C, preferably from 20C to 150°C, optionally under microwave irradiation. Such processes are known in the literature and are described, for example, in Buchwald and Neilsen, JACS, 110(10), 3171-3175 (1988); Frank and Smith, JACS, 68, 2103-2104 (1946); Vetter, Syn. Comm., 28, 3219-3233 (1998). The intermediate IV is then reacted with a compound of formula V as described in 7) to yield a compound of formula Id as described in 7).
9) A further method of preparing the compounds of formula Id as defined in 4) is to react compound of the formula VI wherein R⁶ and Y are as defined above and R^{P} is as defined in 4), 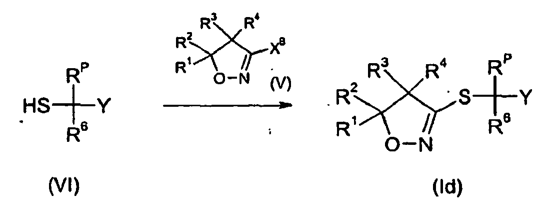 with a compound of formula V as defined in 7), in the presence of a base, e.g. potassium carbonate, optionally in the presence of a diluent, e.g. an amide, such as N,N-dimethylformamide, or an alcohol, such as ethanol, in a temperature range of from 0°C to 100°C, preferably from 20°C to 50°C, and optionally under an inert atmosphere, e.g. nitrogen. Such processes are known in the literature and are described, for example in WO 01/012613, WO 02/062770 and WO 04/010165.
10) Alternatively, the compounds of formula Id as defined in 4) can be prepared by reacting a compound of formula V as defined in 7) with thiourea, optionally in the presence of a diluent e.g. an alcohol, e.g. ethanol, in a temperature range of from -30°C to 150°C, preferably from 0°C to 80°C, to give an isothiourea intermediate of formula VII, 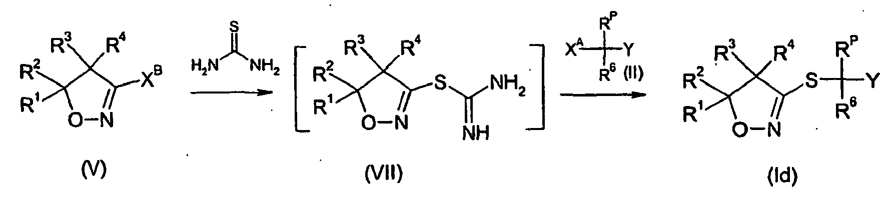 which is then reacted with a compound of formula II as defined in 7) in the presence of a base, such as a carbonate, e.g. potassium carbonate, sodium carbonate or potassium hydrogencarbonate, or a hydroxide, e.g. potassium hydroxide, or an alkoxide, e.g. sodium alkoxide, optionally in the presence of a diluent, such as an alcohol, e.g. ethanol, a polar solvent, e.g. water or N,N-dimethylformamide, or a mixture of solvents, in a temperature range of from 0°C to 200C, preferably from 0°C to 100°C. Such processes are known in the literature and are described, for example, in WO 05/095352.
11) A further method of preparing the compounds of formula Id as defined in 4) is to react an organometal reagent of the formula VIII wherein R⁶ and Y are as defined above, R^{P} is as defined in 4) and M^{B} is a group such as MgCl, MgBr, ZnBr or Li, 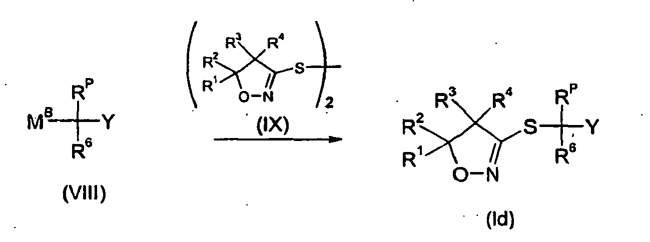 with a compound of formula IX wherein R¹, R², R³ and R⁴ are as defined above optionally in the presence of a diluent, e.g. an ether, such as diethyl ether or tetrahydrofuran, in a temperature range of from -150°C to 100°C, preferably from -80°C to 50°C, and optionally under an inert atmosphere, e.g. nitrogen. The disulfide of formula IX can be formed in situ or prepared separately, e.g. by oxidation of the corresponding sulfide, which in turn is described in JP 2004/224714. Similar processes are known in the literature and are described, for example in J. Chem. Soc. Chem. Commun., 1991, 993-994, J. Chem. Soc. Perkin Trans. 1992 (24) 3371-3375, J. Org. Chem., 1989 (54) 2452-2453.
12) A further method of preparing the compounds of formula Id as defined in 4) is to react a compound of the formula IIa wherein R⁶ and Y are as defined above, R^{P} is as defined in 4) and-X^{C} is functional group that may be cleaved as a radical, e.g. a halogen, such as bromo or chloro, 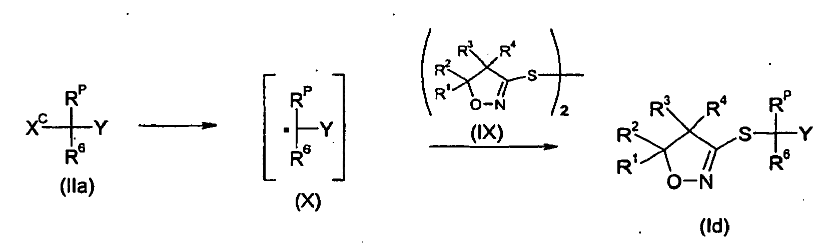 with a radical initiator or a precursor thereof and with a compound of formula IX as defined in 11), optionally in the presence of a base, e.g. a phosphate or hydrogen phosphate such as disodium hydrogen phosphate, a carbonate, e.g. potassium carbonate, sodium carbonate or potassium hydrogencarbonate, optionally in the presence of a diluent, e.g. a polar solvent, such as waster or N,N-dimethylformamide, or mixtures thereof, in a temperature range of from -50°C to 180°C, preferably from -20°C to 50°C, and optionally under an inert atmosphere, e.g. nitrogen. As radical initiator or precursors can be used e.g. sodium dithionite or sodium hydroxymethylsulfinate.
13) A further method of preparing the compounds of formula Id as defined in 4) is to react a compound of the formula II as defined in 7), 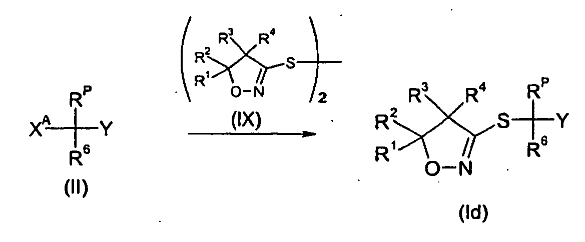 with a compound of formula IX as defined in 11), in the presence of a reducing agent, e.g. a hydride, such as sodium borohydride, a metal, such as zinc, or a hydrosulfite, such as sodium hydrosulfite, optionally in the presence of a base, e.g. a hydroxide, such as sodium hydroxide, a phosphate or hydrogen phosphate, such as disodium hydrogen phosphate, or an amine, such as triethylamine, optionally in the presence of a diluent, e.g. water, an acid, such as acetic acid or hydrochloric acid, an alcohol such as methanol, an ether such as tetrahydrofuran or mixtures thereof, in a temperature range of from -50°C to 180°C, preferably from -20°C to 50°C, and optionally under an inert atmosphere, e.g. nitrogen.

### General methods for making heterocyclic intermediates

15) The compounds of formula II as defined in 7) can be prepared by reacting compounds of formula III as defined in 8) 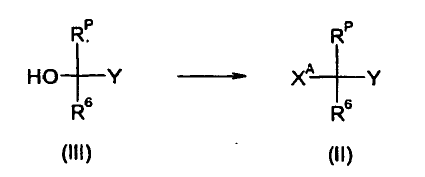 with a halogenating agent, such as hydrogen chloride, hydrogen bromide, phosphorous tribromide, phosphorous trichloride or thionyl chloride, or with an alkyl-, aryl- or haloalkylsulfonyl chloride, such as methanesulfonyl chloride, p-toluenesulfonyl chloride or trifluoromethylsulfonyl chloride, or with a combination of carbon tetrabromide and triphenyl phosphine, optionally in the presence of an inert solvent, e.g. a halogenated hydrocarbon, such as dichloromethane, 1,2-dichloroethane or carbon tetrachloride, an ether, such as diethyl ether or tetrahydrofuran, or an acid, such as acetic acid, optionally in the presence of a base, e.g. an amine, such as triethyl amine, in a temperature range from -50°C to 100°C, preferably from 0°C to 50°C. Such processes are known in the literature and are described, for example, in J. Med. Chem.2005 (48) 3438-3442, J. Org. Chem., 2005 (70) 2274-2284, Org. and Biomolecular Chem., 2005 (3) 1013-1024, Bioorg. Med. Chem. 2004 (13) 363-384, Tetrahedron Asymmetry 2004 (15) 3719-3722.
16) Alternatively, the compounds of formula IIa, wherein R⁶ and Y are as defined above, R^{P} is as defined in 4) and X^{C} is a leaving group such as halogen, e.g. bromo or chloro, can be prepared 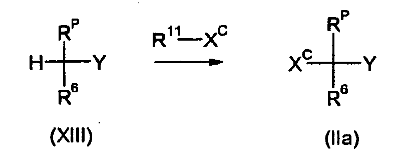 by reacting compounds of formula XIII wherein R⁶ and Y are as defined above, R^{P} is as defined in 4), with compounds of formula R¹¹-X^{c}, wherein X^{c} is a leaving group such as halogen, e.g. bromo or chloro, and R¹¹ is a functional group that may be cleaved to generate X^{C} as a radical, optionally in the presence of a diluent such as a halogenated hydrocarbon, e.g. dichloromethane, 1,2-dichloroethane or carbon tetrachloride, an ether, e.g. tetrahydrofuran, an aromatic compound, e.g. toluene, a polar solvent, e.g. acetonitrile, N,N-dimethylformamide or water, or a mixture thereof. The reactions are usually carried out in a temperature range from -50°C to 120°C, preferably from -5°C to 100°C. The reactions may be carried out optionally in the presence of light and or a radical initiator such as a peroxide, e.g. dibenzoylperoxide, or an azo compound, e.g. 2,2'-azobisisobutyronitrile (AIBN). Suitable compounds of formula R¹¹-X^{c} include compounds in which R¹¹ is a succimmido group, e.g. N-chlorosuccinimide and N-bromosuccinimide. Similar processes are known in the literature and are described, e.g. Tetrahedron, 1988 (44) 461-469; Journal of Organic Chemistry, 1981 (46) 679-686; J. Chem. Soc., Perkin Trans 1, 1985 (6), 1167-1170.
17) The compounds or formula XVa wherein Y is as defined above can conveniently be prepared 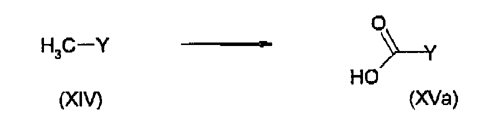 by reacting compounds of formula XIV wherein Y is as defined above with a Suitable inorganic oxidising agent, such as potassium permanganate, in the presence of a base, such as sodium carbonate, in a suitable solvent, such as water. The reactions are usually carried out in a temperature range from 0°C to 150°C, preferably from 80°C to 120°C. Similar processes are known in the literature and are described, e.g. J. Heterocyclic Chem. 1987 (24) 1275-79.
19) Alternatively, the compounds of formula IIIb, wherein Y is as defined above, can be prepared by reacting a compound of formula XV, 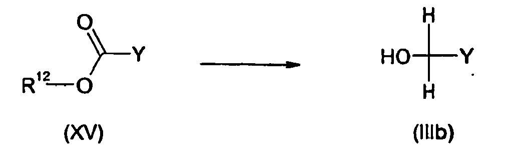 wherein Y is as defined above and R¹² is hydrogen or C₁-C₁₀alkyl, with a reducing agent, e.g. a metal hydride, such as diisobutyl aluminium hydride, lithium aluminium hydride, sodium borohydride, lithium borohydride, or diborane, optionally in the presence of an inert solvent, e.g. an ether, such as diethyl ether, 1,4-dioxane or tetrahydrofuran, an alcohol, such as methanol or ethanol, or an aromatic hydrocarbon, such as toluene. Such reactions are usually carried out in a temperature range from -50°C to 100°C, preferably from (0°C to 80°C. Such processes are known in the literature and are described, for example, in Tetrahedron Asymmetry 2004 (15) 3719-3722, J. Med. Chem., 2004 (47) 2176-2179, Heterocyclic Communications 2002 (8) 385-390, J. Antibiotics, 1995 (48) 1320-1329.

### Methods for making triazole intermediates

20) Compounds of formula XXa or compounds of formula XXb, wherein R⁷ and R⁸ are as defined above, 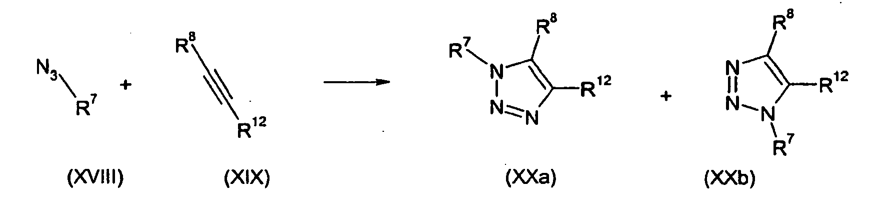 and R¹² is hydrogen, C₁-C₁₀alkyl, C₁-C₁₀haloalkyl, tetrahydro-pyran-2-yloxymethyl, C₁-C₁₀alkoxycarbonyl, formyl, or the group Q 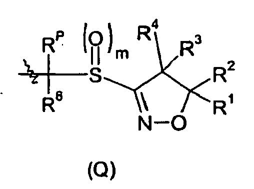 wherein R¹, R², R³, R⁴ and R⁶ are as defined above, R^{P} is as defined in 4), and m is as defined above, preferably m is 0, can be prepared by reacting compounds of formula XVIII, wherein R⁷ is as defined above, with compounds of formula XIX, wherein R⁸ and R¹² are as defined above, optionally in the presence of a catalyst, e.g. a transition metal catalyst, such as CuCl, CuI, CuBr₂, copper powder, optionally in the presence of a diluent such as a halogenated hydrocarbon, e.g. 1,2-dichloroethane or carbon tetrachloride, an ether, e.g. tetrahydrofuran or 1,4-dioxane, an aromatic compound, e.g. toluene, an alcohol, e.g. methanol, an amide, e.g. N,N-dimethylformamide, water or a mixture thereof. The reactions are usually carried out in a temperature range from -50°C to 200°C, preferably from 0°C to 160°C. Depending on the reaction conditions, compounds of formula XVa and XVb are obtained exclusively or as mixtures in varying ratios. Similar processes are known in the literature and are described in, e.g. European Journal of Organic Chemistry, 2004 3789-3791; Journal of Fluorine Chemistry, 2004 (125) 1415-1423;
21) Compounds of formula XXII, wherein R⁸ is as defined above and R¹² is as defined in 20), 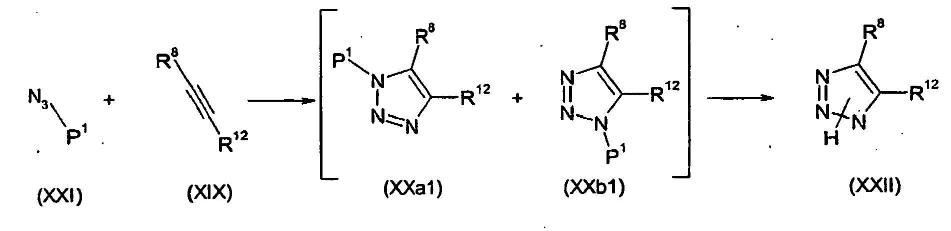 can be prepared by reacting compounds of formula XXI, wherein P¹ is an organic moiety that can be cleaved after the reaction, with compounds of formula XIX, wherein R⁸ is as defined above and R¹² is as defined in 20), optionally in the presence of a catalyst, e.g. a transition metal catalyst, such as CuCI, CuI, CuBr₂, copper powder, optionally in the presence of a diluent such as a halogenated hydrocarbon, e.g. 1,2-dichloroethane or carbon tetrachloride, an ether, e.g. tetrahydrofuran or 1,4-dioxane, an aromatic compound, e.g. toluene, an alcohol, e.g. methanol, an amide, e.g. N,N-dimethyl-formamide, water or a mixture thereof. The reactions are usually carried out in a temperature range from -50°C to 200°C, preferably from 0°C to 160°C. The reaction initially leads to intermediates of formula XXa1 and XXb1. Depending on the reaction conditions, compounds of formula XXa1 and XXb1 are obtained exclusively or as mixtures in varying ratios. Suitable groups P¹ include trialkylsilyl groups, such as trimethylsilyl, or optionally substituted benzyl groups, such as benzyl or 4-methoxybenzyl. The protecting group is cleaved either in situ under the reaction conditions or in a separate step. Similar processes are known in the literature and are described in, e.g. Molecular Diversity, 2003 (7) 171-174; J. Heterocyclic Chemistry, 1976 (13) 589-592; WO 04/106324; J. Med. Chem., 2004 (47) 2176-2179.
22) Alternatively, compounds of formula XXII as defined in 21) can be prepared by reacting compounds of formula XXIII 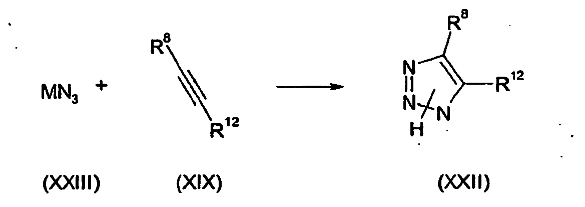 wherein MN₃ is an inorganic azide salt, usually sodium azide, with compounds of formula XIX as defined in 20), optionally in the presence of a diluent such as DMSO, toluene, acetonitrile, an alcohol, e.g. ethanol, an amide, e.g. N,N-dimethylformamide, water or a mixture thereof. The reactions are usually carried out in a temperature range from -50°C to 200°C, preferably from 0°C to 160°C. Similar processes are known in the literature and are described, e.g. US 6,051,717; Tetrahedron Letters, 2001 (42) 9114.
23) Compounds of formula XXa, XXb and / or XXc as defined in 20) can be prepared by reacting compounds of formula XXII as defined in 21) with compounds of formula R⁷-X^{E} 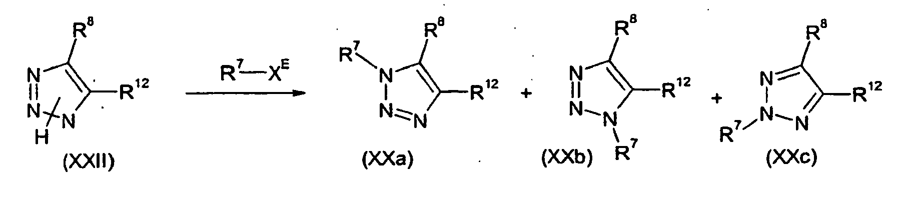 wherein R⁷ is as defined above and X^{E} is a suitable leaving group, such as halogen, e.g. bromide, chloride or iodide, a carboxylate, such as acetate, or an alkylsulfonate, e.g. methylsulfonate, or an arylsulfonate, e.g. p-toluenesulfonate, in the presence of a base, e.g. a carbonate, such as potassium carbonate, a hydroxide, such as potassium hydroxide, a metal hydride, such as sodium hydride, an amine, such as triethylamine, optionally in the presence of a diluent, e.g. an ether, such as tetrahydrofuran, an amide, such as N,N-dimethylformamide, an alcohol, such as methanol, acetonitrile or acetone or mixtures thereof, and optionally in the presence of a phase transfer catalyst, such as trimethylammonium bromide, in a temperature range of from -120°C to 200°C, preferably from -20°C to 80°C. Depending on the reaction conditions, compounds of formula XXa, XXb and XXc are obtained exclusively or as mixtures in varying ratios. Such processes are known in the literature and are described, for example, in Bioorg. Med. Chem. Lett., 2004 (14) 2401-2405; WO 04/018438 and US 4,820,844.
24) The synthesis of 1,2,3-triazoles and derivatives thereof are well known within the literature, so for a general discussion of their synthesis see Tome, A. C. Product class 13: 1,2,3-triazoles. Science of Synthesis (2004), 13 415-601; H. Wamhoff, 1,2,3-Triazoles. Comprehensive Heterocyclic Chemistry (1984), 4A 669-733; W.-Q. Fan, A. Katritzky, 1,2,3-Triazoles. Comprehensive Heterocyclic Chemistry II (1996), 4 1-127; B. B. Sharpless Synthesis, 2005 (9) 1514-1520; K. Banert, European Journal of Organic Chemistry, 2005 3704-3714; V. P. Krivopalov, O. P. Shkurko, Russ. Chem. Rev. 2005 (74) 339-379; M. Begtrup, Acta Chemica Scandinavica (1990), 44(10), 1050-7. R. F. Coles, C. S. Hamilton, Journal of the American Chemical Society (1946), 68 1799-801; M. Begtrup Bull. Soc. Chim. Belg., 1988 (97) 573-597). Xu, Bo; Mae, Masayuki; Hong, Jiyoung A.; Li, Youhua; Hammond, Gerald B., Synthesis (2006), (5), 803-806. Mae, Masayuki; Hong, Jiyoung A.; Xu, Bo; Hammond, Gerald B. Organic Letters (2006), 8(3), 479-482. Buckle, Derek R.; Rockell, Caroline J. M. Journal of the Chemical Society, Perkin Transactions 1: Organic and Bio-Organic Chemistry (1972-1999) (1982), (2), 627-30. Journet, Michel; Cai, Dongwei; Hughes, David L.; Kowal, Jason J.; Larsen, Robert D.; Reider, Paul J., Organic Process Research & Development (2005), 9(4), 490-498. US 6,051,717.

The compounds of formula I according to the invention can be used as herbicides in unmodified form, as obtained in the synthesis, but they are generally formulated into herbicidal compositions in various ways using formulation adjuvants, such as carriers, solvents and surface-active substances. The formulations can be in various physical forms, e.g. in the form of dusting powders, gels, wettable powders, water-dispersible granules, water-dispersible tablets, effervescent pellets, emulsifiable concentrates, microemulsifiable concentrates, oil-in-water emulsions, oil-flowables, aqueous dispersions, oily dispersions, suspo-emulsions, capsule suspensions, emulsifiable granules, soluble liquids, water-soluble concentrates (with water or a water-miscible organic solvent as carrier), impregnated polymer films or in other forms known e.g. from the Manual on Development and Use of FAO Specifications for Plant Protection Products, 5th Edition, 1999. Such formulations can either be used directly or they are diluted prior to use. The dilutions can be made, for example, with water, liquid fertilisers, micronutrients, biological organisms, oil or solvents.

The formulations can be prepared e.g. by mixing the active ingredient with the formulation adjuvants in order to obtain compositions in the form of finely divided solids, granules, solutions, dispersions or emulsions. The active ingredients can also be formulated with other adjuvants, such as finely divided solids, mineral oils, oils of vegetable or animal origin, modified oils of vegetable or animal origin, organic solvents, water, surface-active substances or combinations thereof The active ingredients can also be contained in very fine microcapsules consisting of a polymer. Microcapsules contain the active ingredients in a porous carrier. This enables the active ingredients to be released into the environment in controlled amounts (e.g. slow-release). Microcapsules usually have a diameter of from 0.1 to 500 microns. They contain active ingredients in an amount of about from 25 to 95 % by weight of the capsule weight. The active ingredients can be in the form of a monolithic solid, in the form of fine particles in solid or liquid dispersion or in the form of a suitable solution. The encapsulating membranes comprise, for example, natural or synthetic rubbers, cellulose, styrene/butadiene copolymers, polyacrylonitrile, polyacrylate, polyesters, polyamides, polyureas, polyurethane or chemically modified polymers and starch xanthates or other polymers that are known to the person skilled in the art in this connection. Alternatively, very fine microcapsules can be formed in which the active ingredient is contained in the form of finely divided particles in a solid matrix of base substance, but the microcapsules are not themselves encapsulated.

The formulation adjuvants that are suitable for the preparation of the compositions according to the invention are known *per se.* As liquid carriers there may be used: water, toluene, xylene, petroleum ether, vegetable oils, acetone, methyl ethyl ketone, cyclohexanone, acid anhydrides, acetonitrile, acetophenone, amyl acetate, 2-butanone, butylene carbonate, chlorobenzene, cyclohexane, cyclohexanol, alkyl esters of acetic acid, diacetone alcohol, 1,2-dichloropropane, diethanolamine, p-diethylbenzene, diethylene glycol, diethylene glycol abietate, diethylene glycol butyl ether, diethylene glycol ethyl ether, diethylene glycol methyl ether, N,N-dimethylformamide, dimethyl sulfoxide, 1,4-dioxane, dipropylene glycol, dipropylene glycol methyl ether, dipropylene glycol dibenzoate, diproxitol, alkylpyrrolidone, ethyl acetate, 2-ethylhexanol, ethylene carbonate, 1,1,1-trichloroethane, 2-heptanone, alpha-pinene, d-limonene, ethyl lactate, ethylene glycol, ethylene glycol butyl ether, ethylene glycol methyl ether, gamma-butyrolactone, glycerol, glycerol acetate, glycerol diacetate, glycerol triacetate, hexadecane, hexylene glycol, isoamyl acetate, isobornyl acetate, isooctane, isophorone, isopropylbenzene, isopropyl myristate, lactic acid, laurylamine, mesityl oxide, methoxypropanol, methyl isoamyl ketone, methyl isobutyl ketone, methyl laurate, methyl octanoate, methyl oleate, methylene chloride, m-xylene, n-hexane, n-octylamine, octadecanoic acid, octylamine acetate, oleic acid, oleylamine, o-xylene, phenol, polyethylene glycol (PEG400), propionic acid, propyl lactate, propylene carbonate, propylene glycol, propylene glycol methyl ether, p-xylene, toluene, triethyl phosphate, triethylene glycol, xylenesulfonic acid, paraffin, mineral oil, trichloroethylene, perchloroethylene, ethyl acetate, amyl acetate, butyl acetate, propylene glycol methyl ether, diethylene glycol methyl ether, methanol, ethanol, isopropanol, and alcohols of higher molecular weight, such as amyl alcohol, tetrahydrofurfuryl alcohol, hexanol, octanol, ethylene glycol, propylene glycol, glycerol, N-methyl-2-pyrrolidone and the like. Water is generally the carrier of choice for diluting the concentrates. Suitable solid carriers are, for example, talc, titanium dioxide, pyrophyllite clay, silica, attapulgite clay, kieselguhr, limestone, calcium carbonate, bentonite, calcium montmorillonite, cottonseed husks, wheat flour, soybean flour, pumice, wood flour, ground walnut shells, lignin and similar substances, as described, for example, in CFR 180.1001. (c) & (d).

A large number of surface-active substances can advantageously be used in both solid and liquid formulations, especially in those formulations which can be diluted with a carrier prior to use. Surface-active substances may be anionic, cationic, non-ionic or polymeric and they can be used as emulsifiers, wetting agents or suspending agents or for other purposes. Typical surface-active substances include, for example, salts of alkyl sulfates, such as diethanolammonium lauryl sulfate; salts of alkylarylsulfonates, such as calcium dodecylbenzenesulfonate; alkylphenol/alkylene oxide addition products, such as nonylphenol ethoxylate; alcohol/alkylene oxide addition products, such as tridecylalcohol ethoxylate; soaps, such as sodium stearate; salts of alkylnaphthalenesulfonates, such as sodium dibutylnaphthalenesulfonate; dialkyl esters of sulfosuccinate salts, such as sodium di(2-ethylhexyl)sulfosuccinate; sorbitol esters, such as sorbitol oleate; quaternary amines, such as lauryltrimethylammonium chloride, polyethylene glycol esters of fatty acids, such as polyethylene glycol stearate; block copolymers of ethylene oxide and propylene oxide; and salts of mono- and di-alkylphosphate esters; and also further substances described e.g. in "McCutcheon's Detergents and Emulsifiers Annual" MC Publishing Corp., Ridgewood New Jersey, 1981.

Further adjuvants that can usually be used in pesticidal formulations include crystallisation inhibitors, viscosity modifiers, suspending agents, dyes, anti-oxidants, foaming agents, light absorbers, mixing auxiliaries, antifoams, complexing agents, neutralising or pH-modifying substances and buffers, corrosion inhibitors, fragrances, wetting agents, take-up enhancers, micronutrients, plasticisers, glidants, lubricants, dispersants, thickeners, antifreezes, microbicides, and also liquid and solid fertilisers.

The compositions according to the invention can additionally include an additive comprising an oil of vegetable or animal origin, a mineral oil, alkyl esters of such oils or mixtures of such oils and oil derivatives. The amount of oil additive in the composition according to the invention is generally from 0.01 to 10 %, based on the spray mixture. For example, the oil additive can be added to the spray tank in the desired concentration after the spray mixture has been prepared. Preferred oil additives comprise mineral oils or an oil of vegetable origin, for example rapeseed oil, olive oil or sunflower oil, emulsified vegetable oil, such as AMIGO®(Rhône-Poulenc Canada Inc.), alkyl esters of oils of vegetable origin, for example the methyl derivatives, or an oil of animal origin, such as fish oil or beef tallow. A preferred additive contains, for example, as active components essentially 80 % by weight alkyl esters of fish oils and 15% by weight methylated rapeseed oil, and also 5 % by weight of customary emulsifiers and pH modifiers. Especially preferred oil additives comprise alkyl esters of C₈-C₂₂ fatty acids, especially the methyl derivatives of C₁₂-C₁₈ fatty acids, for example the methyl esters of lauric acid, palmitic acid and oleic acid, being of importance. Those esters are known as methyl laurate (CAS-111-82-0), methyl palmitate (CAS-112-39-0) and methyl oleate (CAS-112-62-9). A preferred fatty acid methyl ester derivative is Emery®2230 and 2231 (Cognis GmbH). Those and other oil derivatives are also known from the Compendium of Herbicide Adjuvants, 5th Edition, Southern Illinois University, 2000.

The application and action of the oil additives can be further improved by combination with surface-active substances, such as non-ionic, anionic or cationic surfactants. Examples of suitable anionic, non-ionic and cationic surfactants are listed on pages 7 and 8 of WO 97/34485. Preferred surface-active substances are anionic surfactants of the dodecylbenzylsulfonate type, especially the calcium salts thereof, and also non-ionic surfactants of the fatty alcohol ethoxylate type. Special preference is given to ethoxylated C₁₂-C₂₂ fatty alcohols having a degree of ethoxylation of from 5 to 40. Examples of commercially available surfactants are the Genapol types (Clariant AG). Also preferred are silicone surfactants, especially polyalkyl-oxide-modified heptamethyltriloxanes which are commercially available e.g. as Silwet L-77®, and also perfluorinated surfactants. The concentration of the surface-active substances in relation to the total additive is generally from 1 to 30 % by weight. Examples of oil additives consisting of mixtures of oil or mineral oils or derivatives thereof with surfactants are Edenor ME SU®, Turbocharge® (Syngenta AG, CH) or ActipronC (BP Oil UK Limited, GB).

If desired, it is also possible for the mentioned surface-active substances to be used in the formulations on their own, that is to say without oil additives.

Furthermore, the addition of an organic solvent to the oil additive/surfactant mixture may contribute to an additional enhancement of action. Suitable solvents are, for example, Solvesso® (ESSO) or Aromatic Solvent® (Exxon Corporation). The concentration of such solvents can be from 10 to 80 % by weight of the total weight. Oil additives that are present in admixture with solvents are described, for example, in US-A-4,834,9.08. A commercially available oil additive disclosed therein is known by the name MERGE® (BASF Corporation). A further oil additive that is preferred according to the invention is SCORE® (Syngenta Crop Protection Canada).

In addition to the oil additives listed above, for the purpose of enhancing the action of the compositions according to the invention it is also possible for formulations of alkylpyrrolidones (e.g. Agrimax®) to be added to the spray mixture. Formulations of synthetic lattices, e.g. polyacrylamide, polyvinyl compounds or poly-1-p-menthene (e.g. Bond®, Courier® or Emerald®) may also be used. It is also possible for solutions that contain propionic acid, for example Eurogkem Pen-e-trate®, to be added to the spray mixture as action-enhancing agent.

The herbicidal compositions generally comprise from 0.1 to 99 % by weight, especially from 0.1 to 95 % by weight, compounds of formula I and from 1 to 99.9 % by weight of a formulation adjuvant which preferably includes from 0 to 25 % by weight of a surface-active substance. Whereas commercial products will preferably be formulated as concentrates, the end user will normally employ dilute formulations.

The rates of application of compounds of formula I may vary within wide limits and depend on the nature of the soil, the method of application (pre- or post-emergence; seed dressing; application to the seed furrow; no tillage application etc.), the crop plant, the grass or weed to be controlled, the prevailing climatic conditions, and other factors governed by the method of application, the time of application and the target crop. The compounds of formula I according to the invention are generally applied at a rate of from 10 to 2000 g/ha, especially from 50 to 1000 g/ha.

Preferred formulations have especially the following compositions (% = percent by weight):

### Emulsifiable concentrates:

| | |
|---|---|
| active ingredient: | 1 to 95 %, preferably 60 to 90 % |
| surface-active agent: | 1 to 30%, preferably 5 to 20 % |
| liquid carrier: | 1 to 80%, preferably 1 to 35 % |

### Dusts:

| | |
|---|---|
| active ingredient: | 0.1 to 10 %, preferably 0.1 to 5 % |
| solid carrier: | 99.9 to 90 %, preferably 99.9 to 99 % |

### Suspension concentrates:

| | |
|---|---|
| active ingredient: | 5 to 75 %, preferably 10 to 50 % |
| water: | 94 to 24%, preferably 88 to 30 % |
| surface-active agent: | 1 to 40 %, preferably 2 to 30 % |

### Wettable powders:

| | |
|---|---|
| active ingredient: | 0.5 to 90 %, preferably 1 to 80 % |
| surface-active agent: | 0.5 to 20 %, preferably 1 to 15 % |
| solid carrier: | 5 to 95 %, preferably 15 to 90 % |

### Granules:

| | |
|---|---|
| active ingredient: | 0.1 to 30 %, preferably 0.1 to 15 % |
| solid carrier: | 99.5 to 70 %, preferably 97 to 85 % |

The following Examples further illustrate, but do not limit, the invention.

### Formulation Examples for herbicides of formula I (% = % by weight)

| F1. Emulsifiable concentrates | a) | b) | c) | d) |
|---|---|---|---|---|
| active ingredient | 5 % | 10 % | 25 % | 50 % |
| calcium dodecylbenzenesulfonate | 6 % | 8 % | 6 % | 8 % |
| castor oil polyglycol ether (36 mol of ethylene oxide) | 4 % | - | 4 % | 4 % |
| octylphenol polyglycol ether (7-8 mol of ethylene oxide) | - | 4 % | - | 2 % |
| NMP | - | - | 10 % | 20 % |
| arom. hydrocarbon mixture C₉-C₁₂ | 85 % | 78 % | 55 % | 16 % |

Emulsions of any desired concentration can be obtained from such concentrates by dilution with water.

| F2. Solutions | a) | b) | c) | d) |
|---|---|---|---|---|
| active ingredient | 5% | 10 % | 50% | 90 % |
| 1-methoxy-3-(3-methoxy-propoxy)-propane | - | 20 % | 20 % | - |
| polyethylene glycol MW 400 | 20 % | 10 % | - | - |
| NMP | - | - | 30% | 10% |
| arom. hydrocarbon mixture C₉-C₁₂ | 75 % | 60 % | - | - |

The solutions are suitable for use in the form of microdrops.

| F3. Wettable powders | a) | b) | c) | d) |
|---|---|---|---|---|
| active ingredient | 5 % | 25 % | 50 % | 80 % |
| sodium lignosulfonate | 4 % | - | 3 % | - |
| sodium lauryl sulfate | 2 % | 3 % | - | 4 % |
| sodium diisobutylnaphthalene-sulfonate | - | 6 % | 5 % | 6 % |
| octylphenol polyglycol ether | - | 1 % | 2 % | - |
| (7-8 mol of ethylene oxide) | | | | |
| highly dispersed silicic acid | 1 % | 3 % | 5 % | 10 % |
| kaolin | 88 % | 62 % | 35 % | - |

The active ingredient is mixed thoroughly with the adjuvants and the mixture is thoroughly ground in a suitable mill, affording wettable powders which can be diluted with water to give suspensions of any desired concentration.

| F4. Coated granules | a) | b) | c) |
|---|---|---|---|
| active ingredient | 0.1 % | 5 % | 15 % |
| highly dispersed silicic acid | 0.9 % | 2 % | 2 % |
| inorganic carrier (diameter 0.1 - 1 mm) e.g. CaCO₃ or SiO₂ | 99.0 % | 93 % | 83 % |

The active ingredient is dissolved in methylene chloride and applied to the carrier by spraying, and the solvent is then evaporated off *in vacuo.*

| F5. Coated granules | a) | b) | c) |
|---|---|---|---|
| active ingredient | 0.1 % | 5 % | 15 % |
| polyethylene glycol MW 200 | 1.0 % | 2 % | 3 % |
| highly dispersed silicic acid | 0.9 % | 1 % | 2 % |
| inorganic carrier (diameter 0.1 - 1 mm) e.g. CaCO₃ or SiO₂ | 98.0 % | 92 % | 80 % |

The finely ground active ingredient is uniformly applied, in a mixer, to the carrier moistened with polyethylene glycol. Non-dusty coated granules are obtained in this manner.

| F6. Extruder granules | a) | b) | c) | d) |
|---|---|---|---|---|
| active ingredient | 0.1 % | 3 % | 5 % | 15 % |
| sodium lignosulfonate | 1.5 % | 2 % | 3 % | 4 % |
| carboxymethylcellulose | 1.4 % | 2 % | 2 % | 2 % |
| kaolin | 97.0 % | 93 % | 90 % | 79 % |

The active ingredient is mixed and ground with the adjuvants, and the mixture is moistened with water. The mixture is extruded and then dried in a stream of air.

| F7. Dusts | a) | b) | c) |
|---|---|---|---|
| active ingredient | 0.1 % | 1 % | 5 % |
| talcum | 39.9 % | 49 % | 35 % |
| kaolin | 60.0 % | 50 % | 60 % |

Ready-to-use dusts are obtained by mixing the active ingredient with the carriers and grinding the mixture in a suitable mill.

| F8. Suspension concentrates | a) | b) | c) | d) |
|---|---|---|---|---|
| active ingredient | 3 % | 10 % | 25 % | 50 % |
| ethylene glycol | 5 % | 5 % | 5 % | 5 % |
| nonylphenol polyglycol ether (15 mol of ethylene oxide) | - | 1 % | 2 % | - |
| sodium lignosulfonate | 3 % | 3 % | 4 % | 5 % |
| carboxymethylcellulose | 1 % | 1 % | 1 % | 1 % |
| 37 % aqueous formaldehyde solution | 0.2 % | 0.2 % | 0.2 % | 0.2 % |
| silicone oil emulsion | 0.8 % | 0.8 % | 0.8 % | 0.8 % |
| water | 87 % | 79 % | 62 % | 38 % |

The finely ground active ingredient is intimately mixed with the adjuvants; giving a suspension concentrate from which suspensions of any desired concentration can be obtained by dilution with water.

The invention also relates to a method of controlling plants which comprises applying to the plants or to the locus thereof a herbicidally effective amount of a compound of formula I, or of a composition comprising such a compound.

The invention also relates to a method of inhibiting plant growth which comprises applying to the plants or to the locus thereof a herbicidally effective amount of a compound of formula I, or of a composition comprising such a compound.

The invention also relates to a method of selectively controlling grasses and weeds in crops of useful plants which comprises applying to the useful plants or locus thereof or to the area of cultivation a herbicidally effective amount of a compound of formula I, or of a composition comprising such a compound.

Crops of useful plants in which the composition according to the invention can be used include cereals, for example barley and wheat, cotton, oilseed rape, maize, rice, soy beans, sugar beet and sugar cane, especially cereals and maize.

Crops can also include trees, such as palm trees, coconut trees or other nuts, and vines such as grapes.

The grasses and weeds to be controlled may be both monocotyledonous species, for example Agrostis, Alopecurus, Avena, Bromus, Cyperus, Digitaria, Echinochloa, Lolium, Monochoria, Rottboellia, Sagittaria, Scirpus, Setaria, Sida and Sorghum, and dicotyledonous species, for example Abutilon, Amaranthus, Chenopodium, Chrysanthemum, Galium, Ipomoea, Nasturtium, Sinapis, Solanum, Stellaria, Veronica, Viola and Xanthium.

Crops are to be understood as also including those crops which have been rendered tolerant to herbicides or classes of herbicides (e.g. ALS-, GS-, EPSPS-, PPO-and HPPD-inhibitors) by conventional methods of breeding or by genetic engineering. An example of a crop that has been rendered tolerant to imidazolinones, e.g. imazamox, by conventional methods of breeding is Clearfield®summer rape (canola). Examples of crops that have been rendered tolerant to herbicides by genetic engineering methods include e.g. glyphosate- and glufosinate-resistant maize varieties commercially available under the trade names RoundupReady®and LibertyLink®.

Crops are also to be understood as being those which have been rendered resistant to harmful insects by genetic engineering methods, for example Bt maize (resistant to European corn borer), Bt. cotton (resistant to cotton boll weevil) and also Bt potatoes (resistant to Colorado beetle). Examples of Bt maize are the Bt 176 maize hybrids of Nk® (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO wand EP-A-427 529. Examples of transgenic plants comprising one or more genes that code for an insecticidal resistance and express one or more toxins are KnockOut® (maize), Yield Gard® (maize), NuCOTIN33B® (cotton), Bollgard® (cotton), NewLeaf® (potatoes), NatureGard® and Protexcta®. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crops are also to be understood as being those which are obtained by conventional methods of breeding or genetic engineering and contain so-called output traits (e.g. improved storage stability, higher nutritional value and improved flavour).

Areas under cultivation include land on which the crop plants are already growing and land intended for cultivation with those crop plants.

The compounds of formula I according to the invention can also be used in combination with other herbicides. In particular, the following mixtures of the compound of formula I are important:

Mixtures of a compound of formula I with S-metolachlor (549) or a compound of formula I with metolachlor (548).

Mixtures of a compound of formula I with a triazine (e.g. compound of formula I + ametryn (20), compound of formula I + atrazine (37), compound of formula I + cyanazine (183), compound of formula I + dimethametryn (259), compound of formula I + metribuzin (554), compound of formula I + prometon (665), compound of formula I + prometryn (666), compound of formula I + propazine (672), compound of formula I + simazine (730), compound of formula I + simetryn (732), compound of formula I + terbumeton (774), compound of formula I + terbuthylazine (775), compound of formula I + terbutryn (776), compound of formula I + trietazine (831)). Particularly preferred are mixtures of a compound of formula I with atrazine, metribuzin, prometryn or with terbuthylazine.

Mixtures of a compound of formula I with an HPPD inhibitor (e.g. compound of formula I + isoxaflutole (479), compound of formula I + mesotrione (515), compound of formula I + sulcotrione (747), compound of formula I + tembotrione (CAS RN 335104-84-2), compound of formula I + topramezone (CAS RN 210631-68-8), compound of formula I + 4-hydroxy-3-[[2-[(2-methoxyethoxy)methyl]-6-(trifluoromethyl)-3-pyridinyl]carbonyl]-bicyclo[3.2.1]oct-3-en-2-one (CAS RN 352010-68-5), compound of formula I + 4-hydroxy-3-[[2-(3-methoxypropyl)-6-(difluoromethyl)-3-pyridinyl]carbonyl]-bicyclo[3.2.]oct-3-en-2-one).

Mixtures of a compound of formula I with an HPPD inhibitor and a triazine.

Mixtures of a compound of formula I with glyphosate (419).

Mixtures of a compound of formula I with glyphosate and an HPPD inhibitor (e.g. compound of formula I + glyphosate + isoxaflutole, compound of formula I + glyphosate + mesotrione, compound of formula I + glyphosate + sulcotrione, compound of formula I + glyphosate + tembotrione, compound of formula I + glyphosate + topramezone, compound of formula I + glyphosate + 4-hydroxy-3-[[2-[(2-methoxyethoxy)methyl]-6-(trifluoromethyl)-3-pyridinyl]carbonyl]-bicyclo[3.2.1]oct-3-en-2-one, compound of formula I + glyphosate + 4-hydroxy-3-[[2-(3-methoxypropyl)-6-(difluoromethyl)-3-pyridinyl]carbonyl]-bicyclo[3.2.1 ]oct-3-en-2-one).

Mixtures of a compound of formula I with glufosinate-ammonium (418).

Mixtures of a compound of formula I with glufosinate-ammonium and an HPPD inhibitor (e.g. compound of formula I + glufosinate-ammonium + isoxaflutole, compound of formula I + glufosinate-ammonium + mesotrione, compound of formula I + glufosinate-ammonium + sulcotrione, compound of formula I + glufosinate-ammonium + tembotrione, compound of formula I + glufosinate-ammonium + topramezone, compound of formula I + glufosinate-ammonium + 4-hydroxy-3-[[2-[(2-methoxyethoxy)methyl]-6-(trifluoromethyl)-3-pyridinyl]carbonyl]-bicyclo[3.2.1]oct-3-en-2-one, compound of formula I + glufosinate-ammonium + 4-hydroxy-3-[[2-(3-methoxypropyl)-6-(difluoromethyl)-3-pyridinyl]carbonyl]-bicyclo[3.2.1]oct-3-en-2-one).

Mixtures of a compound of formula I with a triazolinone (e.g. compound of formula I + amicarbazone (21)).

Mixtures of a compound of formula I with an ALS inhibitor (e.g. compound of formula I + chlorsulfuron (147), compound of formula I + cinosulfuron (154), compound of formula I + cloransulam-methyl (164), compound of formula I + ethametsulfuron-methyl (306), compound of formula I + flazasulfuron (356), compound of formula I + foramsulfuron (402), compound of formula I + flumetsulam (374), compound of formula I + imazamethabenz-methyl (450), compound of formula I + imazamox (451), compound of formula I + imazapic (452), compound of formula I + imazapyr (453), compound of formula I + imazethapyr (455), compound of formula I + iodosulfuron-methyl-sodium (466), compound of formula I + metsulfuron-methyl (555), compound of formula I + nicosulfuron (577), compound of formula I + oxasulfuron (603), compound of formula I + primisulfuron-methyl (657), compound of formula I + prosulfuron (684), compound of formula I+ pyrithiobac-sodium (709), compound of formula I + rimsulfuron (721), compound of formula I + sulfosulfuron (752), compound of formula I + thifensulfuron-methyl (thiameturon-methyl) (795), compound of formula I + triasulfuron (817), compound of formula I + tribenuron-methyl (822), compound of formula I + trifloxysulfuron-sodium (833), compound of formula I + thiencarbazone (BAY636)). Particularly preferred are mixtures of a compound of formula I with flazasulfuron, foramsulfuron, flumetsulam, imazapyr, imazethapyr, iodosulfuron-methyl-sodium, nicosulfuron, rimsulfuron, trifloxysulfuron-sodium or with 4-[(4,5-dihydro-3-methoxy-4-methyl-5-oxo)-1H-1,2,4-triazol-1-ylcarbonylsulfamoyl]-5-methylthiophene-3-carboxylic acid (BAY636).

Mixtures of a compound of formula I with a PPO inhibitor (e.g. compound of formula I + fomesafen (401), compound of formula I + flumioxazin (376), compound of formula I + sulfentrazone (749), compound of formula I + [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetic acid ethyl ester) (CAS RN 353292-31-6). Particularly preferred are mixtures of a compound of formula I with flumioxazin, sulfentrazone or [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetic acid ethyl ester.

Mixtures of a compound of formula I with paraquat dichloride (614).

Mixtures of a compound of formula I with pendimethalin (621) or a compound of formula I with trifluralin (836). Particularly preferred are mixtures of a compound of formula I with pendimethalin.

Mixtures of a compound of formula I with metamitron (521).

Mixtures of a compound of formula I with clomazone (159).

Mixtures of a compound of formula I with metazachlor (524).

Mixtures of a compound of formula I with clodinafop-propargyl (156) or a compound of formula I with pinoxaden (CAS RN 243973-20-8).

The mixing partners of the compound of formula I may also be in the form of esters or salts, as mentioned e.g. in The Pesticide Manual, 13th Edition (BCPC), 2003. The reference to glufosinate-ammonium also applies to glufosinate, the reference to cloransulam-methyl also applies to cloransulam, and the reference to pyrithiobac-sodium also applies to pyrithiobac, etc.

The mixing ratio of the compound of formula I to the mixing partner is preferably from 1:100 to 1000:1.

The mixtures can advantageously be used in the above-mentioned formulations (in which case "active ingredient" relates to the respective mixture of compound of formula I with the mixing partner).

Furthermore, the compounds of formula I according to the invention can also be used in combination with other herbicides: compound of formula I + acetochlor (5), compound of formula I + acifluorfen-sodium (7), compound of formula I + aclonifen (8), compound of formula I + acrolein (10), compound of formula I + alachlor (14), compound of formula I + alloxydim (18), compound of formula I + allyl alcohol (CAS RN 107-18-6), compound of formula I + amidosulfuron (22), compound of formula I + aminopyralid (CAS RN 150114-71-9), compound of formula I + amitrole (aminotriazole) (25), compound of formula I + ammonium sulfamate (26), compound of formula I + anilofos (31), compound of formula I + asulam (36), compound of formula I + atraton (CAS RN 1610-17-9), compound of formula I + aviglycine (39), compound of formula I + azafenidin (CAS RN 68049-83-2), compound of formula I + azimsulfuron (43), compound of formula I + BAS 800H (CAS RN 372137-35-4), compound of formula I + BCPC (CAS RN 2164-13-8), compound of formula I + beflubutamid (55), compound of formula I + benazolin (57), compound of formula I + bencarbazone (CAS RN 173980-17-1), compound of formula I + benfluralin (59), compound of formula I + benfuresate (61), compound of formula I + bensulfuron-methyl (64), compound of formula I + bensulide (65), compound of formula I + bentazone (67), compound of formula I + benzfendizone (CAS RN 158755-95-4), compound of formula I + benzobicyclon (69), compound of formula I + benzofenap (70), compound of formula I + bifenox (75), compound of formula I + bilanafos (bialaphos) (77), compound of formula I + bispyribac-sodium (82), compound of formula I + borax (86), compound of formula I + bromacil (90), compound of formula I + bromobutide (93), compound of formula I + bromofenoxim (CAS RN 13181-17-4), compound of formula I + bromoxynil (95), compound of formula I + butachlor (100), compound of formula I + butafenacil (101), compound of formula I + butamifos (102), compound of formula I + butralin (105), compound of formula I + butroxydim (106), compound of formula I + butylate (108), compound of formula I + cacodylic acid (CAS RN 75-60-5), compound of formula I + calcium chlorate (CAS RN 10137-74-3), compound of formula I + cafenstrole (110), compound of formula I + carbetamide (117), compound of formula I + carfentrazone-ethyl (121), compound of formula I + CDEA (CAS RN 2315-36-8), compound of formula I + CEPC (CAS RN 587-56-4), compound of formula I + chlorbromuron (CAS RN 13360-45-7), compound of formula I + chlorflurenol-methyl (133), compound of formula I + chloridazon (134), compound of formula I + chlorimuron-ethyl (135), compound of formula I + chloroacetic acid (138), compound of formula I + chlorotoluron (143); compound of formula I + chlorpropham (144), compound of formula I + chlorthal-dimethyl (148), compound of formula I + cinidon-ethyl (152), compound of formula I + cinmethylin (153), compound of formula I + cisanilide (CAS RN 34484-77-0), compound of formula I + clefoxydim (CAS RN 211496-02-5), compound of formula I + clethodim (155), compound of formula I + clomeprop (160), compound of formula I + clopyralid (162), compound of formula I + CMA (CAS RN 5902-95-4), compound of formula I + 4-CPB (CAS RN 3547-07-7), compound of formula I + CPMF, compound of formula I + 4-CPP (CAS RN 3307-39-9), compound of formula I + CPPC (CAS RN 2150-32-5), compound of formula I + cresol (CAS RN 1319-77-3), compound of formula I + cumyluron (180), compound of formula I + cyanamide (182), compound of formula I + cyclanilide (186), compound of formula I + cycloate (187), compound of formula I + cyclosulfamuron (189), compound of formula I + cycloxydim (190), compound of formula I + cyhalofop-butyl (195), compound of formula I + 2,4-D (211), compound of formula I + 3,4-DA (CAS RN 588-22-7), compound of formula I + daimuron (213), compound of formula I + dalapon (214), compound of formula I + dazomet (216), compound of formula I + 2,4-DB (217), compound of formula I + 3,4-DB, compound of formula I + 2,4-DEB (CAS RN 94-83-7), compound of formula I + desmedipham (225), compound of formula I + desmetryn (CAS RN 1014-69-3), compound of formula I + dicamba (228), compound of formula I + dichlobenil (229), compound of formula I + ortho-dichlorobenzene (CAS RN 95-50-1), compound of formula I + para-dichlorobenzene (CAS RN 106-46-7), compound of formula I + dichlorprop (234), compound of formula I + dichlorprop-P (235), compound of formula I + diclofop-methyl (238), compound of formula I + diclosulam (241), compound of formula I + difenzoquat metilsulfate (248), compound of formula I + diflufenican (251), compound of formula I + diflufenzopyr (252), compound of formula I + dimefuron (256), compound of formula I + dimepiperate (257), compound of formula I + dimethachlor (258), compound of formula I + dimethenamid (260), compound of formula I + dimethenamid-P, compound of formula I + dimethipin (261), compound of formula I + dimethylarsinic acid (264), compound of formula I + dinitramine (268), compound of formula I + dinoterb (272), compound of formula I + diphenamid (274), compound of formula I + dipropetryn (CAS RN 4147-51-7), compound of formula I + diquat dibromide (276), compound of formula. I + dithiopyr (280), compound of formula I + diuron (281), compounds of formula I + DNOC (282), compound of formula I + 3,4-DP (CAS RN 3307-41-3), compound of formula I + DSMA (CAS RN 144-21-8), compound of formula I + EBEP, compound of formula I + endothal (295), compound of formula I + EPTC (299), compound of formula I + esprocarb (303), compound of formula I + ethalfluralin (305), compound of formula I + ethephon (307), compound of formula I + ethofumesate (311), compound of formula I + ethoxyfen (CAS RN 188634-90-4), compound of formula I + ethoxyfen-ethyl (CAS RN 131086-42-5), compound of formula I + ethoxysulfuron (314), compound of formula I + etobenzanid (318), compound of formula I + fenoxaprop-P-ethyl (339), compound of formula I + fentrazamide (348), compound of formula I + ferrous sulfate (353), compound of formula I + flamprop, compound of formula I + flamprop-M (355), compound of formula I + florasulam (359), compound of formula I + fluazifop-butyl (361), compound of formula I + fluazifop-P-butyl (362), compound of formula I + fluazolate (isopropazol) (CAS RN 174514-07-9), compound of formula I + flucarbazone-sodium (364), compound of formula I + flucetosulfuron (CAS RN 412928-75-7), compound of formula I + fluchloralin (365), compound of formula I + flufenacet (BAY FOE 5043) (369), compound of formula I + flufenpyr-ethyl (371), compound of formula I + flumetralin (373), compound of formula I + flumiclorac-pentyl (375), compound of formula I + flumipropyn (flumipropin) (CAS RN 84478-52-4), compound of formula I + fluometuron (378), compound of formula I + fluoroglycofen-ethyl (380), compound of formula I + flupoxam (CAS RN 119126-15-7), compound of formula I + flupropacil (CAS RN 120890-70-2), compound of formula I + flupropanate (383), compound of formula I + flupyrsulfuron-methyl-sodium (384), compound of formula I + flurenol (387), compound of formula I + fluridone (388), compound of formula I + flurochloridone (389), compound of formula I + fluroxypyr (390), compound of formula I + flurtamone (392), compound of formula I + fluthiacet-methyl (395), compound of formula I + fosamine (406), compound of formula I + halosulfuron-methyl (426), compound of formula I + haloxyfop (427), compound of formula I + haloxyfop-P (428), compound of formula I + HC-252 (429), compound of formula I + hexazinone (440), compound of formula I + imazaquin (454), compound of formula I + imazosulfuron (456), compound of formula I + indanofan (462), compound of formula I + iodomethane (CAS RN 74-88-4), compound of formula I + ioxynil (467), compound of formula I + isoproturon (475), compound of formula I + isouron (476), compound of formula I + isoxaben (477), compound of formula I + isoxachlortole (CAS RN 141112-06-3), compound of formula I + isoxapyrifop (CAS RN 87757-18-4), compound of formula I + karbutilate (482), compound of formula I + lactofen (486), compound of formula I + lenacil (487), compound of formula I + linuron (489), compound of formula I + MAA (CAS RN 124-58-3), compound of formula I + MAMA (CAS RN 2321-53-1), compound of formula I + MCPA (499), compound of formula I + MCPA-thioethyl (500), compound of formula I + MCPB (501), compound of formula I + mecoprop (503), compound of formula I + mecoprop-P (504), compound of formula I + mefenacet (505), compound of formula I + mefluidide (507), compound of formula I + mesosulfuron-methyl (514), compound of formula I + metam (519), compound of formula I + metamifop (mefluoxafop) (520), compound of formula I + methabenzthiazuron (526), compound of formula I + methazole (CAS RN 20354-26-1), compound of formula I + methylarsonic acid (536), compound of formula I + methyldymron (539), compound of formula I + methyl isothiocyanate (543), compound of formula I + metobenzuron (547), compound of formula I + metobromuron (CAS RN 3060-89-7), compound of formula I + metosulam (552), compound of formula I + metoxuron (553), compound of formula I + MK-616 (559), compound of formula I + molinate (560), compound of formula I + monolinuron (562), compound of formula I + MSMA (CAS RN 2163-80-6), compound of formula I + naproanilide (571), compound of formula I + napropamide (572), compound of formula I + naptalam (573), compound of formula I + neburon (574), compound of formula I + nipyraclofen (CAS RN 99662-11-0), compound of formula I + n-methyl-glyphosate, compound of formula I + nonanoic acid (583), compound of formula I + norflurazon (584), compound of formula I + oleic acid (fatty acids) (593), compound of formula I + orbencarb (595), compound of formula I + orthosulfamuron (CAS RN 213464-77-8), compound of formula I + oryzalin (597), compound of formula I + oxadiargyl (599), compound of formula I + oxadiazon (600), compound of formula I + oxaziclomefone (604), compound of formula I + oxyfluorfen (610), compound of formula I + pebulate (617), compound of formula I + penoxsulam (622), compound of formula I + pentachlorophenol (623), compound of formula I + pentanochlor (624), compound of formula I + pentoxazone (625), compound of formula I + pethoxamid (627), compound of formula I + petrolium oils (628), compound of formula I + phenmedipham (629), compound of formula I + picloram (645), compound of formula I + picolinafen. (646), compound of formula I + piperophos (650), compound of formula I + potassium arsenite (CAS RN 10124-50-2), compound of formula I + potassium azide (CAS RN 20762-80-1), compound of formula I + pretilachlor (656), compound of formula I + prodiamine (661), compound of formula I + profluazol (CAS RN 190314-43-3), compound of formula I + profoxydim (663), compound of formula I + prohexadione calcium (664), compound of formula I + propachlor (667), compound of formula I + propanil (669), compound of formula I + propaquizafop (670), compound of formula I + propham (674), compound of formula I + propisochlor (667), compound of formula I + propoxycarbazone-sodium (procarbazone-sodium) (679), compound of formula I + propyzamide (681), compound of formula I + prosulfocarb (683), compound of formula I + pyraclonil (pyrazogyl) (CAS RN 158353-15-2), compound of formula I + pyraflufen-ethyl (691), compound of formula I + pyrasulfotole (CAS RN 365400-11-9), compound of formula I + pyrazolynate (692), compound of formula I + pyrazosulfuron-ethyl (694), compound of formula I + pyrazoxyfen (695), compound of formula I + pyribenzoxim (697), compound of formula I + pyributicarb (698), compound of formula I + pyridafol (CAS RN 40020-01-7), compound of formula I + pyridate (702), compound of formula I + pyriftalid (704), compound of formula I + pyriminobac-methyl (707), compound of formula I + pyrimisulfan (CAS RN 221205-90-9), compound of formula I + pyroxasulfone (CAS RN 447399-55-5), compound of formula I + pyroxsulam (triflosulam) (CAS RN 422556-08-9), compound of formula I + quinclorac (712), compound of formula I + quinmerac (713), compound of formula I + quinoclamine (714), compound of formula I + quizalofop (717), compound of formula I + quizalofop-P (718), compound of formula I + sequestrene, compound of formula I + sethoxydim (726), compound of formula I + siduron (727), compound of formula I + SMA (CAS RN 3926-62-3), compound of formula I + sodium arsenite (CAS RN 7784-46-5), compound of formula I + sodium azide (CAS RN 26628-22-8), compound of formula I + sodium chlorate (734), compound of formula I + sulfometuron-methyl (751), compound of formula I + sulfosate (CAS RN 81591-81-3), compound of formula I + sulfuric acid (755), compound of formula I + tar oils (758), compound of formula I + 2,3,6-TBA (759), compound of formula I + TCA-sodium (760), compound of formula I + tebutam (CAS RN 35256-85-0), compound of formula I + tebuthiuron (765), compound of formula I + tepraloxydim (771), compound of formula I + terbacil (772), compound of formula I + tefuryltrione (CAS RN 473278-76-1), compound of formula I + thenylchlor (789), compound of formula I + thidiazimin (CAS RN 123249-43-4), compound of formula I + thiazafluron (CAS RN 25366-23-8), compound of formula I + thiazopyr (793), compound of formula I + thiobencarb (797), compound of formula I + tiocarbazil (807), compound of formula I + tralkoxydim (811), compound of formula I + tri-allate (816), compound of formula I + triaziflam (819), compound of formula I + tricamba (CAS RN 2307-49-5), compound of formula I + triclopyr (827), compound of formula I + triflusulfuron-methyl (837), compound of formula I + trihydroxytriazine (CAS RN 108-80-5), compound of formula I + trinexapac-ethyl (CAS RN 95266-40-3) and compound of formula I + tritosulfuron (843).

The mixing partners of the compound of formula I may also be in the form of esters or salts, as mentioned e.g. in The Pesticide Manual, 13th Edition (BCPC), 2003. The reference to acifluorfen-sodium also applies to acifluorfen, and the reference to bensulfuron-methyl also applies to bensulfuron, etc.

The mixing ratio of the compound of formula I to the mixing partner is preferably from 1:100 to 1000:1.

The mixtures can advantageously be used in the above-mentioned formulations (in which case "active ingredient" relates to the respective mixture of compound of formula I with the mixing partner).

The compounds of formula I according to the invention can also be used in combination with one or more safeners. Likewise, mixtures of a compound of formula I according to the invention with one or more further herbicides can also be used in combination with one or more safeners. The safeners can be AD 67 (MON 4660) (11), benoxacor (63), cloquintocet-mexyl (163), cyometrinil and the corresponding (Z) isomer, cyprosulfamide (CAS RN 221667-31-8), dichlormid (231), fenchlorazole-ethyl (331), fenclorim (332), flurazole (386), fluxofenim (399), furilazole (413) and the corresponding R isomer, isoxadifen-ethyl (478), mefenpyr-diethyl (506), oxabetrinil (598), naphthalic anhydride (CAS RN 81-84-5) and N-isopropyl-4-(2-methoxy-benzoylsulfamoyl)-benzamide (CAS RN 221668-34-4). Particularly preferred are mixtures of a compound of formula I with benoxacor (i.e. compound of formula I + benoxacor).

The safeners of the compound of formula I may also be in the form of esters or salts, as mentioned e.g. in The Pesticide Manual, 13th. Edition (BCPC), 2003. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048, and the reference to fenchlorazole-ethyl also applies to fenchlorazole, etc.

Preferably the mixing ratio of compound of formula I to safener is from 100:1 1 to :1:10, especially from 20:1 to 1:1.

The mixtures can advantageously be used in the above-mentioned formulations (in which case "active ingredient" relates to the respective mixture of compound of formula I with the safener).

Preferred mixtures of a compound of formula I with further herbicides and safeners include:

Mixtures of a compound of formula I with a triazine and a safener.

Mixtures of a compound of formula I with glyphosate and a safener.

Mixtures of a compound of formula I with glufosinate and a safener.

Mixtures of a compound of formula I with isoxaflutole and a safener.

Mixtures of a compound of formula I with isoxaflutole and a triazine and a safener.

Mixtures of a compound of formula I with isoxaflutole and glyphosate and a safener.

Mixtures of a compound of formula I with isoxaflutole and glufosinate and a safener.

Mixtures of a compound of formula I with mesotrione and a safener.

Mixtures of a compound of formula I with mesotrione and a triazine and a safener.

Mixtures of a compound of formula I with mesotrione and glyphosate and a safener.

Mixtures of a compound of formula I with mesotrione and glufosinate and a safener.

Mixtures of a compound of formula I with sulcotrione and a safener.

Mixtures of a compound of formula I with sulcotrione and a triazine and a safener.

Mixtures of a compound of formula I with sulcotrione and glyphosate and a safener.

Mixtures of a compound of formula I with sulcotrione and glufosinate and a safener.
The following Examples further illustrate the invention.

### Preparation Examples:

### 1) Methods for making 5,5-dimethyl-4,5-dihydro-isoxazole derivatives

### Example I1: Preparation of hydroxyimino-acetic acid

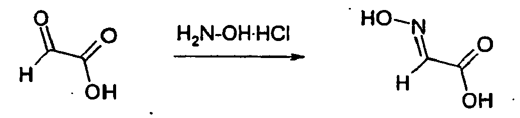

Aqueous glyoxylic acid (50% by weight) (11,9 mol) and hydroxyl amine hydrochloride (627 g, 9 mol) were mixed and concentrated. Acetonitrile (1 1) was added, the solution cooled to 5°C and filtered. The mother liquor was concentrated to 50% of the volume, stored at 5°C overnight and filtered again. This process was repeated twice. The solids were combined and dried to give hydroxyimino-acetic acid as white crystals (546 g, 68% yield).

### Example I2: Preparation of 3-chloro-5,5-dimethyl-4,5-dihydro-isoxazole

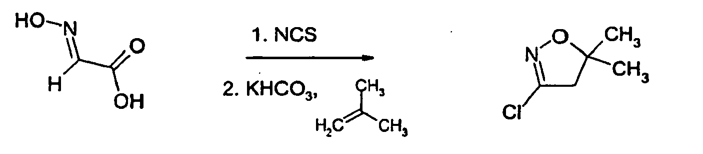

Hydroxyimino-acetic acid (Example I1) (107 g, 1.2 mol) was dissolved in 1,2-dimethoxy-ethane (1.4 1) and heated to 70°C. N-chlorosuccinimide (NCS) (320.4 g, 2.4 mol) was added in portions within 1 hour at 70°C. The reaction mixture was stirred at 70°C for 1 hour. The reaction mixture was cooled to 5°C and potassium hydrogencarbonate (535 g, 4.45 mol) and water (54 g) were added. 2-Methyl-propene (134.6 g, 2.4 mol) was introduced into the suspension for 20 minutes at 5°C. The reaction mixture was allowed to warm to room temperature and was stirred at room temperature for 18 hours. The reaction mixture was poured onto water (1.51) and the mixture extracted with hexane (3x 500 ml). The combined organic extracts were washed with brine, dried over sodium sulfate and concentrated. The residue was distilled to give 3-chloro-5,5-dimethyl-4,5-dihydro-isoxazole as a liquid (60.7 g, 37.8% yield).
¹H-NMR (400 MHz, CDCl₃): 1.47 (s, 6H, Me), 2.92 (s, 2H, CH₂) ppm.

### Example I3: Preparation of 5,5-dimethyl-isoxazolidin-3-one

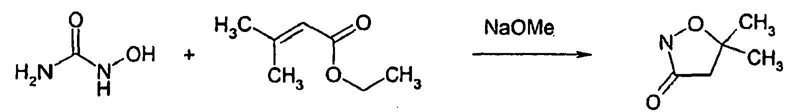

Sodium (1.72 g, 75 mmol) was dissolved in methanol (30 ml) at room temperature. Hydroxy urea (3.8 g, 50 mmol) was added slowly, then ethyl dimethylacrylate (6.4 g, 50 mmol) was added dropwise. The reaction mixture was stored at room temperature for 18 hours. The solid was removed by filtration and the filtrate was concentrated. The residue was dissolved in water and stirred for 15 minutes, then aqueous hydrochloric acid (2M) was added dropwise to acidify the mixture. The aqueous solution was extracted with chloroform. The organic extract was washed with brine, dried over magnesium sulfate and concentrated to give 5,5-dimethyl-isoxazolidin-3-one as a white solid (2.8 g, 49% yield).
¹H-NMR (400 MHz, CDCl₃): 1.43 (s, 6H, Me), 2.58 (s, 2H, CH₂) ppm.

### Example I4: Preparation of 3-chloro-5,5-dimethyl-4,5-dihydro-isoxazole

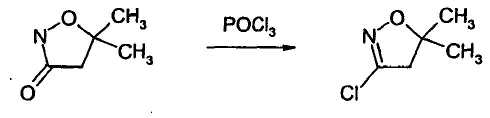

5,5-Dimethyl-isoxazolidin-3-one (Example I3) (0.5 g, 0.57 mmol) was dissolved in phosphorus oxychloride (10 ml) and heated to reflux for 5 hours. The reaction mixture was concentrated and the residue partitioned between water and diethyl ether. The phases were separated and the organic phase washed with brine, dried over magnesium sulfate and concentrated to give 3-chloro-5,5-dimethyl-4,5-dihydro-isoxazole as a brown, mobile oil (0.4 g, 69% yield).

### Example I5: Reparation of 2-(5,5-dimethyl-4,5-dihydro-isoxazol-3-yl)-disulfide

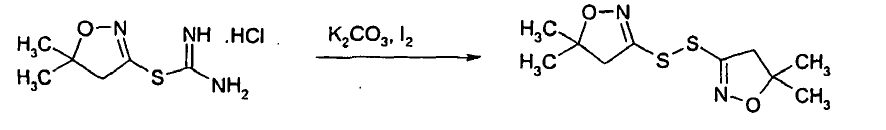

2-(5,5-Dimethyl-4,5-dihydro-isoxazol-3-yl)-isothiourea hydrochloride (29 g, 138 mmol) (prepared as described in WO 06/068092) and potassium carbonate (39 g, 416 mmol) were dissolved in methanol (250 ml) and iodine (18 g, 100 mmol) was added in portions at room temperature. The reaction mixture was stirred at room temperature for 1 hour. The reaction mixture was concentrated, the residue dissolved in water and the aqueous mixture extracted several times with ethyl acetate. The combined organic extracts were washed with water and brine, dried over magnesium sulfate and concentrated to give 2-(5,5-dimethyl-4,5-dihydro-isoxazol-3-yl)-disulfide as a pale yellow solid (14.1 g, 78 % yield).

### 2) Methods for making triazole derivatives

The synthesis of 1-*tert*-butyl-1*H-*[1,2,3]triazole-4-carboxylic acid ethyl ester was described in Synthesis, 1985, 178-180.

1-*tert*-Butyl-1*H*-[1,2,3]triazole-4-carboxylic acid ethyl ester was reduced as described in Example I6 to give [1-*ter*t-butyl-1*H*-[1,2,3]triazol-4-yl]-methanol. ¹H-NMR (400 MHz, CDCl₃): 1.65 (s, 9H, Me), 4.76 (s, 2H, CH₂), 7.60 (s, 1H, CH) ppm.

[1-*tert*-Butyl-1*H*-[1,2,3]triazol-4-yl]-methanol was brominated as described in Example I7 to give 4-bromomethyl-1-*tert*-butyl-1*H*-[1,2,3]triazole.
¹H-NMR (400 MHz, CDCl₃): 1.65 (s, 9H, Me), 4.58 (s, 2H, CH₂), 7.63 (s, 1H, CH) ppm.

The synthesis of 5-(difluoromethyl)-2-methyl-2*H*-1,2,3-triazole-4-carboxylic acid methyl ester was described in WO 2004/018438.

### Examples I6: Preparation of (5-fluoromethyl-2-methyl-2H-[1,2,3]triazol-4-yl)-methanol and (2,5-dimethyl-2H-[1,2,3]triazol-4-yl)-methanol

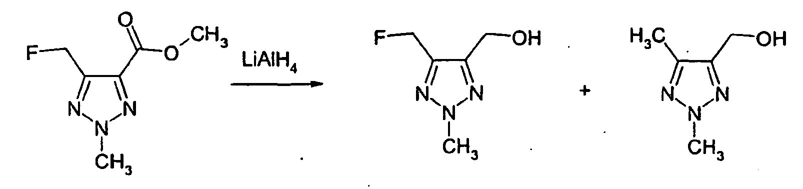

To a solution of lithium aluminium hydride (2M in THF) (2.83 ml, 5.65 mmol) in tetrahydrofuran (10 ml) was added dropwise a solution of 5-fluoromethyl-2-methyl-2*H-*[1,2,3]triazole-4-carboxylic acid methyl ester (prepared as described in WO 04/018438) (978 mg, 5.65 mmol) in tetrahydrofuran (10 ml) at 0°C. The reaction mixture was allowed to warm to room temperature and was stirred at room temperature for 40 minutes. The reaction mixture was quenched by successive addition of water (200µl), aqueous sodium hydroxide (2M) (350ul) and water (400µl). The precipitate was removed by filtration and the filtrate was evaporated to give a 4:1-mixture of (5-fluoromethyl-2-methyl-2*H-*[1,2,3]triazol-4-yl)-methanol and (2,5-dimethyl-2*H-*[1,2,3]triazol-4-yl)-methanol as a pale yellow liquid (610 mg, 76% yield).
¹H-NMR (400 MHz, CDCl₃): 4.19 (d, 3H, Me), 4.83 (s, 2H, CH₂), 5.52 (d, 2H, CH₂) ppm.
¹H-NMR (400 MHz, CDCl₃): 2.31 (s, 3H, Me), 4.11 (s, 3H, Me), 4.72 (s, 2H, CH₂) ppm.

The same method was used with 2-methyl-5-trifluoromethyl-2*H-*[1,2,3]triazole-4-carboxylic acid ethyl ester (prepared as described in WO 04/018438) as the starting material to give (2-methyl-5-trifluoromethyl-2*H*-[1,2,3]triazol-4-yl)-methanol. ¹H-NMR (400 MHz, CDCl₃): 2.03 (t, 1H, OH), 4.24 (s, 3H, Me), 4.84 (d, 2H, CH₂) ppm.

### Example I7: Preparation of 4-bromomethyl-5-fluoromethyl-2-methyl-2H-[1,2,3]triazole and 4-bromomethyl-2,5-dimethyl-2H-[1,2.3]triazole

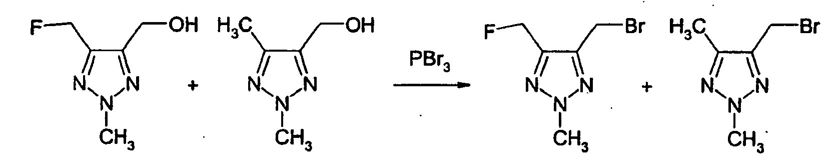

The 4:1-mixture of (5-fluoromethyl-2-methyl-2*H*-[1,2,3]triazol-4-yl)-methanol and (2,5-dimethyl-2*H*-[1,2,3]triazol-4-yl)-methanol (610 mg, 4.21 mmol) (Example I6) was dissolved in diethyl ether (10 ml) under nitrogen at room temperature and phosphorous tribromide (395µl, 4.21 mmol) was added. The reaction mixture was stirred at room temperature for 24 hours. The reaction mixture was quenched by addition of cold water, and the mixture was extracted three times with ethyl acetate. The combined organic extracts were washed with brine, dried over magnesium sulfate and concentrated to give a 4:1-mixture of 4-bromomethyl-5-fluoromethyl-2-methyl-2*H*-[1,2,3]triazole and 4-bromomethyl-2,5-dimethyl-2*H*-[1,2,3]-triazole as an orange liquid (954 mg).
¹H-NMR (400 MHz, CDCl₃): 4.18 (d, 3H, Me), 4.57 (s, 2H, CH₂), 5:51 (d, 2H, CH₂) ppm.
¹H-NMR (400 MHz, CDCl₃): 2.31 (s, 3H, Me), 4.11 (s, 3H, Me), 4.50 (s, 2H, CH₂) ppm.

The same method was used with (2-methyl-5-trifluoromethyl-2*H*-[1,2,3]triazol-4-yl)-methanol as the starting material to give 4-bromomethyl-2-methyl-5-trifluoromethyl-2*H*-[1,2,3]triazole.
¹H-NMR (400 MHz, CDCl₃): 4.19 (s, 3H, Me), 4.49 (s, 2H, CH₂) ppm.

### Example I8: Preparation of 3-(methyl-hydrazono)-butan-2-one oxime

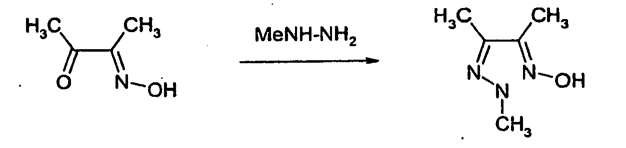

Butane-2,3-dione monooxime (2.6 g, 25.8 mmol) was dissolved in ethanol (100 ml) then methylhydrazine (1.13 g, 24.5 mmol) was added at room temperature. The reaction mixture was stirred at 80°C for 2 hours. Methylhydrazine (0.6 g, 12 mmol) was added and the reaction mixture stirred at 80°C for 1.5 hours. Methylhydrazine (0.6 g, 12 mmol) was added and the reaction mixture stirred at 80°C for another 1.5 hours, then at room temperature for 48 hours. The reaction mixture was concentrated to give 3-(methyl-hydrazono)-butan-2-one oxime as a pale yellow, crystalline solid (3.3 g, 100% yield). ¹H-NMR (400 MHz, CDCl₃):1.89 (s, 3H, Me), 2.11 (s, 3H, Me), 3.09 (s, 3H, Me), 4.8-5.3 (bs, 1H, OH) ppm.

### Example I9: Preparation of 2,4,5-trimethyl-2H-[1,2,3]triazole 1-oxide

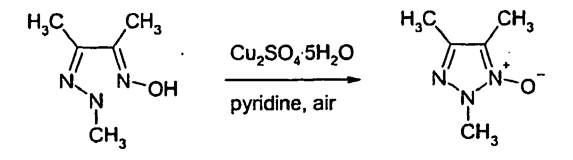

3-(Methyl-hydrazono)-butan-2-one oxime (3.3-g, 25.8 mmol) (Example I8) was dissolved in tetrahydrofuran (135 ml), then aqueous pyridine (15%) (157 ml, 0.29 mol) was added, followed by a slurry of copper(II)-sulfate (15.7 g, 62.8 mmol) in water (35 ml). The reaction mixture was stirred at 80°C for 2 hours, then cooled to room temperature and extracted three times with ethyl acetate. The combined organic extracts were washed with aqueous copper(II) sulfate (10%), dried over magnesium sulfate and concentrated. The residue was purified by column chromatography on silica gel (eluent: methanol / dichloromethane) to give 2,4,5-trimethyl-2*H*-[1,2,3]triazole 1-oxide) as colourless liquid (0.7 g, 21% yield).
¹H-NMR (400 MHz, CDCl₃): 2.19 (s, 3H, Me), 2.23 (s, 3H, Me), 3.93 (s, 3H, Me) ppm.

### Example I10: Preparation of 5-bromomethyl-2,4-dimethyl-2H-[1,2,3]triazole 1-oxide

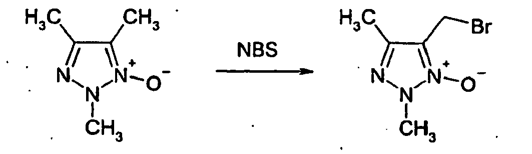

N-Bromosuccinimide (NBS) (186 mg, 1.0 mmol) and 2,2'-azobisisobutyronitrile (AIBN) (14 mg, 0.087 mmol) were added to a solution of 2,4,5-trimethyl-2*H*-[1,2,3]-triazole 1-oxide (110 mg, 0.87 mmol) (Example I9) in carbon tetrachloride (7 ml). The reaction mixture was heated to 70°C for 1 hour then cooled to room temperature and filtered. The solvent was removed to give 5-bromomethyl-2,4-dimethyl-2*H*-[1,2,3]-triazole 1-oxide as a brown gum.
¹H-NMR (400 MHz, CDCl₃): 2.31 (s, 3H, Me), 3.95 (s, 3H, Me), 4.42 (s, 2H, CH₂) ppm.

### Example I11: Alternative preparation of 4-bromomethyl-2,5-dimethyl-2H-[1,2,3]triazole

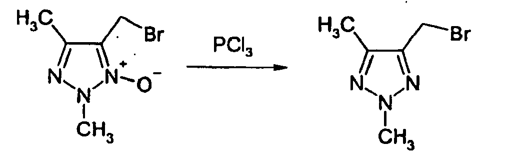

5-Bromomethyl-2,4-dimethyl-2*H*-[1,2,3]triazole 1-oxide (4.83 g, 23.3 mmol) (Example I10) was dissolved in carbon tetrachloride (50 ml), then phosphorous trichloride (6.8 ml, 77.8 mol) was added dropwise. The reaction mixture was stirred at 75°C for 2.5 hours. The reaction mixture was quenched by slow addition into hot water, then cooled and diluted with cold water. The phases were separated and the aqueous phase was extracted twice with dichloromethane. The combined organic extracts were washed with water and brine, dried over magnesium sulfate and concentrated. The residue was purified by column chromatography on silica gel (eluent: methanol / dichloromethane) to give 4-bromomethyl-2,5-dimethyl-2*H*-[1,2,3]triazole as a yellow oil (1.47 g, 33% yield). ¹H-NMR (400 MHz, CDCl₃): 2.3 (s, 3H, Me), 4.1 (s, 3H, Me), 5.5 (s, 2H, CH₂) ppm.

### Example 112: Preparation of 1-(4-methoxy-benzyl)-5-methyl-1H-[1,2,3]triazole-4-carboxylic acid methyl ester and 3-(4-methoxy-benzyl)-5-methyl-3H-[1,2,3]triazole-4-carboxylic acid methyl ester

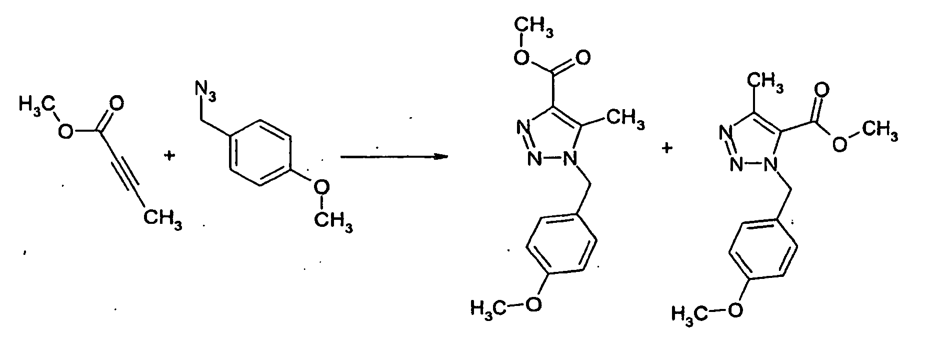

4-Methoxybenzyl azide (preparation described in e.g. J. Chem. Soc., Perkin Trans. 1, 1982 (2), 627-630) (5.47 g, 33.6 mmol) was dissolved in toluene (20 ml) and methyl-2-butynoate (6.72 ml, 67.1 mmol) was added dropwise at room temperature over 5 minutes. The reaction mixture was heated to 100°C for 14 hours. The solution was cooled to room temperature and the solvent was evaporated to yield a bright yellow liquid which was purified by column chromatography on silica gel (eluent: 0-50% ethyl acetate in hexane) to give a mixture of 1-(4-methoxy-benzyl)-5-methyl-1*H-*[1,2,3]-triazole-4-carboxylic acid methyl ester and 3-(4-methoxy-benzyl)-5-methyl-3*H-*[1,2,3]-triazole-4-carboxylic acid methyl ester as a bright yellow oil (7.35 g, 85% yield). Isomer 1 (major) ¹H*-*NMR (400 MHz, CDCl₃): 2.46 (s, 3H, Me), 3.79 (s, 3H, Me), 3.94 (s, 3H, Me), 5.47 (s, 2H, CH₂), 6.82-6.88 (m, 2H, CH), 7.13 (d, 2H, CH) ppm. Isomer 2 (minor) ¹H-NMR (400 MHz, CDCl₃): 2.51 (s, 3H, Me), 3.77 (s, 3H, Me), 3.90 (s, 3H, Me), 5.80 (s, 2H, CH₂), 6.82-6.88(m, 2H, CH), 7.28 (d, 2H, CH) ppm.

The same method was used with pent-2-ynoic acid ethyl ester as the starting material to give a mixture of 5-ethyl-1-(4-methoxy-benzyl)-1*H-*[1,2,3]triazole-4-carboxylic acid ethyl ester and 5-ethyl-3-(4-methoxy-benzyl)-3*H-*[1,2,3]triazole-4-carboxylic acid ethyl ester.
Isomer 1 (major) ¹H-NMR (400 MHz, CDCl₃): 1.00 (t, 3H, Me), 1.42 (t, 3H, Me), 2.88-2.96 (m, 2H, CH₂), 3.79 (s, 3H, Me), 4.42 (q, 2H, CH₂), 5.48 (s, 2H, CH₂), 6.82-6.87 (m, 2H, CH), 7.14 (d, 2H, CH) ppm.
Isomer 2 (minor) ¹H-NMR (400 MHz, CDCl₃): 1.29 (t, 3H, Me), 1.36 (t, 3H, Me), 2.88-2.96 (m, 2H, CH₂), 3.78 (s, 3H, Me), 4.35 (q, 2H, CH₂), 5.81 (s, 2H, CH₂), 6.82-6.87 (m, 2H, CH), 7.28 (d, 2H, CH) ppm.

### Example I13: Preparation of 5-methyl-2H-[1,2,3]triazole-4-carboxylic acid methyl ester

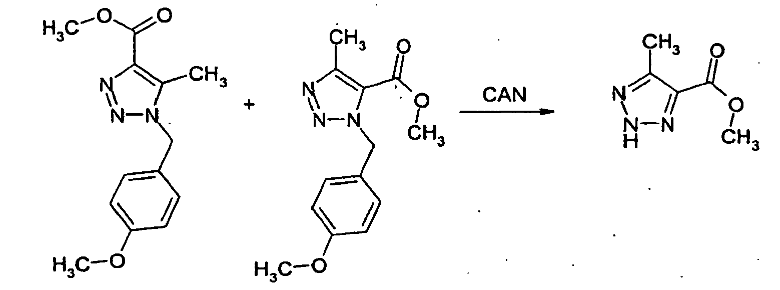

The mixture of 1-(4-methoxy-benzyl)-5-methyl-1*H-*[1,2,3]triazole-4-carboxylic acid methyl ester and 3-(4-methoxy-benzyl)-5-methyl-3*H-*[1,2,3]triazole-4-carboxylic acid methyl ester (Example I12) (7.35 g, 28.6 mmol) was dissolved in acetonitrile / water (9:1) (100 ml) and cerium(IV) ammonium nitrate (CAN) (31.4 g, 57.2 mmol) was added. The solution was stirred at room temperature for 16 hours. The solvent was evaporated and the residue partitioned between water and ethyl acetate. The phases were separated and the aqueous phase was extracted twice with more ethyl acetate. The combined organic extracts were washed with brine, dried over magnesium sulfate and concentrated. Dichloromethane (50 ml) was added to the residue which caused a white solid to precipitate (1.77 g, 40% yield). The solid was isolated via filtration. The mother liquor was evaporated and the residue again treated with dichloromethane (50 ml). This caused more white solid to precipitate. The solid was isolated via filtration and the combined solids were dried to give 5-methyl-2*H-*[1,2,3]triazole-4-carboxylic acid methyl ester (2.6 g, 59% yield).
¹H-NMR (400 MHz, CDCl₃): 2.62 (s, 3H, Me), 4.0 (s, 3H, Me) ppm.

The same method was used with the mixture of 5-ethyl-1-(4-methoxy-benzyl)-1*H*-[1,2,3]triazole-4-carboxylic acid ethyl ester and 5-ethyl-3-(4-methoxy-benzyl)-3*H-*[1,2,3]triazole-4-carboxylic acid ethyl ester as the starting material to give 5-ethyl-2*H-*[1,2,3]triazole-4-carboxylic acid ethyl ester.
¹H-NMR (400 MHz, CDCl₃): 1.36 (t, 3H, Me), 1.42 (t, 3H, Me), 3.08 (q, 2H, CH₂), 4.44 (q, 2H, CH₂) ppm.

### Example I14: Preparation of 2-ethyl-5-methyl-2H-[1,2,3]triazole-4-carboxylic acid methyl ester 3-ethyl-5-methyl-3H-[1,2,3]triazole-4-carboxylic acid methyl ester and 1-ethyl-5-methyl-1H-[1,2,3]triazole-4-carboxylic acid methyl ester

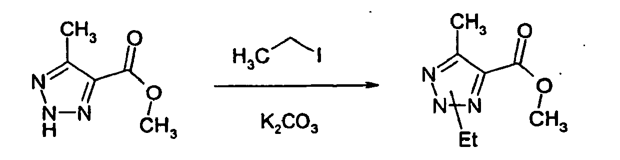

5-Methyl-2*H-*[1,2,3]triazole-4-carboxylic acid methyl ester (1.77 g, 12.6 mmol) (Example I13) was dissolved in N,N-dimethylformamide (12 ml) and ethyl iodide (16.3 mmol, 1.31 ml) and potassium carbonate (2.26 g, 16.3 mmol) were added at 0°C. The reaction mixture was warmed to room temperature and stirred for 5 hours under an atmosphere of nitrogen. The solution was partitioned between water and diethyl ether. The phases were separated and the aqueous phase was extracted twice with more diethyl ether. The combined organic extracts were washed with brine, dried over magnesium sulfate and concentrated to give a mixture of 2-ethyl-5-methyl-2*H-*[1,2,3]triazole-4-carboxylic acid methyl ester, 3-ethyl-5-methyl-3*H-*[1,2,3]triazole-4-carboxylic acid methyl ester and 1-ethyl-5-methyl-3*H-*[1,2,3]triazole-4-carboxylic acid methyl ester as a yellow oil (1.10 g, 52% yield).
Isomer 1 (major) ¹H-NMR (400 MHz, CDCl₃): 1.6 (t, 3H, Me), 2.52 (s, 3H, Me), 3.95 (s, 3H, Me), 4.45 (q, 2H, CH₂) ppm.
Isomer 2 (middle) ¹H-NMR (400 MHz, CDCl₃): 1.5 (t, 3H, Me), 2.54 (s, 3H, Me), 3.96 (s, 3H, Me), 4.73 (q, 2H, CH₂) ppm.
Isomer 3 (minor) ¹H-NMR (400 MHz, CDCl₃): 1.53 (t, 3H, Me), 2.6 (s, 3H, Me), 3.95 (s, 3H, Me), 4.35 (q, 2H, CH₂) ppm.

The same method was used with 5-methyl-2*H-*[1,2,3]triazole-4-carboxylic acid ethyl ester as the starting material (prepared as described in Example I12 from ethyl-2-butynoate, followed by deprotection as described in Example I13) and *iso*-propyl iodide as reagent to give 2-*iso-*propyl-5-methyl-2*H-*[1,2,3]triazole-4-carboxylic acid ethyl ester, 3-*iso*-propyl-5-methyl-3*H-*[1,2,3]triazole-4-carboxylic acid ethyl ester and 1-*iso-*propyl-5-methyl-1*H-*[1,2,3]triazole-4-carboxylic acid ethyl ester.
Isomer 1 (major) ¹H-NMR (400 MHz, CDCl₃): 1.38-1.44 (m, 3H, Me), 1.56-1.63 (m, 6H, Me), 2.52 (s, 3H, Me), 4.31-4.45 (m, 2H, CH₂), 4.83 (sept, 1H, CH) ppm.
Isomer 2 (middle) ¹H-NMR (400 MHz, CDCl₃): 1.38-1.44 (m, 3H, Me), 1.56-1.63 (m, 6H, Me), 2.53 (s, 3H, Me), 4.31-4.45 (m, 2H, CH₂), 5.44 (sept, 1H, CH) ppm.
Isomer 3 (minor) ¹H-NMR (400 MHz, CDCl₃): 1.38-1.44 (m, 3H, Me), 1.56-1.63 (m, 6H, Me), 2.59 (s, 3H, Me), 4.31-4.45 (m, 2H, CH₂), 4.58 (sept, 1H, CH) ppm.

The same method was used with 5-ethyl-2*H-*[1,2,3]triazole-4-carboxylic acid ethyl ester as the starting material and methyl iodide as reagent to give 5-ethyl-2-methyl-2*H-*[1,2,3]triazole-4-carboxylic acid ethyl ester.
¹H-NMR (400 MHz, CDCl₃): 1.32 (t, 3H, Me), 1.41 (t, 3H, Me), 2.95 (q, 2H, CH₂), 4.20 (s, 3H, Me), 4.42 (q, 2H, CH₂) ppm.

The same method was used with 5-ethyl-2*H-*[1,2,3]triazole-4-carboxylic acid ethyl ester as the starting material and ethyl iodide as reagent to give 2,5-diethyl-2*H-*[1,2,3]triazole-4-carboxylic acid ethyl ester.
¹H-NMR (400 MHz, CDCl₃): 1.2-1.30(m, 3H, Me), 1.35-1.45 (m, 3H, Me), (1.41 (t, 3H, Me), 2.95 (q, 2H, CH₂), 4.38-4.44 (m, 4H, CH₂) ppm.

The esters were reduced as described in Example 16, the alcohols brominated as described in Example 17, and the bromomethyl intermediates coupled as described in Example 139.

### Example I15: Preparation of 2-difluoromethyl-5-methyl-2H-[1,2,3]triazole-4-carboxylic acid ethyl ester and 3-difluoromethyl-5-methyl-3H-[1,2,3]triazole-4-carboxylic acid ethyl ester

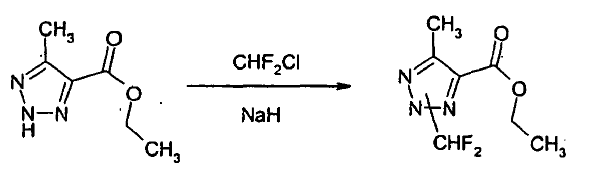

Sodium hydride (60% dispersion in mineral oil) (570 mg, 14.2 mmol) was washed under nitrogen with *iso*-hexane and suspended in dry tetrahydrofuran (50 ml) in a three necked flask fitted with a dry ice condenser. A solution of 5-methyl-2*H-*[1,2,3]-triazole-4-carboxylic acid ethyl ester (2 g, 12.9 mmol) (Example I13) in tetrahydrofuran (10 ml) was added dropwise. The reaction mixture was stirred at room temperature for 30 minutes. Chlorodifluoromethane was passed through the solution for 10 minutes and the reaction mixture stirred at room temperature for 1.5 hours. More chlorodifluoromethane was passed through the solution for a few minutes and the reaction mixture stirred for another 1.5 hours. The reaction was quenched by addition of water and the mixture was extracted with ethyl acetate. The combined organic extracts were washed with water and brine, dried over magnesium sulfate and concentrated. The residue was purified by column chromatography on silica gel (eluent: ethyl acetate / *iso*-hexane) to give 2-difluoromethyl-5-metyl-2*H-*[1,2,3]triazole-4-carboxylic acid ethyl ester (1.3 g, 49% yield) and a 1:2-mixture of 2-difluoromethyl-5-methyl-2*H-*[1,2,3]triazole-4-carboxylic acid ethyl ester and 3-difluoromethyl-5-methyl-3*H-*[1,2,3]triazole-4-carboxylic acid ethyl ester (250 mg, 9% yield).
Isomer 1 (major) ¹H-NMR (400 MHz, CDCl₃): 1.44 (t, 3H, Me), 2.80 (s, 3H, Me), 4.47 (q, 2H, CH₂), 7.64 (t, 1H, CHF₂) ppm.
Isomer 2 (minor) ¹H-NMR (400 MHz, CDCl₃): 1.45 (t, 3H, Me), 2.60 (s, 3H, Me), 4.47 (q, 2H, CH₂), 7.33 (t, 1H, CHF₂) ppm.

The esters were reduced as described in Example 16, the alcohols brominated as described in Example 17, and the bromomethyl intermediates coupled as described in Example 139.

### Example I16: Preparation of 1-(4-methoxy-benzyl)-5-trifluoromethyl-1H-[1,2,3]triazole-4-carboxylic acid ethyl ester and 3-(4-methoxy-benzyl)-5-trifluoromethyl-3H-[1,2,3]-triazole-4-carboxylic acid ethyl ester

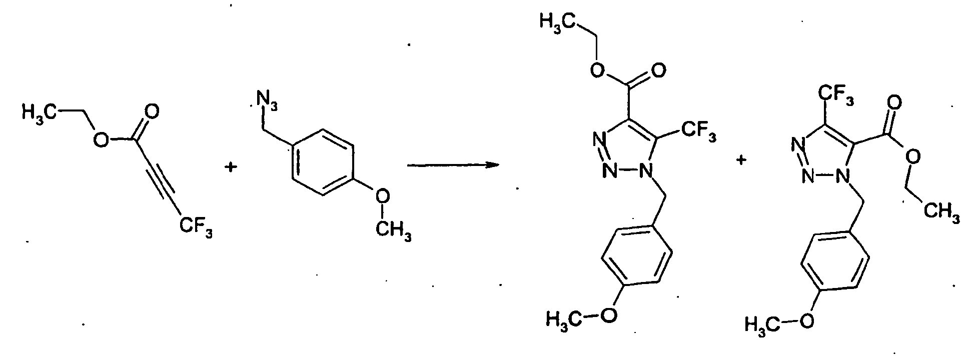

Ethyl 4,4,4-trifluoro-2-butynecarboxylate (prepared according to Organic Syntheses, 1992, 70, 246-55) (1.65 g, 10.1 mmol) was added to a solution of 4-methoxybenzyl azide (preparation described in e.g. J. Chem. Soc., Perkin Trans. 1, 1982 (2), 627-630) (1.65 g, 9.9 mmol) in toluene (10 ml) and the reaction mixture was stirred at room temperature for 16 hours. The mixture was concentrated and the residue purified by column chromatography on silica gel (eluent: ethyl acetate / *iso*-hexane) to give a 1.4 : 1 mixture of 1-(4-methoxy-benzyl)-5-trifluoromethyl-1*H-*[1,2,3]triazole-4-carboxylic acid ethyl ester and 3-(4-methoxy-benzyl)-5-trifluoromethyl-3*H-*[1,2,3]triazole-4-carboxylic acid ethyl ester (2.85 g, 87% yield).
Isomer 1 (minor) ¹H-NMR (400 MHz, CDCl₃): 1.4 (t, 3H, Me), 3.8 (s, 3H, Me), 4.4 (q, 2H, CH₂), 5.85 (s, 2H, CH₂), 6.9 (d, 2H, CH), 7.35 (d, 2H, CH) ppm.
Isomer 2 (major) ¹H-NMR (400 MHz, CDCl₃): 1.4 (t, 3H, Me), 3.8 (s, 3H, Me), 4.45 (q, 2H, CH₂), 5.7 (s, 2H, CH₂), 6.85 (d, 2H, CH), 7.2 (d, 2H, CH) ppm.

### Example I17: Preparation of 5-trifluoromethyl-2H-[1,2,3]triazole-4-carboxylic acid ethyl ester

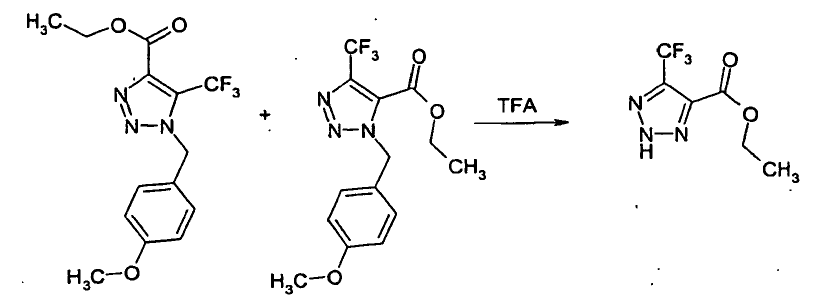

The 1.4 : 1 mixture of 1-(4-methoxy-benzyl)-5-trifluoromethyl-1*H-*[1,2,3]triazole-4-carboxylic acid ethyl ester and 3-(4-methoxy-benzyl)-5-trifluoromethyl-3*H-*[1,2,3]triazole-4-carboxylic acid ethyl ester (2.85 g, 8.66 mmol) (Example 116) was dissolved in trifluoroacetic acid (TFA) (15 ml) and the mixture was heated to 60°C for 2 hours. The reaction mixture was concentrated and the residue purified by column chromatography on silica gel (eluent: methanol / dichloromethane) to give 5-trifluoromethyl-2*H-*[1,2,3]triazole-4-carboxylic acid ethyl ester (1.75 g, 96.6% yield). ¹H-NMR (400 MHz, CDCl₃): 1.4 (t, 3H, Me), 4.5 (q, 2H, CH₂), 12.2-12.6 (bs, 1H, NH) ppm.

### Example I18: Preparation of 2-methyl-5-trifluoromethyl-2H-[1,2,3]triazole-4-carboxylic acid ethyl ester 3-methyl-5-trifluoromethyl-3H-[1,2,3]triazole-4-carboxylic acid ethyl ester and 1-methyl-5-trifluoromethyl-1H-[1,2,3]triazole-4-carboxylic acid ethyl ester

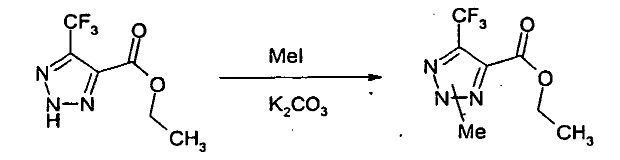

5-Trifluoromethyl-2*H-*[1,2,3]triazole-4-carboxylic acid ethyl ester (2.1 g, 10.0 mmol) (Example I17) was dissolved in acetonitrile (15 ml) and methyl iodide (1.12ml, 12.0 mmol) and potassium carbonate (2.76 g, 20.0 mmol) were added at room temperature. The reaction mixture was stirred at room temperature for 16 hours. The reaction mixture was diluted with dichloromethane (50 ml) and water (50 ml). The phases were separated and the aqueous phase was extracted several times with dichloromethane. The combined organic extracts were dried over magnesium sulfate and concentrated. The residue was purified by column chromatography on silica gel (eluent: ethyl acetate / *iso-*hexane) to give a 64:30:6 mixture of 2-methyl-5-trifluoromethyl-2*H-*[1,2,3]triazole-4-carboxylic acid ethyl ester, 3-methyl-5-trifluoromethyl-3*H-*[1,2,3]triazole-4-carboxylic acid ethyl ester and 1-methyl-5-trifluoromethyl-1*H-*[1,2,3]triazole-4-carboxylic acid ethyl ester (1.71 g, 76% yield).
Isomer 1 (major) ¹H-NMR (400 MHz, CDCl₃): 1.4 (t, 3H, Me), 4.32 (s, 3H, Me), 4.45 (q, 2H, CH₂) ppm.
Isomer 2 (middle) ¹H-NMR (400 MHz, CDCl₃): 1.4 (t, 3H, Me), 4.38 (s, 3H, Me), 4.45 (q, 2H, CH₂) ppm.
Isomer 2 (minor) ¹H-NMR (400 MHz, CDCl₃): 1.4 (t, 3H, Me), 4.30 (m, 3H, Me), 4.45 (q, 2H, CH₂) ppm.

The same method was used with ethyl iodide as reagent to give 2-ethyl-5-trifluoromethyl-2*H-*[1,2,3]triazole-4-carboxylic acid ethyl ester and 3-ethyl-5-trifluoromethyl-3*H-*[1,2,3]triazole-4-carboxylic acid ethyl ester (3.4:1 mixture).
Isomer 1 (major) ¹H-NMR (400 MHz, CDCl₃): 1.4 (t, 3H, Me), 1.65 (t, 3H, Me), 4.45 (q, 2H, CH₂), 4.6 (q, 2H, CH₂) ppm.
Isomer 2 (minor) ¹H-NMR (400 MHz, CDCl₃): 1.4 (t, 3H, Me), 1.55 (t, 3H, Me), 4.45 (q, 2H, CHF₂), 4.8 (q, 2H, CH₂) ppm.

The same method was used with *iso-*propyl iodide as reagent to give 2*-iso-*propyl-5-trifluoromethyl-2*H-*[1,2,3]triazole-4-carboxylic acid ethyl ester and 3*-iso-*propyl-5-trifluoromethyl-3*H-*[1,2,3]triazole-4-carboxylic acid ethyl ester (4.5:1 mixture). Isomer 1 (major) ¹H-NMR (400 MHz, CDCl₃): 1.4 (t, 3H, Me), 1.65 (d, 6H, Me), 4.45 (q, 2H, CH₂), 4.95-4.5 (m, 1H, CH) ppm.
Isomer 2 (minor) ¹H-NMR (400 MHz, CDCl₃): 1.4 (t, 3H, Me), 1.7 (d, 6H, Me), 4.45 (q, 2H, CH₂), 5.4-5.5 (m, 1H, CH) ppm.

The same method was used with cyclopentyl iodide as reagent to give 2-cyclopentyl-5-trifluoromethyl-2*H-*[1,2,3]triazole-4-carboxylic acid ethyl ester.
¹H-NMR (400 MHz, CDCl₃): 1.4 (t, 3H, Me), 1.7-1.8 (m, 2H, CH₂), 1.9-2.0 (m, 2H, CH₂), 2.2-2.3 (m, 4H, CH₂), 4.45 (q, 2H, CH₂), 5.1 (m, 1H, CH) ppm.

The same method was used with allyl iodide as reagent to give 2-allyl-5-tri-fluoromethyl-2*H-*[1,2,3]triazole-4-carboxylic acid ethyl ester and 3-allyl-5-trifluoromethyl-3*H-*[1,2,3]triazole-4-carboxylic acid ethyl ester (4:1 mixture).
Isomer 1 (major): ¹H-NMR (400 MHz, CDCl₃): 1.4 (t, 3H, Me), 4.45 (q, 2H, CH₂), 5.1 (d, 2H, CH₂), 5.35-5.45 (m, 2H, CH₂), 6.0-6.1 (m, 1H, CH) ppm.

The same method was used with 2-methoxy-ethyl iodide as reagent to give 2-(2-methoxy-ethyl)-5-trifluoromethyl-2*H-*[1,2,3]triazole-4-carboxylic acid ethyl ester and 3-(2-methoxy-ethyl)-5-trifluoromethyl-3*H-*[1,2,3]triazole-4-carboxylic acid ethyl ester (4:1 mixture).
Isomer 1 (major) ¹H-NMR (400 MHz, CDCl₃): 1.4 (t, 3H, Me), 3.35 (s, 3H, Me), 3.9 (t, 2H, CH₂), 4.45 (q, 2H, CH₂), 4.7 (t, 2H, CH₂) ppm.
Isomer 2 (minor) ¹H-NMR (400 MHz, CDCl₃): 1.4 (t, 3H, Me), 3.3 (s, 3H, Me), 3.8 (t, 2H, CH₂), 4.45 (q, 2H, CHF), 4.95 (t, 2H, CH₂) ppm.

The same method was used with cyclobutylmethyl iodide as reagent to give 2-(cyclobutyl-methyl)-5-trifluorometllyl-2*H-*[1,2,3]triazole-4-carboxylic acid ethyl ester and 3-(cyclobutyl-methyl)-5-trifluoromethyl-3*H-*[1,2,3]triazole-4-carboxylic acid ethyl ester (4:1 mixture).
Isomer 1 (major) ¹H-NMR (400 MHz, CDCl₃): 1.4 (t, 3H, Me), 1.8-2.0 (m, 4H, CH₂), 2.0-2.15 (m, 2H, CH₂), 2.95-3.1 (m, 1H, CH), 4.55 (q, 2H, CH₂), 4.65 (d, 2H, CH₂) ppm.
Isomer 2 (minor) ¹H-NMR (400 MHz, CDCl₃): 1.4 (t, 3H, Me), 1.8-2.0 (m, 4H, CH₂), 2.0-2.15 (m, 2H, CH₂), 2.85-2.95 (m, 1H, CH), 4.55 (q, 2H, CHF), 4.78 (d, 2H, CH₂) ppm.

The esters were reduced as described in Example 16, the alcohols brominated as described in Example 17, and the bromomethyl intermediates coupled as described in Example 139.

### Example I19: Preparation of 2-methyl-2H-[1,2,3]triazole-4-carboxylic acid methyl ester and 3-methyl-3H-[1,2,3]triazole-4-carboxylic acid methyl ester

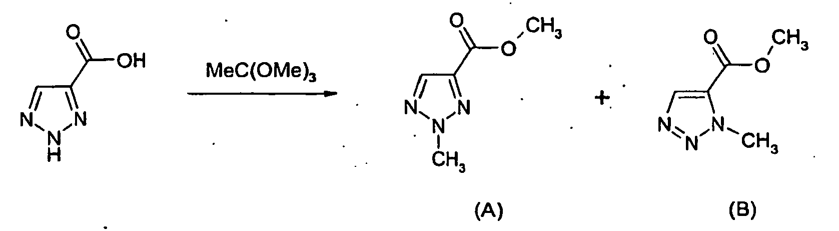

To a solution of 2*H-*[1,2,3]triazole-4-carboxylic acid (734 mg, 6.5 mmol) in toluene (10 ml), was added trimethylorthoacetate (2.48 ml, 19.5 mmol). The reaction mixture was heated at reflux for 16 hours. The reaction mixture was allowed to cool to room temperature and was concentrated. The residue was purified by column chromatography on silica gel (eluent: 10-30% ethyl acetate in hexane) to give 2-methyl-2*H-*[1,2,3]triazole-4-carboxylic acid methyl ester (isomer A) (40 mg, 4% yield) and 3-methyl-3*H-*[1,2,3]triazole-4-carboxylic acid methyl ester (isomer B) (223 mg, 24% yield).
Isomer A ¹-NMR (400 MHz, CDCl): 3.96 (s, 3H, Me), 4.28 (s, 3H, Me), 8.05 (s, 1H, CH) ppm.

The same method was used with triethylorthoacetate as reagent to give 2-ethyl-2*H-*[1,2,3]triazole-4-carboxylic acid methyl ester (isomer A) and 3-ethyl-3*H-*[1,2,3]-triazole-4-carboxylic acid methyl ester (isomer B).
Isomer A ¹-NMR (400 MHz, CDCl₃): 1.41 (t, 3H, Me), 1.60 (t, 3H, Me), 4.43 (q, 2H, CH₂), 4.55 (q, 2H, CH₂), 8.04 (s, 1H, CH) ppm.

The esters were reduced as described in Example 16, the alcohols brominated as described in Example 17, and the bromomethyl intermediates coupled as described in Example I39.

### Example I20: Preparation of 4,5-dibromo-1H-[1,2,3]triazole

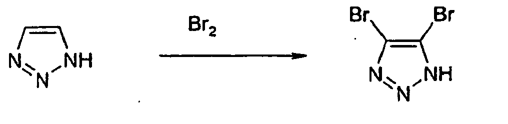

To a solution of 1*H-*[1,2,3]triazole (1.26 ml, 21.7 mmol) in water (10 ml) at 50°C, was added bromine (1.5 ml, 29 mmol). The reaction mixture was stirred at 50°C for 1.5 hours. The white solid (2.375 g) was isolated via filtration and washed with water (5 ml). To the combined filtrates was added more bromine (1.5 ml, 29 mmol). The reaction mixture was stirred at room temperature for 20 hours. More white solid (1.83 g) was isolated via filtration and washed with water (5 ml). To the combined filtrates was added more bromine (1.5 ml, 29 mmol). The reaction mixture was stirred at room temperature for 20 hours. More white solid (375 mg) was isolated via filtration and washed with water (5ml). The white solids were combined and dried to give 4,5-dibromo-1*H-*[1,2,3]-triazole (4.92 g, 93% yield). M.p. 194.7°C.

### Example I21: Preparation of 4,5-dibromo-1-methyl-1H-[1,2,3]triazole and 4,5-dibromo-2-methyl-2H-[1,2,3]triazole

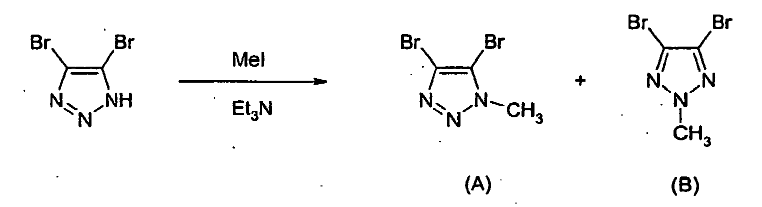

To a solution of 4,5-dibromo-1*H*-[1,2,3]triazole (2.26 g, 10 mmol) (Example I20) and triethyl amine (1.5 ml, 10 mmol) in dichloromethane (50 ml), was added methyl iodide (625 µl, 10 mmol). The reaction mixture was stirred at room temperature for 24 hours. More,triethyl amine (0.75 ml, 5 mmol) and more methyl iodide (312 µl, 5 mmol) were added and the mixture was stirred for 3 hours. The reaction mixture was quenched by addition of aqueous ammonium chloride (saturated) (15 ml). The phases were separated and the organic phase was dried over magnesium sulfate and concentrated. The residue was purified by column chromatography on silica gel (eluent: 10-30% ethyl acetate in hexane) to give 4,5-dibromo-2-methyl-2H6[1,2,3]triazole (isomer B) (625 mg, 26% yield) and 4,5-dibromo-1-methyl-1*H-*[1,2,3]triazole (isomer A) (825 mg, 34% yield).
Isomer A ¹H-NMR (400 MHz, CDCl₃): 4.09 (s, 3H, Me) ppm.
Isomer B ¹H-NMR (400 MHz, CDCl₃): 4.18 (s, 3H, Me) ppm.

### Example I22: Preparation of 4-bromo-1,5-dimethyl-1H-[1,2,3]triazole

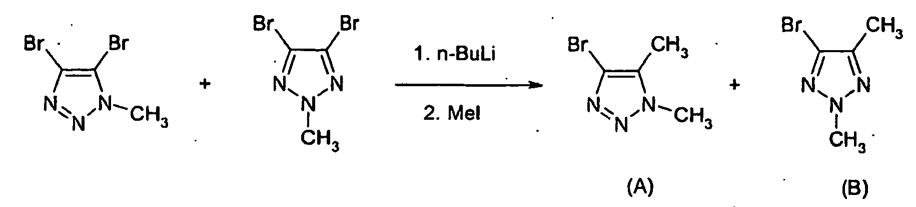

To a solution of 4,5-dibromo-1-methyl-1*H-*[1,2,3]triazole and 4,5-dibromo-2-methyl-2*H-*[1,2,3]triazole (1.5 g, 6.23 mmol) (Example I21) in tetrahydrofuran (25 ml) at -80°C, was slowly added n-butyl lithium (2.5M in THF) (3 ml, 7.48 mmol) followed 20 minutes later by methyl iodide (775 µl, 12.46 mmol). The reaction mixture was stirred at -80°C for 2 hours. The reaction mixture was allowed to warm to room temperature and quenched with by addition of aqueous hydrochloric acid (1M). The mixture was extracted with dichloromethane, the organic extract dried over magnesium sulfate and concentrated to give a mixture of 4-bromo-1,5-dimethyl-1*H-*[1,2,3]triazole (isomer A) and 4-bromo-2,5-dimethyl-2*H-*[1,2,3]triazole (isomer B) (1.096 g, 89% yield).
Isomer A ¹H-NMR (400 MHz, CDCl₃): 2.30 (s, 3H, Me), 4.00 (s, 3H, Me) ppm.
Isomer B ¹H-NMR (400MHz, CDCl₃): 2.24 (s, 3H, Me), 4.10 (s, 3H, Me) ppm.

### Example I23: Preparation of 4-bromo-5-bromomethyl-1-methyl-1H-[1,2,3]triazole and 4-bromo-5-bromomethyl-2-methyl-2H-[1,2,3]triazole

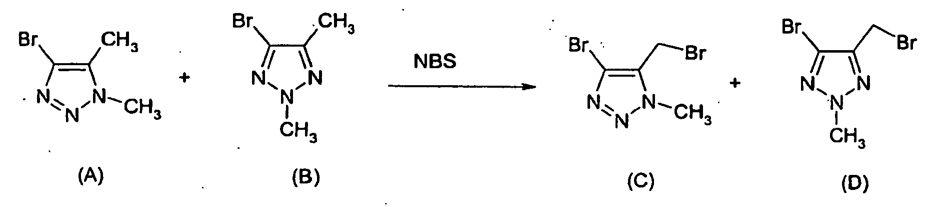

The mixture of 4-bromo-1,5-dimethyl-1*H-*[1,2,3]triazole and 4-bromo-2,5-dimethyl-2*H-*[1,2,3]triazole (Example I22) (950mg, 4.32 mmol) were dissolved in carbon tetrachloride(15ml). N-bromosuccinimide (NBS) (845mg, 4.75 mmol) and 2,2'-azobisisobutyronitrile (AIBN) (71 mg, 0.43 mmol) were added under nitrogen. The reaction mixture was stirred and irradiated with a UV lamp. After a short time, the mixture was refluxing with the heat of the lamp. The mixture was filtered, the solid was washed with dichloromethane and the combined filtrates were concentrated. The residue was purified by column chromatography on silica gel (eluent: ethyl acetate / hexane) to give 4-bromo-5-bromomethyl-1-methyl-1*H-*[1,2,3]triazole (isomer C) (422 mg, 38% yield) and 4-bromo-5-bromomethyl-2-methyl-2*H-*[1,2,3]triazole (isomer D) (373 mg, 34% yield).
Isomer C ¹H-NMR (400 MHz, CDCl₃): 4.12 (s, 3H, Me), 4.45 (s, 2H, CH₂) ppm.
Isomer D ¹H-NMR (400 MHz, CDCl₃): 4.17 (s, 3H, Me), 4.45 (s, 2H, CH₂) ppm.

The bromomethyl intermediates were coupled as described in Example I39.

### Example I24: Preparation of 5-hydroxy-1-(4-methoxy-benzyl)-1H-[1,2,3]triazole-4-carboxylic acid ethyl ester

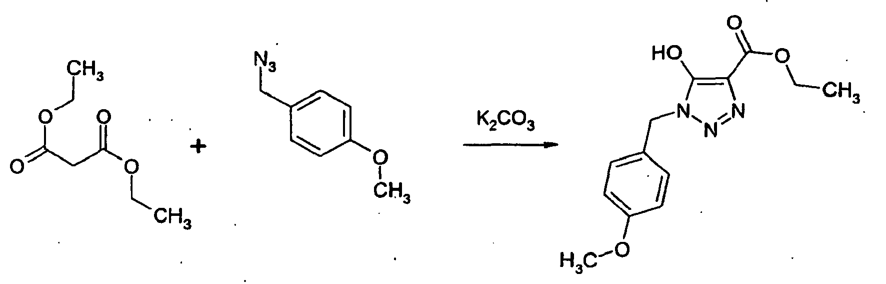

4-Methoxybenzyl azide (preparation described in e.g. J. Chem. Soc., Perkin Trans. 1, 1982 (2), 627-630) (42 g, 0.253 mol) was dissolved in dry dimethylsulfoxide (272 ml) and powdered potassium carbonate (139 g, 1 mol) was added. The reaction mixture was stirred at room temperature while diethyl malonate (56 g, 0.35 mol) was added. The reaction mixture was stirred at 40°C for 72 hours. The reaction mixture was cooled to 0°C and quenched by addition of aqueous hydrochloric acid (5M) (675 ml). The mixture was stirred at room temperature for 2 hours. The solid was isolated via filtration, washed with water and hexane and dried to give 5-hydroxy-1-(4-methoxy-benzyl)-1*H-*[1,2,3]triazole-4-carboxylic acid ethyl ester as an off white solid (47.5 g, 68% yield).
¹H-NMR (400 MHz, CDCl₃): 1.29 (t, 3H, Me), 3.72 (s, 3H, Me), 4.25 (q, 2H, CH₂), 5.27 (s, 2H, CH₂), 7.07 (q, 4H, CH) ppm.

### Example I25: Preparation of 5-methoxy-1-(4-methoxy-benzyl)-1H-[1,2,3]triazole-4-carboxylic acid ethyl ester

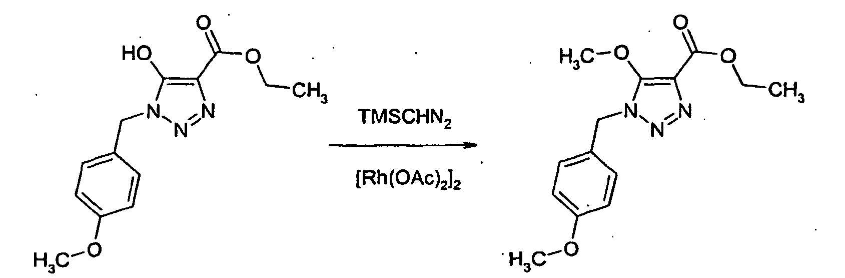

5-Hydroxy-1-(4-methoxy-benzyl)-1*H-*[1,2,3]triazole-4-carboxylic acid ethyl ester (26.18 g, 0.944 mol) (Example I24) was dissolved in dry N,N-dimethylformamide (300 ml) and rhodium(II) acetate dimer (300 mg) was added. The reaction mixture was cooled to 20°C and (trimethylsilyl)diazomethane (TMSCHN₂) (2M in diethyl ether) (142 ml, 0.284 mol) was added over 2 hours and the reaction mixture stirred at room temperature for 16 hours. The reaction mixture was cooled to 0°C and quenched by sequential addition of methanol (75 ml), glacial acetic acid (5 ml) and water (1 ml). The mixture was extracted three times with ethyl acetate. The combined organic extracts were washed three times with brine, dried over magnesium sulfate and concentrated. The residue was purified by column chromatography on silica gel (eluent: 10-55% ethyl acetate in hexane) to give 5-methoxy-1-(4-methoxy-benzyl)-1*H-*[1,2,3]triazole-4-carboxylic acid ethyl ester as a straw coloured oil (15.2 g, 55% yield).
¹H-NMR (400 MHz, CDCl₃): 1.41 (q, 3H, Me), 3.80 (s, 3H, Me), 4.13 (s, 3H, Me), 4.41 (t, 2H, CH₂), 5.31 (s, 2H, CH₂), 7.1 (q, 4H, CH) ppm.

5-Methoxy-1-(4-methoxy-benzyl)-1*H-*[1,2,3]triazole-4-carboxylic acid ethyl ester was deprotected as described in Example I13 to give 5-methoxy-1*H-*[1,2,3]triazole-4-carboxylic acid ethyl ester.
¹H-NMR (400 MHz, CDCl₃): 1.41 (t, 3H, Me), 4.11 (s, 3H, Me), 4.47 (q, 2H, CH₂) ppm.

5-Methoxy-1*H-*[1,2,3]triazole-4-carboxylic acid ethyl ester was methylated with methyl iodide according to the method described in Example I14 to give 5-methoxy-1-methyl-1*H-*[1,2,3]triazole-4-carboxylic acid ethyl ester (isomer A) and 5-methoxy-2-methyl-2*H-*[1,2,3]triazole-4-carboxylic acid ethyl ester (isomer B).
Isomer A ¹H-NMR (400 MHz, CDCl₃): 1.39 (q, 3H, Me), 4.15 (s, 3H, Me), 4.24 (s, 3H, Me), 4.40 (q, 2H, CH₂) ppm.
Isomer B ¹H-NMR (400 MHz, CDCl₃): 1.35 (t, 3H, Me), 3.99 (s, 3H, Me), 4.07 (s, 3H, Me), 4.36 (q, 2H, CH₂) ppm.

The esters were reduced as described in Example I6, the alcohols brominated as described in Example I7, and the bromomethyl intermediates coupled as described in Example I39.

### Example 126: Preparation of 5-difluoromethoxy-1-(4-methoxy-benzyl)-1H-[1,2.3]-triazole-4-carboxylic acid ethyl ester

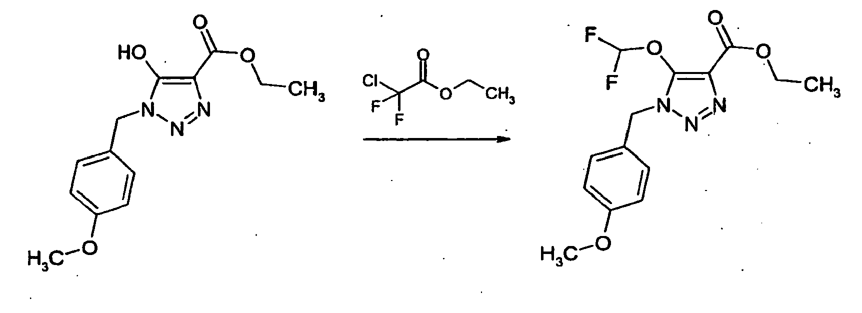

To a solution of 5-hydroxy-1-(4-methoxy-benzyl)-1*H-*[1,2,3]triazole-4-carboxylic acid ethyl ester (1.0 g, 3.606 mmol) (Example I24) in N,N-dimethylformamide (18 ml) was added potassium carbonate (1.568 g, 11.36 mmol). The reaction mixture was heated to 80°C and ethyl 2-chloro-2,2-difluoroacetate (1.715 g, 10.8 mmol) added at 80°C over 10 minutes. The reaction mixture was stirred at 80°C for 1 hour. The reaction mixture was allowed to cool to room temperature and was quenched by addition of water (5 ml), ethyl acetate (10 ml) and aqueous hydrochloric acid (2M) (9 ml). The phases were separated and the aqueous phase extracted twice with more ethyl acetate. The combined organic extracts were washed with brine, dried over magnesium sulfate and concentrated. The residue was purified by column chromatography on silica gel (eluent: 10-40% ethyl acetate in hexane) to give 5-difluoromethoxy-1-(4-methoxy-benzyl)-1*H-*[1,2,3]triazole-4-carboxylic acid ethyl ester of a straw coloured oil which solidified on standing (0.726 g, 62% yield).
¹H-NMR (400 MHz, CDCl₃): 1.41 (t, 3H, Me), 3.80 (s, 3H, Me), 4.43 (q, 2H, CH₂), 5.39 (s, 2H, CH₂), 6.88 (d, 2H, CH), 7.3 (d, 2H, CH), 7.32 (t, 1H, CHF₂) ppm.

5-Difluoromethoxy-1-(4-methoxy-benzyl)-1*H-*[1,2,3]triazole-4-carboxylic acid ethyl ester was deprotected as described in Example I13 to give 5-difluoromethoxy-1*H-*[1,2,3]triazole-4-carboxylic acid ethyl ester.
¹H-NMR (400 MHz, CDCl₃): 1.43 (t, 3H, Me), 4.48 (t, 2H, CH₂), 7.05 (t, 1H, CHF₂) ppm.

5-Difluoromethoxy-1*H-*[1,2,3]triazole-4-carboxylic acid ethyl ester was methylated with methyl iodide according to the method described in Example I14 to give 5-difluoromethoxy-1-methyl-1*H-*[1,2,3]triazole-4-carboxylic acid ethyl ester (isomer A) and 5-difluoromethoxy-2-methyl-2*H-*[1,2,3]triazole-4-carboxylic acid ethyl ester (isomer B).
Isomer A ¹H-NMR (400 MHz, CDCl₃): 1.41 (t, 3H, Me), 4.49 (s, 3H, Me), 4.41 (q, 2H, CH₂), 7.19 (t, 1H, CHF₂) ppm.
Isomer B ¹H-NMR (400 MHz, CDCl₃): 1.41 (t, 3H, Me), 4.18 (s, 3H, Me), 4.42 (q, 2H, CH₂), 6.92 (t, 1H, CHF₂) ppm.

The esters were reduced as described in Example I6, the alcohols brominated as described in Example I7, and the bromomethyl intermediates coupled as described in Example I39.

### Example I27: Preparation of 2-(4-bromo-4,4-difluoro-but-2-ylnyloxy)-tetrahdro-pyran

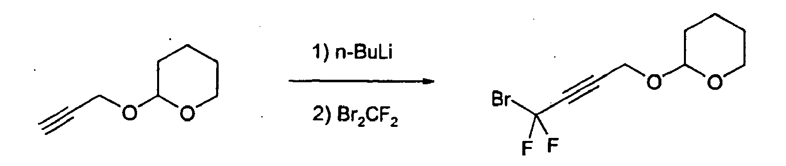

2-Prop-2-ynyloxy-tetrahydro-pyran (10.0 g, 71.3 mmol) was dissolved in dry tetrahydrofuran (175 ml) under nitrogen and the solution was cooled to -40°C. n-Butyl lithium (1.6 M in hexane) (44.6 ml, 71.3 mmol) was added over 5 minutes, keeping the temperature between -35°C and -40°C. The reaction was stirred for 15 minutes, then dibromodifluoromethane (14.89 g, 71.3 mmol) was added in one portion and the reaction mixture was allowed to warm to room temperature over 1 hour. The reaction mixture was quenched with aqueous potassium dihydrogenphosphate (10%) and extracted three times with diethyl ether. The combined organic extracts were washed with water and brine, dried over magnesium sulfate and concentrated. The residue was purified by column chromatography on silica gel (eluent: diethyl ether / hexane) to give 2-(4-bromo-4,4-difluoro-but-2-ynyloxy)-tetrahydro-pyran as a colourless oil (17.6 g, 91.7% yield).
¹H-NMR (400 MHz, CDCl₃): 1.5-1.9 (m, 6H, CH₂), 3.5-3.6 (m, 1H, CH₂), 3.8-3.9 (m, 1H, CH₂). 4.4 (m, 2H, CH₂), 4.8 (m, 1H, CH) ppm.

The same method was used with acetic anhydride as the reagent to give 5-(tetra-hydro-pyran-2-yloxy)-pent-3-yn-2-one.
¹H-NMR (400 MHz, CDCl₃): 1.5-1.85 (m, 6H, CH₂), 1.9-2.08 (s, 3H, Me), 3.53 (m, 1H, CH₂), 3.82 (m, 1H, CH₂), 4.29 (m, 1H, CH₂), 4.33 (m, 1H, CH₂), 4.8 (m, 1H, CH) ppm.

The same method was used with methyl chloroformate as the reagent to give 5-(tetrahydro-pyran-2-yloxy)-pent-3-yn-2-one.
¹H-NMR (400 MHz, CDCl₃): 1.5-1.85 (m, 6H, CH₂), 3.56 (m, 1H, CH₂), 3.8 (s, 3H, Me), 3.82 (m, 1H, CH₂), 4.4 (s, 2H, CH₂), 4.81 (m, 1H, CH) ppm.

### Example I28: Preparation of 4-(bromo-difluoro-methyl)-1-(4-methoxy-benzyl)-5-(tetrahydro-pyran-2-yloxymethyl-1H-[1,2,3]triazole and 5-(bromo-difluoro-methyl)-1-(4-methoxy-benzyl)-4-(tetrahydro-pyran-2-yloxymethyl)-1H-[1,2,3]triazole

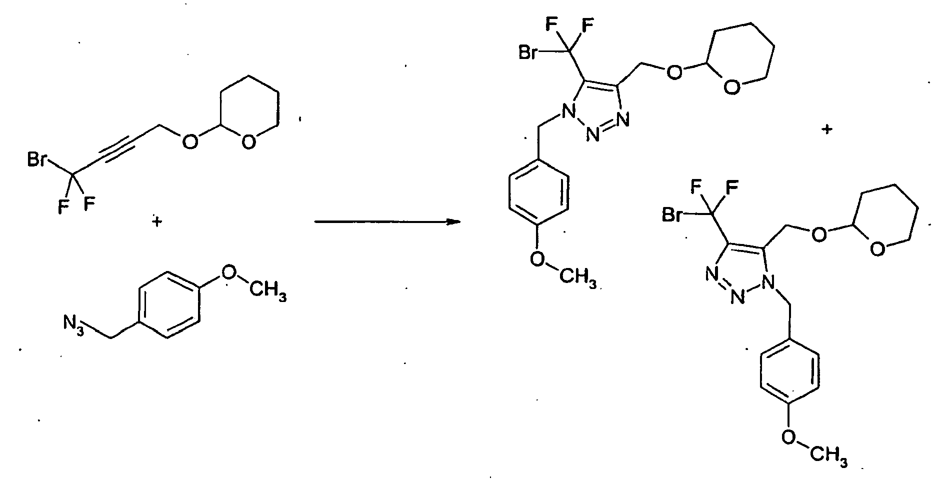

2-(4-Bromo-4,4-difluoro-but-2-ynyloxy)-tetrahydropyran (Example I27) (10.0 g, 37 mmol) was dissolved in toluene (250 ml), 4-methoxybenzyl azide (preparation described in e.g. J. Chem. Soc., Perkin Trans. 1, 1982 (2), 627-630) (6.0 g, 37 mmol) was added and the mixture was heated to reflux for 16 hours. The mixture was cooled to room temperature and concentrated. The residue was purified by column chromatography on silica gel (eluent: ethyl acetate / hexane) to give a 2:1-mixture of 4-(bromo-difluoromethyl)-1-(4-methoxy-benzyl)-5-(tetrahydro-pyran-2-yloxymethyl)-1*H-*[1,2,3]triazole and 5-(bromo-difluoro-methyl)-1-(4-methoxy-benzyl)-4-(tetrahydro-pyran-2-yloxy-methyl)-1*H-*[1,2,3]triazole (14.39 g, 89% yield).
Major isomer ¹H-NMR (400 MHz, CDCl₃): 1.5-1.8 (m, 6H, CH₂), 3.5-3.6 (m, 2H, CH₂), 3.8 (s, 3H, Me), 4.55 (d, 1H, CH₂), 4.55-4.6 (m, 1H, CH), 4.75 (d, 1H, CH₂), 5.5 (s, 2H, CHF), 6.85 (d, 2H, CH), 7.2 (d, 2H, CH) ppm.
Minor isomer ¹H-NMR (400 MHz, CDCl₃): 1.5-1.8 (m, 6H, CH₂), 3.8 (s, 3H, Me), 3.85-3.95 (m, 2H, CH₂), 4.65 (d, 1H, CH₂), 4.75-4. (m, 1H, CH), 4.9 (d, 1H, CH₂), 5.52 (s, 2H, CH₂), 6.85 (d, 2H, CH), 7.25 (d, 2H, CH) ppm.

The same method was used with 5-(tetrahydro-pyran-2-yloxy)-pent-3-yn-2-one as the starting material to give a mixture of 1-[1-(4-methoxy-benzyl)-5-(tetrahydro-pyran-2-yloxymethyl)-1*H-*[1,2,3]triazol-4-yl]-ethanone and 1-[3-(4-methoxy-benzyl)-5-(tetra-hydro-pyran-2-yloxymethyl)-3*H-*[1,2,3]triazol-4-yl]-ethanone.
¹H-NMR (400 MHz, CDCl₃): 1.5-1.85 (m, 6H, CH₂), 2.63-2.7 (s, 3H, Me), 3.5-3.58 (m, 1H, CH₂), 3.79-3.80 (s, 3H, Me), 4.63-4.73 (m, 1H, CH), 4.8 (m, 1H, CH₂), 5.06 (d, 2H, CH₂), 5.68-5.8 (s, 2H, CH₂), 6.84 (m, 2H, CH), 7.22-7.3 (m, 2H, CH) ppm.

The same method was used with 4-(tetrahydro-pyran-2-yloxy)-but-2-ynoic acid methyl ester as the starting material to give a mixture of 1-(4-methoxy-benzyl)-5-(tetra-hydro-pyran-2-yloxymethyl)-1*H-*[1,2,3]triazole-4-carboxylic acid methyl ester and 3-(4-methoxy-benzyl)-5-(tetrahydro-pyran-2-yloxymethyl)-3*H-*[1,2,3]triazole-4-carboxylic acid methyl ester.
¹H-NMR (400 MHz, CDCl₃): 1.5-1.8 (m, 6H, CH₂), 3.54-3.85 (m, 1H, CH₂), 3.78-3.79 (s, 3H, Me), 3.91-3.95 (s, 3H, Me), 4.63-4.82 (m, 1H, CH), 4.74-4.85 (d, 1H, CH₂), 4.99-5.01 (d, 2H, CH₂), 5.64-5.82 (s, 2H, CH₂), 6.84 (m, 2H, CH), 7.2-7.3 (m, 2H, CH) ppm.

### Example I29: Preparation of [5-(bromo-difluoro-methyl)-3-(4-methoxy-benzyl)-3H-[1,2,3]triazol-4yl]-methanol and [5-(bromo-difluoro-methyl)-1-(4-methoxy-benzyl)-1H-[1.2,3]triazol-4-yl]-methanol

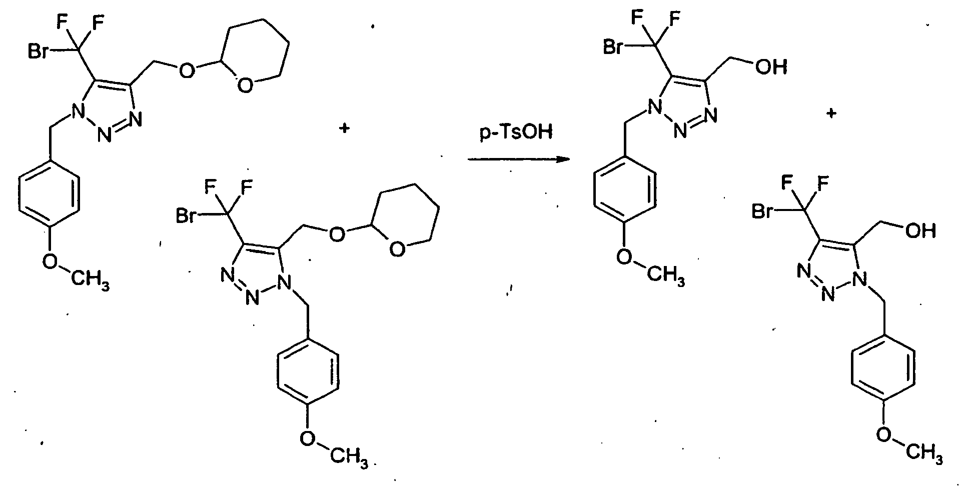

The 2:1-mixture of 4-(bromo-difluoro-methyl)-1-(4-methoxy-benzyl)-5-(tetra-hydro-pyran-2-yloxymethyl)-1*H-*[1,2,3]triazole and 5-(bromo-difluoro-methyl)-1-(4-methoxy-benzyl)-4-(tetrahydro-pyran-2-yloxymethyl)-1*H-*[1,2,3]triazole (Example I28) (10.5 g, 24.8 mmol) was dissolved in methanol (100 ml), and p-toluene sulfonic acid monohydrate (5.4 g, 28.3 mmol) was added. The reaction mixture was stirred at room temperature for 1hour. The reaction mixture was concentrated and the residue purified by column chromatography on silica gel (eluent: ethyl acetate / hexane) to give a 2:1-mixture of [5-(bromo-difluoro-methyl)-3-(4-methoxy-benzyl)-3*H-*[1,2,3]triazol-4-yl]-methanol and [5-(bromo-difluoro-methyl)-1-(4-methoxy-benzyl)-1*H-*[1,2,3]triazol-4-yl]-methanol as a colourless oil (7.04 g, 82% yield).
Major isomer ¹H-NMR (400 MHz; CDCl₃): 3.8 (s, 3H, Me), 4.81 (s, 2H, CH₂), 5.8 (s, 2H, CH₂), 6.85 (d, 2H, CH), 7.2 (d, 2H, CH) ppm.
Minor isomer ¹H-NMR (400 MHz, CDCl₃): 3.8 (s, 3H, Me), 4.83 (s, 2H, CH₂), 5.9 (s, 2H, CH₂), 6.85 (d, 2H, CH), 7.25 (d, 2H, CH) ppm.

The same method was used with the mixture of 1-[1-(4-methoxy-benzyl)-5-(tetrahydro-pyran-2-yloxymethyl)-1*H-*[1,2,3]triazol-4-yl]-ethanone and 1-[3-(4-methoxybenzyl)-5-(tetrahydro-pyran-2-yloxymethyl)-3*H-*[1,2,3]triazol-4-yl]-ethanone as the starting material to give a mixture of 1-[5-hydroxymethyl-1-(4-methoxy-benzyl)-1*H-*[1,2,3]triazol-4-yl]-ethanone and 1-[5-hydroxymethyl-3-(4-methoxy-benzyl)-3*H-*[1,2,3]-triazol-4-yl]-ethanone. The mixture was used without further purification.

The same method was used with the mixture of 1-(4-methoxy-benzyl)-5-(tetra-hydro-pyran-2-yloxymethyl)-1*H-*[1,2,3]triazole-4-carboxylic acid methyl ester and 3-(4-methoxy-benzyl)-5-(tetrahydro-pyran-2-yloxymethyl)-3*H-*[1,2,3]triazole-4-carboxylic acid methyl ester as the starting material to give a mixture of 5-hydroxymethyl-1-(4-methoxy-benzyl)-1*H-*[1,2,3]triazole-4-carboxylic acid methyl ester and 5-hydroxy-methyl-3-(4-methoxy-benzyl)-3*H-*[1,2,3]triazole-4-carboxylic acid methyl ester. The mixture was used without further purification.

### Example I30: Preparation of 4-(bromo-difluoro-methyl)-5-bromomethyl-1-(4-methoxy-benzyl)-1H-[1,2,3]triazole and 5-(bromo-difluoro-methyl)-4-bromomethyl-1-(4-methoxy-benzyl)-1H-[1,2,3]triazole

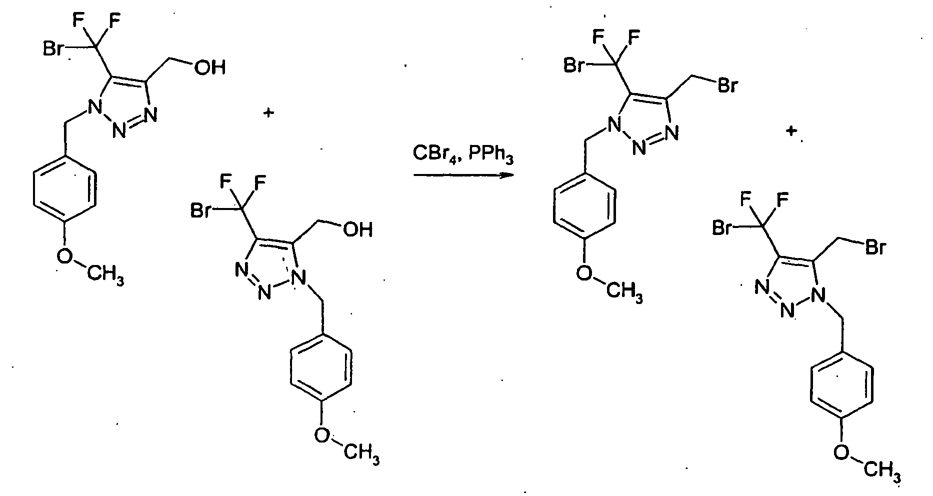

The 2:1-mixture of [5-(bromo-difluoro-methyl)-3-(4-methoxy-benzyl)-3*H-*[1,2,3]triazol-4-yl]-methanol and [5-(bromo-difluoro-methyl)-1-(4-methoxy-benzyl)-1*H-*[1,2,3]triazol-4-yl]-methanol (Example I29) (7.0 g, 20 mmol) was dissolved in dichloromethane (100 ml) and triphenyl phosphine (6.3 g, 24.0 mmol) was added. The reaction mixture was cooled to 0°C and a solution of carbon tetrabromide (7.96 g, 24.0 mmol) in dichloromethane (10 ml) was added dropwise. The reaction mixture was stirred for 2 hours at 0°C, concentrated and the residue was purified by column chromatography on silica gel (eluent: ethyl acetate / hexane) to give a 3:1-mixture of 4-(bromo-difluoromethyl)-5-bromomethyl-1-(4-methoxy-benzyl)-1*H-*[1,2,3]triazole and 5-(bromo-difluoro-methyl)-4-bromomethyl-1-(4-methoxy-benzyl)-1*H-*[1,2,3]triazole as a colourless oil (7.35 g, 89% yield).

Major isomer ¹H-NMR (400 MHz, CDCL₃): 3.8 (s, 3H, Me), 4.35 (s, 2H, CH₂), 5.61 (s, 2H, CH₂), 6.9 (d, 2H, CH), 7.25 (d, 2H, CH) ppm.

Minor isomer ¹H-NMR (400 MHz, CDCL₃): 3.78 (s, 3H, Me), 4.6 (s, 2H, CH₂), 5.61 (s, 2H, CH₂), 6.9 (d, 2H, CH), 7.25 (d, 2H, CH) ppm.

### Example I31: Preparation of methanesulfonic acid 5-acetyl-1-(4-methoxy-benzyl)-1H-[1,2,3]triazol-4-ylmethyl ester and methanesulfonic acid 5-acetyl-3-(4-methoxy-benzyl)-3H-[1,2,3]triazol-4-yhmethyl ester

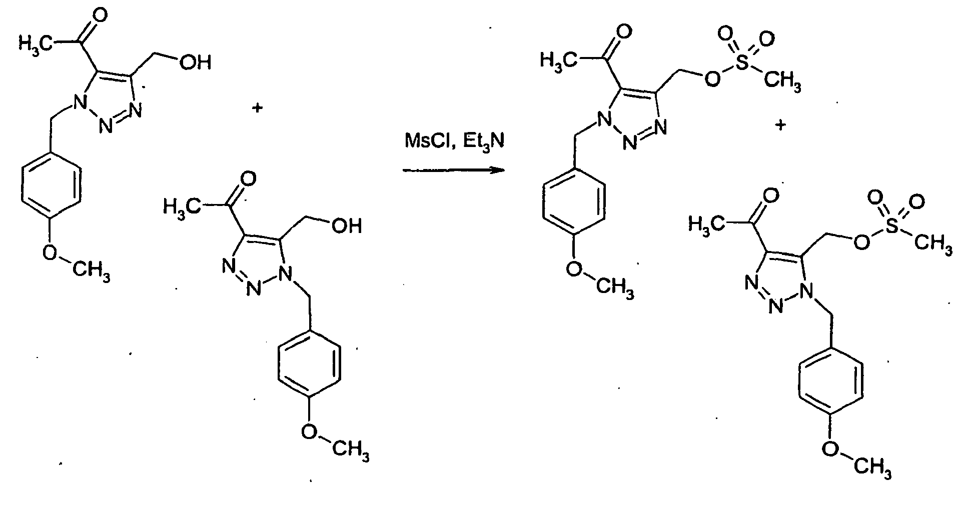

To a solution of the mixture of 1-[5-hydroxymethyl-1-(4-methoxy-benzyl)-1*H-*[1,2,3]triazol-4-yl]-ethanone and 1-[5-hydroxymethyl-3-(4-methoxy-benzyl)-3*H-*[1,2,3]-triazol-4-yl]-ethanone (3.19 g, 12.2 mmol) in dichloromethane (75 ml) was added methanesulfonyl chloride (0.95 ml, 12.2 mmol) followed by addition of triethylamine (1.87 ml, 13.4 mmol) at room temperature. The reaction mixtures were stirred at room temperature for 2 hours. The reaction mixtures were concentrated and re-dissolved in acetonitrile. The solids were removed by filtration and washed with acetonitrile. The combined filtrates were concentrated to give a mixture of methanesulfonic acid 5-acetyl-1-(4-methoxy-benzyl)-1*H-*[1,2,3]triazol-4-ylmethyl ester and methanesulfonic acid 5-acetyl-3-(4-methoxy-benzyl)-3*H-*[1,2,3]triazol-4-ylmethyl ester. The mixture was used without further purification.

The same method was used with the mixture of 5-hydroxymethyl-1-(4-methoxybenzyl)-1*H-*[1,2,3]triazole-4-carboxylic acid methyl ester and 5-hydroxymethyl-3-(4-methoxy-benzyl)-3*H-*[1,2,3]triazole-4-carboxylic acid methyl ester as the starting material to give a mixture of 5-methanesulfonyloxymethyl-1-(4-methoxy-benzyl)-1*H-*[1,2,3]triazole-4-carboxylic acid methyl ester and 5-methanesulfonyloxymethyl-3-(4-methoxy-benzyl)-3*H-*[1,2,3]triazole-4-carboxylic acid methyl ester. The mixture was used without further purification.

### Example I32: Preparation of 4-(bromo-difluoro-methyl)-5-(5,5-dimethyl-4,5-dihydro-isoxazol-3-ylsulfanylmethyl)-2-methyl-2H-[1.2.3]triazole

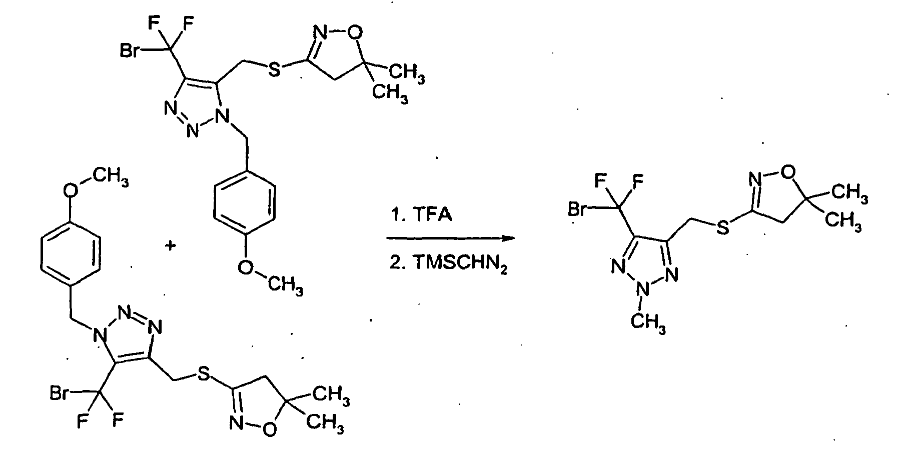

The mixture of 4-(bromo-difluoro-methyl)-5-(5,5-dimethyl-4,5-dihydro-isoxazol-3-ylsulfanylmethyl)-1-(4-methoxy-benzyl)-1*H-*[1,2,3]triazole and 5-(bromo-difluoromethyl)-4-(5,5-dimethyl-4,5-dihydro-isoxazol-3-ylsulfanylmethyl)-1-(4-methoxybenzyl)-1*H-*[1,2,3]triazole (Example I40) (200 mg, 0.43 mmol) was dissolved in trifluoroacetic acid (245 mg, 2.15 mmol) and anisole (69 mg, 0.64 mmol). The mixture was heated to 60-65°C for 3 hours. The mixture was diluted with fluorobenzene and concentrated.

The residue was dissolved in dry methanol (10 ml) and trimethylsilyl diazomethane (TMSCHN₂) (2M in hexane) (3 ml, 6 mmol) was added dropwise over 10 minutes. More trimethylsilyl diazomethane (TMSCHN₂) (2M in hexane) (4 ml, 8 mmol) was added and the mixture stirred for 15 minutes. The reaction mixture was concentrated and the residue purified by column chromatography on silica gel (eluent: ethyl acetate / hexane), to give 4-(bromo-difluoro-methyl)-5-(5,5-dimethyl-4,5-dihydro-isoxazol-3-ylsulfanylmethyl)-2-methyl 2*H-*[1,2,3]triazole (110 mg, 72% yield).
¹H-NMR (400 MHz, CDCl₃): 1.41 (s, 6H, Me), 2.82 (s, 2H, CH₂), 4.21 (s, 3H, Me), 4.4 (s, 2H, CH₂) ppm.

The same method was used with the mixture of 1-[5-(5,5-dimethyl-4,5-dihydro-isoxazol-3-ylsulfanylmethyl)-1-(4-methoxy-benzyl)-1*H-*[1,2,3]triazol-4-yl]-ethanone and 1-[5-(5,5-dimethyl-4,5-dihydro-isoxazol-3-ylsulfanylmethyl)-3-(4-methoxy-benzyl)-3*H-*[1,2,3]triazol-4-yl]-ethanone (Example I40) as the starting material to give 1-[5-(5,5-dimethyl-4,5-dihydro-isoxazol-3-ylsulfanylmethyl)-2*H-*[1,2,3]triazol-4-yl]-ethanone. ¹H-NMR (400 MHz, CDCl₃): 1.44 (s, 6H, Me), 2.72 (s, 3H, Me), 2.83 (s, 2H, CH₂), 4.61 (s, 2H, CH₂) ppm.

1-[5-(5,5-Dimethyl-4,5-dihydro-isoxazol-3-ylsulfanylmethyl)-2*H-*[1,2,3]triazol-4-yl]-ethanone was reacted with methyl iodide as described in Example I14 to give 1-[5-(5,5-dimethyl-4,5-dihydro-isoxazol-3-ylsulfanylmethyl)-2-methyl-2*H-*[1,2,3]triazol-4-yl]-ethanone.
¹H-NMR (400 MHz, CDCl₃): 1.41 (s, 6H, Me), 2.83 (s, 2H, CH₂), 3.96 (s, 3H, Me), 4.22 (s, 3H, Me), 4.58 (s, 2H, CH₂) ppm.

The same method was used with the mixture of 5-(5,5-dimethyl-4,5-dihydro-isoxazol-3-ylsulfanylmethyl)-1-(4-methoxy-benzyl)-1*H-*[1,2,3]triazole-4-carboxylic acid methyl ester and 5-(5,5-dimethyl-4,5-dihydro-isoxazol-3-ylsulfanylmethyl)-3-(4-methoxy-benzyl)-3*H-*[1,2,3]triazole-4-carboxylic acid methyl ester (Example I40) as the starting material to give 5-(5,5-dimethyl-4,5-dihydro-isoxazol-3-ylsulfanylmethyl)-2*H-*[1,2,3]triazole-4-carboxylic acid methyl ester, which was reacted with methyl iodide as described in Example I14 to give 5-(5,5-dimethyl-4,5-dihydro-isoxazol-3-ylsulfanylmethyl)-2-methyl-2*H-*[1,2,3]triazole-4-carboxylic acid methyl ester.
¹H-NMR (400 MHz, CDCl₃): 1.41 (s, 6H, Me), 2.87 (s, 2H, CH₂), 3.97 (s, 3H, Me), 4.3 (s, 3H, Me), 4.57 (s, 2H, CH₂) ppm.

### Example I33: Preparation of 1-[5-(5,5-dimethyl-4,5-dihydro-isoxazol-3-ylsulfanyl-methyl)-2-methyl-2H-[1,2.3]triazol-4-yl]-ethanol

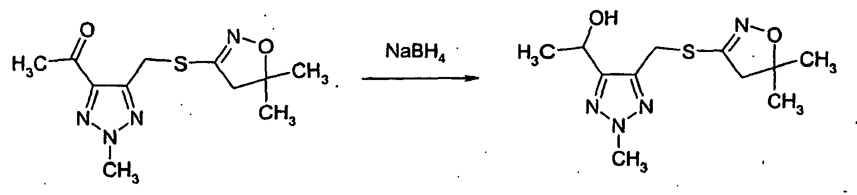

1-[5-(5,5-Dimethyl-4,5-dihydro-isoxazol-3-ylsulfanylmethyl)-2-methyl-2*H-*[1,2,3]triazol-4-yl]-ethanone (Example I32) (408 mg, 1.52 mmol) was dissolved in methanol (10 ml) at room temperature. Sodium borohydride (29 mg, 0.76 mmol) was added in one portion at room temperature. The reaction mixture was stirred at room temperature for 1 hour. The reaction mixture was quenched by addition of water and the mixture concentrated. The residue was partitioned between dichloromethane and water and the phases separated. The organic extract was dried over magnesium sulfate and concentrated to yield 1-[5-(5,5-dimethyl-4,5-dihydro-isoxazol-3-ylsulfanylmethyl)-2-methyl-2*H*-[1,2,3]triazol-4-yl]-ethanol as a colourless oil (375 mg, 91% yield).
¹H-NMR (400 MH_{z}, CDCl₃): 1.39 (s, 3H, Me), 1.4 (s, 3H, Me), 1.58 (d, 3H, Me), 2.8 (s, 2H, CH₂), 4.11 (s, 3H, Me), 4.36 (s, 2H, CH₂), 5.08 (m, 1H, CH) ppm.

### Example I34: Preparation of 4-(5,5-dimethyl-4,5-dihydro-isoxazol-3-ylsulfanylmethyl)-5-(1-fluoro-ethyl)-2-methyl-2H-[1,2,3]triazole

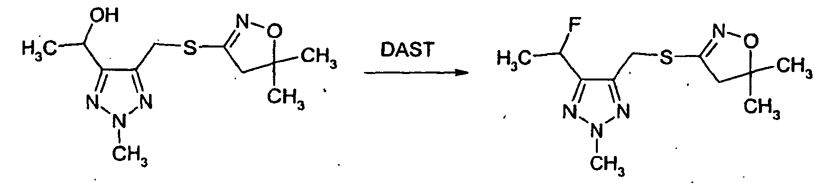

To a solution of 1-[5-(5,5-dimethyl-4,5-dihydro-isoxazol-3-ylsulfanylmethyl)-2-methyl-2*H*-[1,2,3]triazol-4-yl]-ethanol (Example I33) (375 mg, 1.39 mmol) in dichloromethane (10 ml) under nitrogen was added (diethylamino)sulfur trifluoride (DAST) (0.46 ml, 3.48 mmol) dropwise at room temperature. The reaction mixture was stirred at room temperature for 1 hour. The reaction mixture was quenched by addition of aqueous sodium bicarbonate (saturated) and the mixture extracted with dichloromethane. The combined organic extracts were dried over magnesium sulfate and concentrated to give 4-(5,5-dimethyl-4,5-dihydro-isoxazol-3-ylsulfanylmethyl)-5-(1-fluoro-ethyl)-2-methyl-2*H-*[1,2,3]triazole as an oil (282 mg, 74% yield).
¹H-NMR (400 MHz, CDCl₃): 1.39 (s, 3H, Me), 1.41 (s, 3H, Me), 1.76 (d, 3H, Me), 2.81 (s, 2H, CH₂), 4.15 (s, 3H, Me), 4.3 (d, 1H, CH₂), 4.4 (d, 1H, CH₂), 5.86 (m, 1H, CH) ppm.

### Example I35: Preparation of 1,5-dimethyl-1H-[1,2,3]triazole-4-carboxylic acid ethyl ester

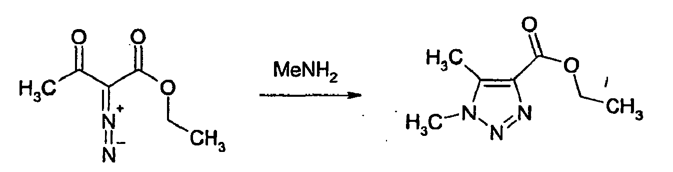

2-Diazo-3-oxo-butyric acid ethyl ester (5 g, 32 mmol) was dissolved in tetrahydrofuran (50 ml) and acetic acid (6 ml) and methylhydrazine was passed through the solution until a white precipitate crystallised out. More acetic acid (10 ml) was added and the solution was refluxed for 2 days. The reaction mixture was concentrated, the residue diluted with water, extracted with ethyl acetate, washed three times with aqueous sodium bicarbonate (saturated), water and brine, dried and concentrated to give 1,5-dimethyl-1H [1,2,3]triazole-4-carboxylic acid ethyl ester (4.4 g, 81% yield).
¹H-NMR (400 MHz, CDCl₃): 1.41 (t, 3H, Me), 2.49 (s, 3H, Me), 3.92 (s, 3H, Me), 4.3 (q, 2H, CH₂) ppm.

The ester was reduced as described in Example I6, the alcohol brominated as described in Example I7, and the bromomethyl intermediate coupled as described in Example I39.

### Example I36: Preparation of 5-cyclopropyl-1-methyl-1H-[1,2,3]triazole-4-carboxylic acid methyl ester

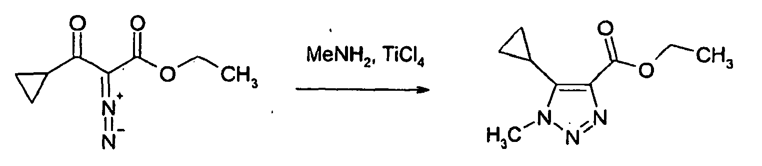

3-Cyclopropyl-2-diazo-3-oxo-propionic acid ethyl ester (preparation described in J. Org. Chem. 1950 (15) 74-80) (1 g, 5.5 mmol) was dissolved in dichloroethane (15 ml). Methylamine (340 mg, 10.9 mmol) was added followed by titanium tetrachloride (1M in dichloromethane) (5.5 ml, 5.5 mmol) to give an orange precipitate. The reaction mixture was heated to reflux. After addition of water, the mixture was concentrated, extracted with ethyl acetate, washed with aqueous sodium bicarbonate (saturated), water and brine, dried over magnesium sulfate and concentrated. The residue was purified by column chromatography on silica gel (eluent: ethyl acetate, iso-hexane) to give 5-cyclopropyl-1-methyl-1*H*-[1,2,3]triazole-4-carboxylic acid ethyl ester (740 mg, 71 % yield).
¹H-NMR (400 MHz, CDCl₃): 0.98 (m, 2H, CH₂), 1.19 (m, 2H, CH₂), 1.42 (t, 3H, Me), 1.84 (m, 1H, CH), 4.09 (s, 3H, Me), 4.41 (q, 2H, CH₂) ppm.

The ester was reduced as described in Example I6, the alcohol brominated as described in Example I7, and the bromomethyl intermediate coupled as described in Example I39.

### Example I37: Preparation of 5-cyclopropyl-2H-[1,2,3]triazole-4-carboxylic acid ethyl ester

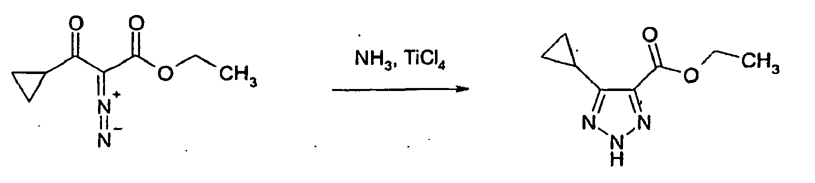

Ammonia (approximately 200 mg) was condensed into a flask at -78°C, dichloroethane (15ml) was added, followed by 3-cyclopropyl-2-diazo-3-oxo-propionic acid ethyl ester (preparation described in J. Org. Chem. 1950 (15) 74-80). Titanium tetrachloride (1M in dichloromethane) (5.5 ml, 5.5 mmol) was added to give an orange precipitate. The solution was warmed to room temperature, then heated to reflux for 16 hours. The reaction was quenched by addition of water (1 ml) and concentrated. The residue was extracted with ethyl acetate, washed with aqueous sodium bicarbonate (saturated), water and brine, dried over magnesium sulfate and concentrated to give 5-cyclopropyl-2*H-*[1,2,3]triazole-4-carboxylic acid ethyl ester as an orange oil (960 mg, 96% yield) which solidified on standing.
¹H-NMR (400 MHz, CDCl₃): 1.03 (m, 2H, CH₂), 1.10 (m, 2H, CH₂), 1.40 (t, 3H, Me), 2.56 (m, 1H, CH), 4.44 (q, 2H, CH₂) ppm.

### Example I38: Preparation of 5-cyclopropyl-2-methyl-2H-[1,2,3]triazole-4-carboxylic acid ethyl ester and 5-cyclopropyl-3-methyl-3H-[1,2,3]triazole-4-carboxylic acid ethyl ester

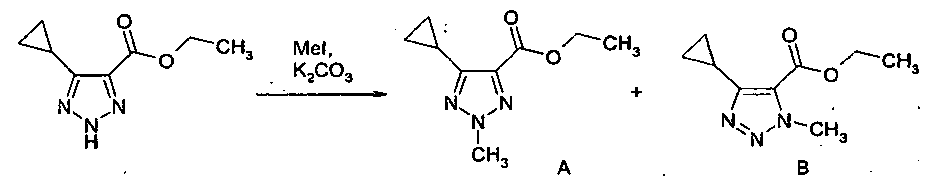

5-Cyclopropyl-2*H-*[1,2,3]triazole-4-carboxylic acid ethyl ester (Example I37) (110 mg, 0.61 mmol) was dissolved in N,N-dimethylformamide (5 ml), potassium carbonate (168 mg, 1.22 mmol) was added, followed by methyl iodide (0.057 ml, 0.91 mmol). The solution was stirred at room temperature for 3 hours. The reaction mixture was quenched by addition of water (10 ml) and the mixture extracted with ethyl acetate. The organic layer was washed with aqueous hydrochloric acid (1N), water and brine, dried over magnesium sulfate and concentrated. The residue was purified by column chromatography on silica (eluent: ethyl acetate, dichloromethane, iso-hexane) to give 5-cyclopropyl-2-methyl-2*H*-[1,2,3]triazole-4-carboxylic acid ethyl ester (51 mg, 43% yield) and 5-cyclopropyl-3-methyl-3*H*-[1,2,3]triazole-4-carboxylic acid ethyl ester (46 mg, 39% yield).
Isomer A ¹H-NMR (400 MHz, CDCl₃): 0.92 (m, 2H, CH₂), 1.02 (m, 2H, CH₂), 1.40 (t, 3H, Me), 2.50 (m, 1H, CH), 4.12 (s, 3H, Me), 4.42 (q, 2H, CH₂) ppm.
Isomer B ¹H-NMR (400 MHz, CDCl₃): 1.01 (m, 2H, CH₂), 1.12 (m, 2H, CH₂), 1.41 (t, 3H, Me), 2.39 (m, 1H, CH), 4.23 (s, 3H, Me), 4.41 (q, 2H, CH₂) ppm.

The esters were reduced as described in Example I6, the alcohols brominated as described in Example I7, and the bromomethyl intermediates coupled as described in Example I39.

### 3) Methods of couplings and oxidations

### Example I39: Preparation of 4-(5,5-dimethyl-4,5-dihydro-isoxazol-3-ylsulfanylmethyl)-5-fluoromethyl-2-methyl-2H-[1,2,3]triazole and 4-(5,5-dimethyl-4,5-dihydro-isoxazol-3-ylsulfanylmethyl)-2,5-dimethyl-2H-[1,2,3]triazole

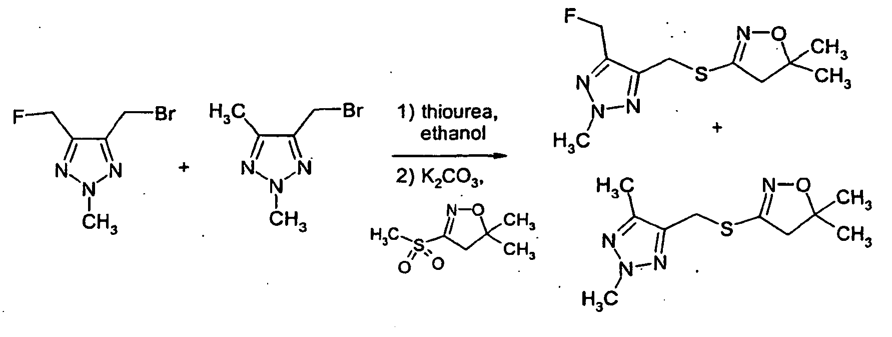

The 4:1-mixture of 4-bromomethyl-5-fluoromethyl-2-methyl-2*H*-[1,2,3]triazole and 4-bromomethyl-2,5-dimethyl-2*H-*[1,2,3]triazole (954 mg, 4.59 mmol) (Example I7) was stirred in ethanol (15 ml) before thiourea (384 mg, 5.05 mmol) was added. The reaction mixture was stirred at room temperature until the entire solid had dissolved. 3-Methanesulfonyl-5,5-dimethyl-4,5-dihydro-isoxazole (prepared according to EP 1364946) (893 mg, 5.05 mmol) and the potassium carbonate (698 mg, 5.05 mmol) were added to the reaction mixture and the reaction mixture was heated at reflux for 2.5 hours. The reaction mixture was cooled to room temperature and concentrated. The residue was partitioned between ethyl acetate (20 ml) and water (20 ml). The phases were separated and the aqueous phase was extracted twice with ethyl acetate. The combined organic extracts were washed with brine, dried over magnesium sulfate and concentrated. The residue was purified by column chromatography on silica gel (eluent: ethyl acetate / hexane) to yield a 4:1-mixture of 4-(5,5-dimethyl-4,5-dihydro-isoxazol-3-ylsulfanyl-methyl)-5-fluoromethyl-2-methyl-2*H*-[1,2,3]triazole and 4-(5,5-dimethyl-4,5-dihydro-isoxazol-3-ylsulfanylmethyl)-2,5-dimethyl-2*H*-[1,2,3]triazole as a colourless oil (1.05 g, 89% yield).
¹H-NMR (400 MHz, CDCl₃): 1.41 (s, 6H, Me), 2.80 (s, 2H, CH₂), 4.17 (d, 3H, Me), 4.34 (s, 2H, CH₂), 5.52 (d, 2H, CH₂) ppm.
¹H-NMR (400 MHz, CDCl₃): 1.42 (s, 6H, Me), 2.30 (s, 3H, Me), 2.82 (s, 2H, CH₂), 4.09 (s, 3H, Me), 4.27 (s, 2H, CH₂) ppm.

The same method was used with 4-bromomethyl-2-methyl-5-trifluoromethyl-2*H-*[1,2,3]triazole as the starting material to give 4-(5,5-dimethyl-4,5-dihydro-isoxazol-3-yl-sulfanylmethyl)-2-methyl-5-trifluoromethyl-2*H-*[1,2,3]triazole.
¹H-NMR (400 MHz, CDCl₃): 1.43 (s, 6H, Me), 2.83 (s, 2H, CH₂), 4.23 (s, 3H, Me), 4.40 (s, 2H, CH₂) ppm.

### Example I40: Preparation of 4-(bromo-difluoro-methyl)-5-(5,5-dimethyl-4,5-dihydro-isoxazol-3-ylsulfanylmethyl)-1-(4-methoxy-benzyl)-1H-[1,2,3]triazole and 5-(bromo-difluoro-methyl)-4-(5,5-dimethyl-4,5-dihydro-isoxazol-3-ylsulfanylmethyl)-1-(4-methoxy-benzyl)-1H-[1,2,3]triazole

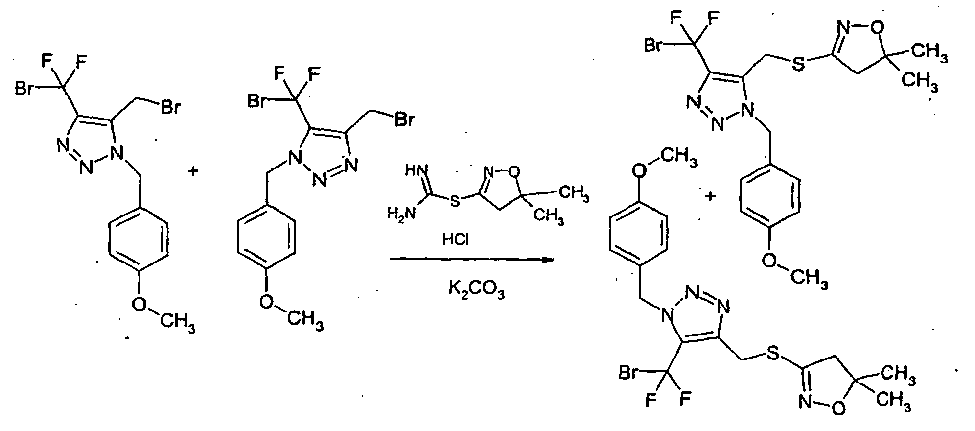

4-(Bromo-difluoro-methyl)-5-bromomethyl-1-(4-methoxy-benzyl)-1*H*-[1,2,3]-triazole and 5-(bromo-difluoro-methyl)-4-bromomethyl-1-(4-methoxy-benzyl)-1*H* [1,2,3]triazole (Example I30) (687 mg, 1.67 mmol) and 2-(5,5-dimethyl-4,5-dihydro-isoxazol-3-yl)-isothiourea hydrochloride (prepared as described in WO 06/068092) (305 mg, 1.67 mmol) were dissolved in acetonitrile (10 ml). Potassium carbonate (576 mg, 4.17 mmol) was added and the mixture was stirred at room temperature for 3 hours. The reaction mixture was quenched by addition of water and extracted several times with ethyl acetate. The combined organic extracts were washed with water, brine, dried over magnesium sulfate and concentrated. The residue was purified by column chromatography on silica gel (eluent: ethyl acetate / hexane) to give the desired product as a 9: 1-mixture of isomers (660 mg, 86% yield).
¹H-NMR (400 MHz, CDCl₃) (major): 1.45 (s, 6H, Me), 2.74 (s, 2H, CH₂), 3.8 (s, 3H, Me), 4.31 (s, 2H, CH₂), 5.68 (s, 2H, CH₂), 6.89 (d, 2H, CH), 7.3 (d, 2H, CH) ppm. ¹H-NMR (400 MHz, CDCl₃) (minor): 1.45 (s, 6H, Me), 2.84 (s, 2H, CH₂), 3.8 (s, 3H, Me), 4.43 (s, 2H, CH₂), 5.6 (s, 2H, CH₂), 6.89 (d, 2H, CH), 7.3 (d, 2H, CH) ppm.

The same method was used with the mixture of methanesulfonic acid 5-acetyl-1-(4-methoxy-benzyl)-1*H*-[1,2,3]triazol-4-ylmethyl ester and methanesulfonic acid 5-acetyl-3-(4-methoxy-benzyl)-3*H-*[1,2,3]triazol-4-ylmethyl ester (Example I31) as the starting material to give a mixture of 1-[5-(5,5-dimethyl-4,5-dihydro-isoxazol-3-ylsulfanylmethyl)-1-(4-methoxy-benzyl)-1*H*-[1,2,3]triazol-4-yl]-ethanone and 1-[5-(5,5-dimethyl-4,5-dihydro-isoxazol-3-ylsulfanylmethyl)-3-(4-methoxy-benzyl)-3*H-*[1,2,3]triazol-4-yl]-ethanone.
¹H-NMR (400 MHz, CDCl₃): 1.52 (s, 6H, Me), 2.68-2.73 (s, 2H, CH₂), 2.62-2.72 (s, 3H, Me), 3.8 (s, 3H, Me), 4.43-4.53 (s, 2H, CH₂), 5.77-5.81 (s, 2H, CH₂), 6.85 (m, 2H, CH), 7.2-7.3 (m, 2H, CH) ppm.

The same method was used with the mixture of 5-methanesulfonyloxymethyl-1-(4-methoxy-benzyl)-1*H*-[1,2,3]triazole-4-carboxylic acid methyl ester and 5-methane-sulfonyloxymethyl-3-(4-methoxy-benzyl)-3*H*-[1,2,3]triazole-4-carboxylic acid methyl ester (Example I31) as the starting material to give a mixture of 5-(5,5-dimethyl-4,5-dihydro-isoxazol-3-ylsulfanylmethyl)-1-(4-methoxy-benzyl)-1*H*-[1,2,3]triazole-4-carboxylic acid methyl ester and 5-(5,5-dimethyl-4,5-dihydro-isoxazol-3-ylsulfanylmethyl)-3-(4-methoxy-benzyl)-3*H*-[1,2,3]triazole-4-carboxylic acid methyl ester.
¹H-NMR (400 MHz, CDCl₃): 1.52-1.53 (s, 6H, Me), 2.75-2.85 (s, 2H, CH₂), 3.8 (s, 3H, Me), 3.92-3.97 (s, 3H, Me), 4.43-4.53 (s, 2H, CH₂), 5.77-5.81 (s, 2H, CH₂), 6.85 (m, 2H, CH), 7.2-7.3 (m, 2H, CH) ppm.

### Example I41: Preparation of 4-(5,5-dimethyl-4,5-dihydro-isoxazol-3-ylsulfanylmethyl)-2,5-dimethyl-2H-[1,2,3]triazole

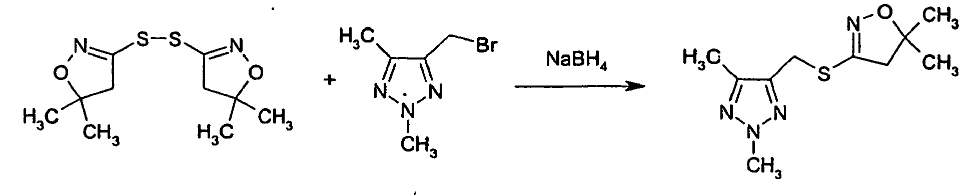

2-(5,5-Dimethyl-4,5-dihydro-isoxazol-3-yl)-disulfide (Example I5) (300 mg, 1.15 mmol) and sodium hydroxide (88 mg, 2.19 mmol) were dissolved in dry ethanol (10 ml) and sodium borohydride (88 mg, 2.30 mmol) was added at room temperature. The reaction mixture was stirred at room temperature for 10 minutes. 4-Bromomethyl-2,5-dimethyl-2*H*-[1,2,3]triazole (Example I7, Example I11) (460 mg, 2.42 mmol) was added. The reaction mixture stirred at room temperature for 30 minutes and then concentrated. The residue was partitioned between water and ethyl acetate. The phases were separated and the organic phase was washed water and brine, dried over magnesium sulfate and concentrated. The residue was purified by column chromatography on silica gel (eluent: 0-50% ethyl acetate / hexane) to give 4-(5,5-dimethyl-4,5-dihydro-isoxazol-3-ylsulfanyl-methyl)-2,5-dimethyl-2*H-*[1,2,3]triazole (470 mg, 85% yield).
¹H-NMR (400 MHz, CDCl₃): 1.42 (s, 6H, Me), 2.30 (s, 3H, Me), 2.82 (s, 2H, CH₂), 4.09 (s, 3H, Me), 4.27 (s, 2H, CH₂) ppm.

### Example I42: Alternative preparation of 4-(5,5-dimethyl-4,5-dihydro-isoxazol-3-yl-sulfanylmethyl)-2,5-dimethyl-2H-[1,2,3]triazole

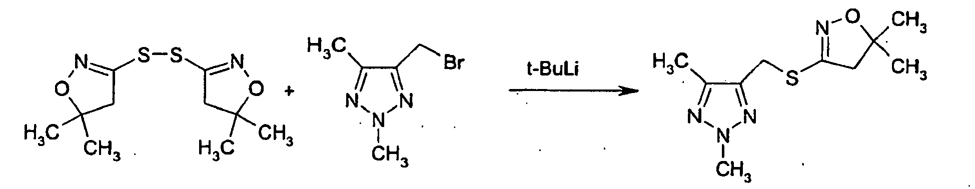

*tert*-Butyl lithium (1.7 M in hexane) (1.12 ml, 1.89 mmol) was added to diethyl ether (5 ml) at -78°C. A solution of 4-bromomethyl-2,5-dimethyl-2*H-*[1,2,3]triazole (Example I7, Example I11) (300 mg, 1.58 mmol) in diethyl ether (5 ml) was added at -78°C and the reaction mixture stirred at -78°C for 5 minutes. A solution of 2-(5,5-dimethyl-4,5-dihydro-isoxazol-3-yl)-disulfide (Example 15) (410 mg, 1.58 mmol) in tetrahydrofuran (5 ml) was added at -78°C and the reaction mixture stirred at -78°C for 10 minutes. The reaction mixture was allowed to warm to room temperature within 1 hour. The reaction mixture was quenched by addition of aqueous ammonium chloride (saturated) and the mixture extracted with ethyl acetate. The combined organic extracts were washed with water and brine, dried over magnesium sulfate and concentrated. The residue was purified by column chromatography on silica gel (eluent: 0-50% ethyl acetate / hexane) to give 4-(5,5-dimethyl-4,5-dihydro-isoxazol-3-ylsulfanylmethyl)-2,5-dimethyl-2*H*-[1,2,3]triazole (154 mg, 41% yield).

### Example I43: Alternative preparation of 4-(5,5-dimethyl-4,5-dihydro-isoxazol-3-yl-sulfanylmethyl)-2,5-dimethyl-2H-[1,2,3]triazole

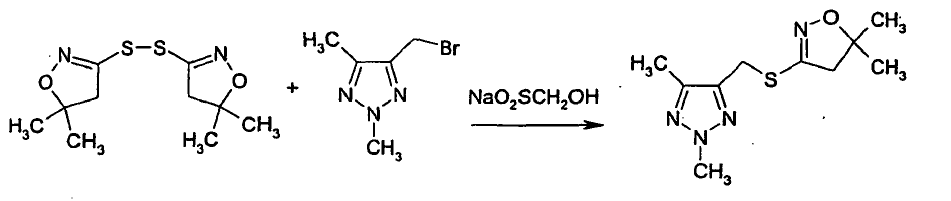

2-(5,5-Dimethyl-4,5-dihydro-isoxazol-3-yl)-disulfide (Example I5) (137 mg, 0.53 mmol) was dissolved in N,N-dimethylformamide (4 ml) and potassium carbonate (290 mg, 2.1 mmol) and sodium hydroxymethylsulfinate (Rongalite) (323 mg, 2.1 mmol) were added. The mixture was cooled to 0°C and 4-bromomethyl-2,5-dimethyl-2*H*-[1,2,3]-triazole (Example I7, Example I11) (200 mg, 1.05 mmol) was added dropwise as a solution in N,N-dimethylformamide (3 ml). The mixture was stirred at room temperature for 1.5 hours and then poured into water. The mixture was extracted several times with diethyl ether. The combined organic extracts were washed three times with water, dried over magnesium sulfate and concentrated. The residue was purified by column chromatography on silica gel (eluent: ethyl acetate / iso-hexane) to give 4-(5,5-dimethyl-4,5-dihydro-isoxazol-3-ylsulfanylmethyl)-2,5-dimethyl-2*H*-[1,2,3]triazole (170 mg, 67.2%).

### Example I44: Preparation of 4-(5,5-dimethyl-4,5-dihydro-isoxazole-3-sulfinylmethyl)-2,5-dimethyl-2H-[1,2,3]triazole

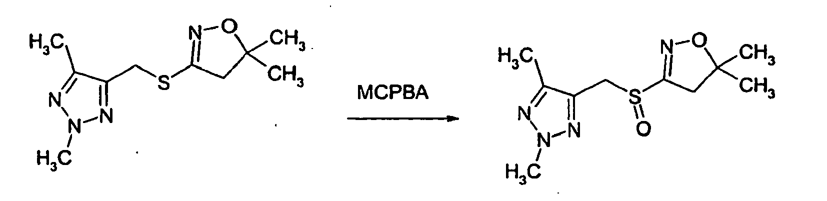

4-(5,5-Dimethyl-4,5-dihydro-isoxazole-3-sulfinylmethyl)-2,5-dimethyl-2*H-*[1,2,3]triazole (Example I39) (75% pure, 3.53g) was dissolved in dichloromethane and 3-chloroperoxybenzoic acid (MCPBA) (60% by weight) (4.2 g, 14.6 mmol) was added at 0°C. The mixture was stirred for 30 minutes at 0°C. The reaction mixture was quenched by addition of aqueous sodium metabisulfite (20%) (80 ml) and the mixture stirred for 10 minutes. The phases were separated and the aqueous phase was washed with aqueous sodium bicarbonate (saturated), water and brine, dried over magnesium sulfate and concentrated. The residue was purified by column chromatography on silica gel (eluent: ethyl acetate / iso-hexane) to give 4-(S,5-dimethyl-4,5-dihydro-isoxazole-3-sulfinyl-methyl)-2,5-dimethyl-2*H*-[1,2,3]triazole (2.52 g, 89.7% yield)
¹H-NMR (400 MHz, CDCl₃): 1.5 (s, 3H, Me), 1.51 (s, 3H, Me), 2.32 (s, 3H, Me), 3.05 (d, 1H, CH₂), 3.18 (d, 1H, CH₂), 4.1 (s, 3H, Me), 4.3 (d, 1H, CH₂), 4.35 (d, 1H, CH₂) ppm.

### Example I45: Preparation of 4-(5,5-dimethyl-4,5-dihydro-isoxazol-3-ylsulfonylmethyl)-5-fluoromethyl-2-methyl-2H-[1,2,3]triazole and 4-(5,5-dimethyl-4,5-dihydro-isoxazol-3-ylsulfonylmethyl)-2,5-dimethyl-2H-[1,2,3]triazole

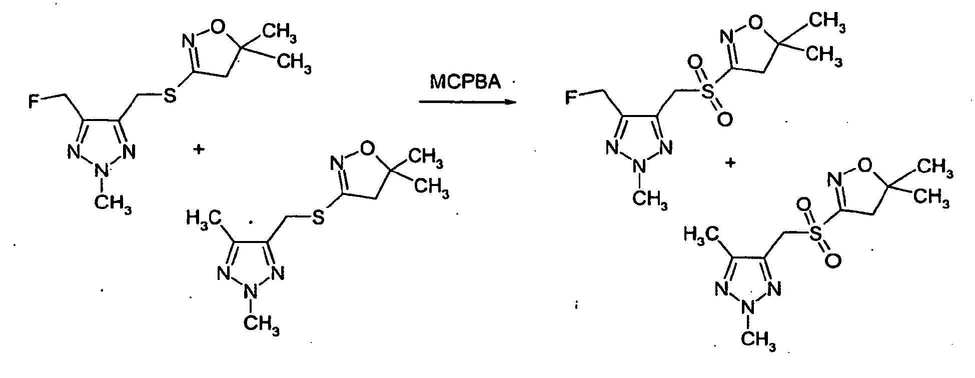

The 4:1-mixture of 4-(5,5-dimethyl-4,5-dihydro-isoxazol-3-ylsulfanylmethyl)-5-fluoromethyl-2-methyl-2*H-*[1,2,3]triazole and 4-(5,5-dimethyl-4,5-dihydro-isoxazol-3-ylsulfanylmethyl)-2,5-dimethyl-2*H-*[1,2,3]triazole (Example I39) (672 mg, 2.6 mmol) was dissolved in dichloromethane (30 ml) and 3-chloroperoxybenzoic acid (MCPBA) (1.46 g, 6.51 mmol) was added. The solution was stored at room temperature for 16 hours. The reaction mixture was quenched by addition of aqueous sodium metabisulfite (10%). The mixture was diluted with water and aqueous sodium hydroxide (2M) was added. The phases were separated and the aqueous phase extracted with dichloromethane. The combined organic extracts were washed twice with aqueous sodium hydroxide (2M), dried over magnesium sulfate and concentrated. The residue was purified by column chromatography on silica gel (eluent: ethyl acetate / hexane) to give 4-(5,5-dimethyl-4,5-dihydro-isoxazol-3-ylsulfanylmethyl)-5-fluoromethyl-2-methyl-2*H-*[1,2,3]triazole (430 mg, 70% yield) as a white solid and 4-(5,5-dimethyl-4,5-dihydro-isoxazol-3-ylsulfanylmethyl)-2,5-dimethyl-2*H-*[1,2,3]triazole (82 mg, 57% yield) as a white solid.
¹H-NMR (400 MHz, CDCl₃): 1.51 (s, 6H, Me), 3.05 (s, 2H, CH₂),4.21 (s, 3H, Me), 4.77 (s, 2H, CH₂), 5.57 (d, 2H, CH₂) ppm.
¹H-NMR (400 MHz, CDCl₃): 1.50 (s, 6H, Me), 2.36 (s, 3H, Me), 3.01 (s, 2H, CH₂), 4.14 (s, 3H, Me), 4.64 (s, 2H, CH₂) ppm.

The same method was used with 4-(5,5-dimethyl-4,5-dihydro-isoxazol-3-yl-sulfanylmethyl)-2-methyl-5-trifluoromethyl-2*H-*[1,2,3]triazole as the starting material to give 4-(5,5-dimethyl-4,5-dihydro-isoxazol-3-ylsulfonylmethyl)-2-methyl-5-trifluoromethyl-2*H*-[1,2,3]triazole.
¹H-NMR (400 MHz, CDCl₃): 1.51 (s, 6H, Me), 3.09 (s, 2H, CH₂), 4.29 (s, 3H, Me), 4.83 (s, 2H, CH₂) ppm.

### 4) Methods for halogenations and alkylations

### Example P1: Preparation of 4-[(5,5-dimethyl-4,5-dihydro-isoxazole-3-sulfonyl)-difluoromethyl]-5-fluoromethyl-2-methyl-2H-[1,2,3]triazole

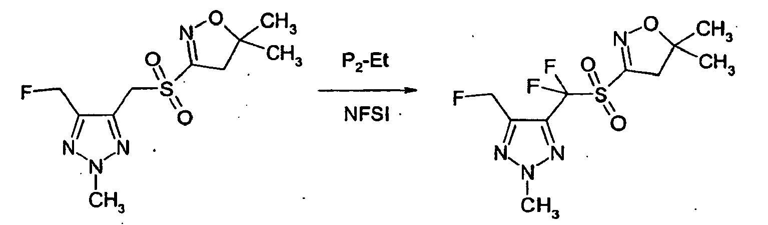

4-(5,5-Dimethyl-4,5-dihydro-isoxazole-3-sulfonylmethyl)-5-fluoromethyl-2-methyl-2*H-*[1,2,3]triazole (Example I45) (117 mg, 0.4 mmol) was stirred in dry tetrahydrofuran (2 ml) under nitrogen at 0°C. 1-Ethyl-2,2,4,4,4-pentakis(dimethylamino)-2-lambda⁵,4lambda⁵-catenadi(phosphazene) (P₂-Et) (0.28 ml, 0.82 mmol) was added dropwise, and then N-fluorobenzene sulfonimide (NFSI) (257 mg, 0.82 mmol) was added in one portion. The reaction was stirred for 1 hour at room temperature. The reaction mixture was concentrated and the residue was purified by column chromatography on silica gel (eluent: ethyl acetate / hexane) to give Compound No. 1.01 of Table 1 as a white solid (66 mg, 50% yield).

The same method was used to synthesise the following compounds: Compound No. 1.05 and 1.09 of Table 1.

### Example P2: Preparation of 4-[chloro-(5,5-dimethyl-4,5-dihydro-isoxazole-3-sulfonyl)-methyl]-5-fluoromethyl-2-ethyl-2H-[1,2,3]triazole

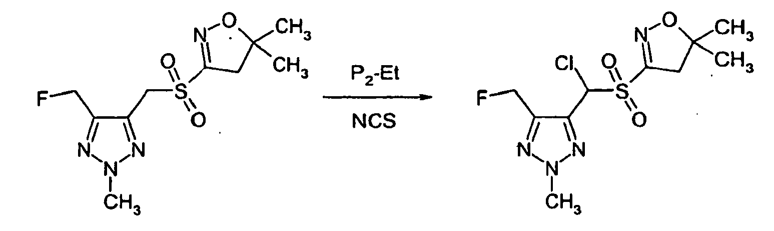

4-(5,5-Dimethyl-4,5-dihydro-isoxazole-3-sulfonylmethyl)-5-fluoromethyl-2-methyl-2*H*-[1,2,3]triazole (Example I45) (152 mg, 0.52 mmol) was dissolved in tetrahydrofuran (2 ml) at room temperature under nitrogen and 1-ethyl-2,2,4,4,4-pentakis(dimethylamino)-2-lambda⁵,4lambda⁵-catenadi-(phosphazene) (P₂-Et) (0.18 ml, 0.55 mmol) was added. N-Chlorosuccinimide (NCS) (73 ing, 0.55 mmol) was then added in one portion, and the reaction mixture was stirred for 1 hour. The reaction mixture was concentrated and the residue was purified by column chromatography on silica gel (eluent: ethyl acetate / hexane) to give Compound No. 1.02 of Table 1 as a colourless oil (116 mg, 68% yield).

The same method was used to synthesise the following compound: Compound No. 1.06 of Table 1.

### Example P3: Preparation of 4-[chloro-(5,5-dimethyl-4,5-dihydro-isoxazole-3-sulfonyl)-fluoro-methyl]-5-fluoromethyl-2-methyl-2H-[1,2,3]triazole

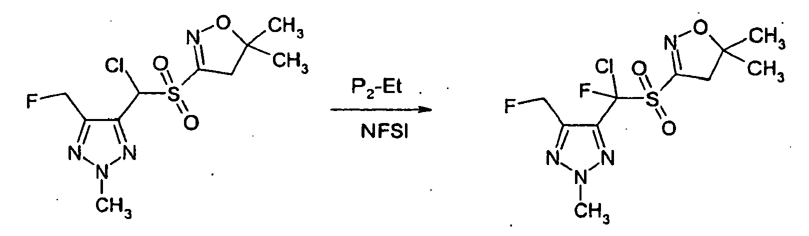

4-[Chloro-(5,5-dimethyl-4,5-dihydro-isoxazole-3-sulfonyl)-methyl]-5-fluoromethyl-2-ethyl-2*H*-[1,2,3]triazole (Example P2) (116 mg, 0.36 mmol) was dissolved in tetrahydrofuran (2 ml) under nitrogen and 1-ethyl-2,2,4,4,4-pentakis(dimethylamino)-2-lambda ⁵4lambda⁵-catenadi(phosphazene) (P₂-Et) (0.13 ml, 0.38 mmol) was added at room temperature. N-Fluorobenzene sulfonimide (NFSI) (118 mg, 0.38 mmol) was added in one portion at room temperature and the reaction mixture was stirred for 1 hour at room temperature. The reaction mixture was concentrated and the residue was purified by column chromatography on silica gel (eluent: ethyl acetate / hexane) to give Compound No. 1.03 of Table 1 as a white solid (85 mg, 69% yield).

The same method was used to synthesise the following compounds: Compound No. 1.07 and 1.10 of Table 1.

### Example P4: Preparation of 4-[(5,5-dimethyl-4,5-dihydro-isoxazole-3-sulfonyl)-fluoromethyl]-2,5-dimethyl-2H-[1,2,3]triazole

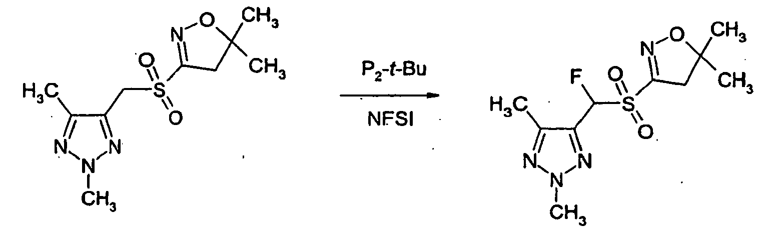

4-(5,5-Dimethyl-4,5-dihydro-isoxazole-3-sulfonylmethyl)-2,5-dimethyl-2*H-*[1,2,3]triazole (Example I45) (112 mg, 0.4 mmol) was stirred in dry tetrahydrofuran (3 ml) under nitrogen at 0°C. 1-*tert*-Butyl-2,2,4,4,4-pentakis(dimethylamino)-2-lambda⁵, 4lambda⁵-catenadi(phosphazene) (P₂-*t*-Bu) (2M in THF) (0.2 ml, 0.4 mmol) was added dropwise at 0°C, and N-fluorobenzene sulfonimide (NFSI) (130 mg, 0.4 mmol) was added in one portion at 0°C. The reaction was stirred for 30 minutes at room temperature. The reaction mixture was concentrated and the residue was purified by column chromatography on silica gel (eluent: ethyl acetate / hexane) to give Compound No. 1.04 of Table 1 as a white solid (35 mg, 29.3% yield).

The same method was used to synthesise the following compounds: Compound No. 1.11, 1.12 and 1.28 of Table 1.

### Example P5: Preparation of 4-[(5,5-dimethyl-4,5-dihydro-isoxazole-3-sulfinyl)-difluoromethyl]-2,5-dimethyl-2H-[1,2,3]triazole

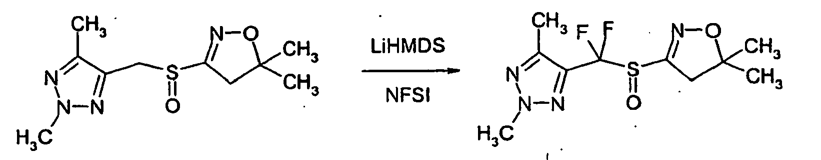

4-(5,5-Dimethyl-4,5-dihydro-isoxazole-3-sulfinylmethyl)-2,5-dimethyl-2*H-*[1,2,3]triazole (Example 144) (200 mg, 0.78 mmol) and N-fluorobenzene sulfonimide (NFSI) (516 mg, 1.64 mmol) were dissolved in dry tetrahydrofuran under nitrogen and cooled to -78°C. Lithium hexamethyldisilazide (LiHMDS) (1M solution in THF) (1.64 ml, 1.64 mmol) was added dropwise at -78°C and the solution was stirred and allowed to warm slowly to -20°C over 3 hours. The reaction mixture was quenched at -20°C by addition of several drops of aqueous ammonium chloride (saturated). The mixture was allowed to warm to room temperature, concentrated onto silica gel and purified by column chromatography on silica gel (eluent: hexane / ethyl acetate) to give Compound No. 1.08 of Table 1 (160 mg, 70% yield).

The same method was used to synthesise the following compounds:
Compound No. 1.16, 1.18, 1.27, 1.36, 1.44, 1.47, 1.49, 1.51 and 1.58 of Table 1,
Compound No. 2.07 of Table 2, and
Compound No. 3.03 and 3.10 of Table 3.

### Example P6: Preparation of 4-(bromo-difluoro-methyl)-5-[(5,5-dimethyl-4,5-dihydro-isoxazole-3-sulfonyl)-difluoro-methyl]-2-methyl-2H-[1,2,3]triazole

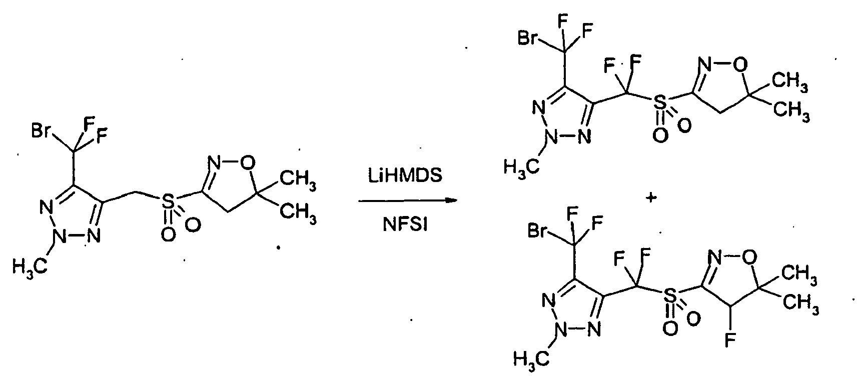

4-(Bromo-difluoro-methyl)-5-(5,5-dimethyl-4,5-dihydro-isoxazole-3-sulfonylmethyl)-2-methyl-2*H*-[1,2,3]triazole (see Example I32 and Example I45) (500 mg, 1.3 mmol) and N-fluorobenzene sulfonimide (NFSI) (820 mg, 2.6 mmol) were dissolved in dry tetrahydrofuran under nitrogen and cooled to -78°C. Lithium hexamethyldisilazide (LiHMDS) (1M in THF) (2.8 ml, 2.8 mmol) was added dropwise at -78°C and the solution was stirred and allowed to warm slowly to room temperature over 4 hours. The reaction mixture was quenched at by addition of aqueous ammonium chloride (saturated) and was extracted several times with ethyl acetate. The combined organic fractions were washed with water and brine, dried over magnesium sulfate and concentrated. The residue was purified by column chromatography on silica gel (eluent: ethyl acetate / hexane) to give Compound No. 1.59 of Table 1 (209 mg, 37% yield) and 4-(bromo-difluoro-methyl)-5-[difluoro-(4-fluoro-5,5-dimethyl-4,5-dihydro-isoxazole-3-sulfonyl)-methyl]-2-methyl-2*H*-[1,2,3]triazole (100 mg, 17.4% yield).

The same method was used to synthesise the following compounds:
Compound No. 1.13, 1.15, 1.17, 1.23, 1.26, 1.29, 1.32, 1.35, 1.41, 1.43, 1.48, 1.50, 1.52, 1.54, 1.57, 1.61 and 1.63 of Table 1,
Compound No. 3.04 and 3.07 of Table 3, and
Compound No. 4.01 of Table 4.

### Example P7: Preparation of 5-cyclopropyl-4-[(5,5-dimethyl-4,5-dihydro-isoxazole-3-sulfinyl)-fluoro-methyl]-1-methyl-1H-[1,2,3]triazole and 5-cyclopropyl-4-[(5,5-dimethyl-4,5-dihydro-isoxazole-3-sulfinyl)-difluoro-methyl]-1-methyl-1H-[1,2,3]triazole

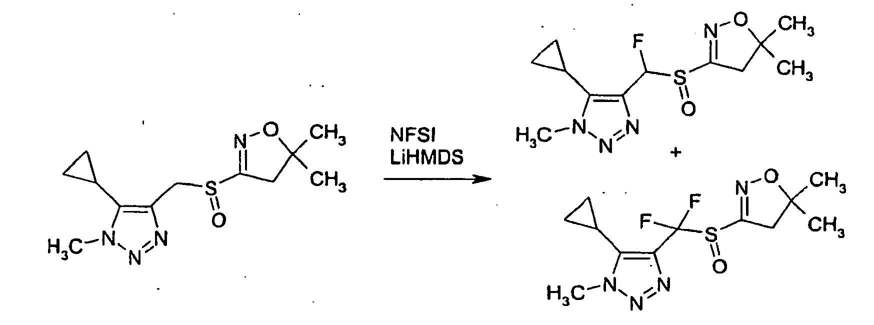

5-Cyclopropyl-4-(5,5-dimethyl-4,5-dihydro-isoxazole-3-sulfinylmethyl)-1-methyl-1*H-*[1,2,3]triazole (95 mg, 0.34 mmol) was dissolved in dry tetrahydrofuran (5 ml) and cooled to -78°C. Lithium hexamethyldisilazide (LiHMDS) (1M in THF) (0.51 ml, 0.51 mmol) was added dropwise and the solution was stirred for 5 minutes at -78°C. N-fluorobenzene sulfonimide (NFSI) (160 mg, 0.51 mmol) was added and the reaction mixture stirred for 2 hours at -78°C. The reaction mixture was quenched by addition of aqueous ammonium chloride (saturated) (3ml) and extracted with ethyl acetate. The organic phase was washed with water and brine, dried over magnesium sulfate and concentrated. The residue was purified by column chromatography on silica gel (eluent: ethyl acetate / iso-hexane) to give Compound No: 2.10 of Table 2 (42 mg, 39% yield) and Compound No. 2.11 of Table 2 (45 mg, 44% yield).

The same method was used to synthesise the following compounds:
Compound No. 1.21, 1.22, 1.24, 1.25, 1.30, 1.31, 1.33, 1.34, 1.39, 1.40, 1.55, 1.56, 1.68 and 1.69 of Table 1,
Compound No. 2.03 and 2.04 of Table 2, and
Compound No. 3.05 and 3.06 of Table 3.

### Example P8: Preparation of 5-cyclopropyl-4-[(5,5-dimethyl-4,5-dihydro-isoxazole-3-sulfonyl)-fluoro-methyl]-1-methyl-1H-[1,2,3]triazole and 5-cyclopropyl-4-[(5,5-dimethyl-4,5-dihydro-isoxazole-3-sulfonyl)-difluoro-methyl]-1-methyl-1H-[1,2,3]-triazole

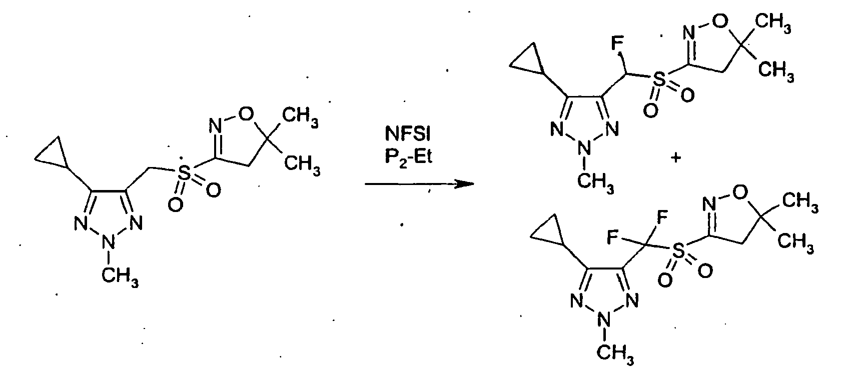

5-Cyclopropyl-4-(5,5-dimethyl-4,5-dihydro-isoxazole-3-sulfonylmethyl)-1-methyl-1*H-*[1,2,3]triazole (200 mg, 0.67 mmol) and N-fluorobenzene sulfonimide (NFSI) (338 mg, 1.07 mmol) were dissolved in dry tetrahydrofuran (10 ml) and 1-ethyl-2,2,4,4,4-pentakis(dimethylamino)-2-lambda⁵,4lambda⁵-catenadi(phosphazene) (P₂-Et) (364 mg, 1.07 mmol) was added dropwise to give a yellow solution. After the addition was complete, the solution was stirred for 2 hours at room temperature. The mixture was evaporated onto silica gel and purified by column chromatography (eluent: ethyl acetate/dichloromethane/iso-hexane) to give Compound No. 1.66 of Table 1 (47 mg, 21% yield) and Compound No. 1.67 of Table 1 (115 mg, 54% yield).

The same method was used to synthesise the following compounds:
Compound No. 1.19, 1.20, 1.37, 1.38, 1.45 and 1.46 of Table 1,
Compound No. 2.01, 2.02, 2.05, 2.06, 2.08 and 2.09 of Table 2, and
Compound No. 3.01, 3.02, 3.08 and 3.09 of Table 3.

The compounds mentioned in the following Tables can be prepared in analogous manner.

**Table 1: Compounds of formula I.1**

| | | | | | | | |
|---|---|---|---|---|---|---|---|
| 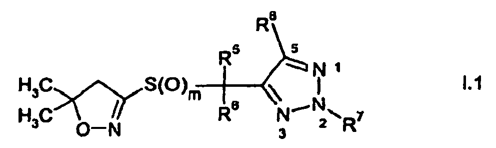 | | | | | | | |

| **No.** | **m** | **R⁵** | **R⁶** | **R⁷** | **R⁸** | **M.p. [°C]** | **¹H-NMR (400 MHz, CDCl₃)** |
|---|---|---|---|---|---|---|---|
| 1.01 | 2 | F | F | Me | -CH₂F | 54 | 1.57 (s, 6H, Me), 3.19 (s, 2H, CH₂), 4.31 (s, 3H, Me), 5.54 (s, 2H, CH₂). |
| 1.03 | 2 | Cl | F | Me | -CH₂F | 80 | 1.55 (s, 3H, Me), 1.57 (s, 3H, Me), 3.15 (d, 1H, CH₂), 3.2 (d, 1H, CH₂), 4.29 (s, 3H, Me), 5.56 (d, 2H, CH₂). |
| 1.05 | 2 | F | F | Me | Me | 75 | 1.56 (s, 6H, Me), 2.45 (s, 3H, Me), 3.17 (s, 2H, CH₂), 4.21 (s, 3H, Me). |
| 1.07 | 2 | Cl | F | Me | Me | | 1.55 (s, 6H, Me), 2.4 (s, 3H, Me), 3.25 (d, 1H, CH₂), 3.4 (d, 1H, CH₂), 4.35 (s, 3H, Me). |
| 1.08 | 1 | F | F | Me | Me | | 1.45 (s, 3H, Me), 1.46 (s, 3H, Me), 3.35 (s, 3H, Me), 2.9 (d, 1H, CH₂), 3.15 (d, 1H, CH₂), 4.1 (s, 3H, Me). |
| 1.09 | 2 | F | F | Me | -CF₃ | 80 | 1.57 (s, 6H, Me), 3.19 (s, 2H, CH₂), 4.38 (s, 3H, Me). |
| 1.10 | 2 | Cl | F | Me | -CF₃ | 66-69 | 1.54 (s, 3H, Me), 1.56 (s, 3H, Me), 3.18 (d, 1H, CH₂), 3.22 (d, 1H, CH₂), 4.35 (s, 3H, Me). |
| 1.13 | 2 | F | F | Et | Et | 50-51 | 1.35 (t, 3H, Me), 1.5-1.7 (m, 9H, Me), 2.7 (q, 2H, CH₂), 3.35 (s, 2H, CH₂), 4.45 (q, 2H, CH₂). |
| 1.15 | 2 | F | F | Et | -CF₃ | | 1.57 (s, 6H, Me), 1.66 (t, 3H, Me), 3.19 (s, 2H, CH₂), 4.62 (q, 2H, CH₂)- |
| 1.16 | 1 | F | F | Et | -CF₃ | | 1.50 (s, 6H, Me), 1.64 (t, 3H, Me), 3.06 (d, 1H, CH₂), 3.26 (d, 1H, CH₂), 4.59 (q, 2H, CH₂). |
| 1.17 | 2 | F | F | 'Pr | -CF₃ | | 1.55 (s, 6H, Me), 1.66 (d, 6H, Me), 3.19 (s, 2H, CH₂), 4.97 (sept, 1 H, CH). |
| 1.18 | 1 | F | F | 'Pr | -CF₃ | | 1.49 (s, 6H, Me), 1.63 (d, 6H, Me), 3.06 (d, 1H, CH₂), 3.26 (d, 1H, CH₂), 4.93 (sept, 1H, CH). |
| 1.20 | 2 | F | F | -CHF₂ | Me | | 1.56 (s, 6H, Me), 2.70 (s, 3H, Me), 3.19 (s, 2H, CH₂), 7.62 (t, 1H, CH). |
| 1.22 | 1 | F | F | -CHF₂ | Me | | 1.49 (s, 3H, Me), 1.51 (s, 3H, Me), 2.66 (s, 3H, Me), 2.95 (d, 1H, CH₂), 3.13 (d, 1H, CH₂), 7.60 (t, 1H, CH). |
| 1.23 | 2 | F | F | -CH₂CH ₂OCH₃ | -CF₃ | | 1.57 (s, 6H, Me), 3.18 (s, 2H, CH₂), 3.35 (s, 3H, Me), 3.95 (t, 2H, CH₂), 4.72 (t, 2H, CH₂). |
| 1.25 | 1 | F | F | -CH₂CH ₂OCH₃ | -CF₃ | | 1.48 (s, 3H, Me), 1.49 (s, 3H, Me), 3.04 (d, 1H, CH₂), 3.25 (d, 1H, CH₂), 3.35 (s, 3H, Me), 3.93 (t, 2H, CH₂), 4.69 (t, 2H, CH₂). |
| 1.26 | 2 | F | F | Me | Et | | 1.29 (t, 3H, Me), 1.55 (s, 6H, Me), 2.83 (q, 2H, CH₂), 3.16 (s, 2H, CH₂), 4.21 (s, 3H, Me). |
| 1.27 | 1 | F | F | Me | Et | | 1.29 (t, 3H, Me), 1.50 (s, 6H, Me), 2.79 (q, 2H, CH₂), 3.01 (d, 1H, CH₂), 3.23 (d, 1H, CH₂), 4.18 (s, 3H, Me). |
| 1.29 | 2 | F | F | -^{c}Pe | -CF₃ | | 1.57 (s, 6H, Me), 1.7-1.8 (m, 2H, CH₂),1.85-1.95.(m, 2H, CH₂), 2.15-2.3 (m, 4H, CH₂), 3.18 (s, 2H, CH₂),5.05-5.1 (m, 1H, CH). |
| 1.31 | 1 | F | F | -^{c}Pe | -CF₃ | | 1.49 (s, 6H, Me), 1.7-1.8 (m, 2H, CH₂), 1.85-1.95 (m, 2H, CH₂), 2.1-2.3 (m, 4H, CH₂), 3.05 (d, 1H, CH₂), 3.26 (d, 1H, CH₂), 5.05-5.15 (m, 1H, CH). |
| 1.32 | 2 | F | F | -CH₂CH =CH₂ | -CF₃ | | 1.57 (s, 6H, Me), 3.18 (s, 2H, CH₂), 5.16 (d, 2H, CH₂), 5.39-5.46 (m, 2H, CH₂), 6.04-6.14 (m, 1H, CH). |
| 1.34 | 1 | F | F | -CH₂CH =CH₂ | -CF₃ | | 1.49 (s, 6H, Me), 3.06 (d, 1H, CH₂), 3.25 (d, 1H, CH₂), 5.13 (d, 2H, CH₂), 5.37-5.45 (m, 2H, CH₂), 6.02-6.12 (m, 1H, CH). |
| 1.35 | 2 | F | F | Me | H | | 1.56 (s, 6H, Me), 3.17 (s, 2H, CH₂), 4.31 (s, 3H, Me), 7.98 (s, 1H, CH). |
| 1.36 | 1 | F | F | Me | H | | 1.49 (s, 3H, Me), 1.50 (s, 3H, Me), 2.95 (d, 1H, CH₂), 3.21 (d, 1H, CH₂), 4.28 (s, 3H, Me), 7.89 (s, 1H, CH). |
| 1.38 | 2 | F | F | Et | H | | 1.56 (s, 6H, Me), 1.62 (t, 3H, Me), 3.17 (s, 2H, CH₂), 4.57 (q, 2H, CH₂), 7.98 (s, 1H, CH). |
| 1.40 | 1 | F | F | Et | H | | 1.49 (s, 3H, Me), 1.50 (s, 3H, Me), 1.60 (t, 3H, Me), 2.94 (d, 1H, CH₂), 3.21 (d, 1H, CH₂), 4.54 (q, 2H, CH₂), 7.89 (s, 1H, CH). |
| 1.41 | 2 | F | F | 'Pr | Me | 94-95 | 1.56 (s, 6H, Me), 1.57 (d, 6H, Me), 2.45 (s, 3H, Me), 3.17 (s, 2H, CH₂), 4.81 (sept, 1H, CH). |
| 1.43 | 2 | F | F | Me | -OCH₃ | | 1.55 (s, 6H, Me), 3.18 (s, 2H, CH₂), 4.01 (s, 3H, Me), 4.13 (s, 3H, Me). |
| 1.44 | 1 | F | F | Me | -OCH₃ | | 1.49 (s, 6H, Me), 3.07 (dd, 1H, CH₂), 3.24 (d, 1H, CH₂), 3.99 (s, 3H, Me), 4.09 (s, 3H, Me). |
| 1.46 | 2 | F | F | Me | Br | 96-99 | 1.57 (s, 6H, Me), 3.18 (s, 2H, CH₂), 4.28 (s, 3H, Me). |
| 1.47 | 1 | F | F | Me | Br | | 1.50 (s, 6H, Me), 3.05 (d, 1H, CH₂), 3.24 (d, 1H, CH₂), 4.25 (s, 3H, Me). |
| 1.48 | 2 | F | F | -CH₂^{c}B u | -CF₃ | 66 | 1.54 (s, 3H, Me), 1.57 (s, 3H, Me), 1.75-1.95 (m, 4H, CH₂), 2.1-2.2 (m, 2H, CH₂), 2.95-3.05 (m, 1H, CH), 3.18 (s, 2H, CH₂), 4.56 (d, 2H, CH₂). |
| 1.49 | 1 | F | F | -CH₂^{c}B u | -CF₃ | | 1.45 (s, 6H, Me). 1.75-1.95 (m. 4H, CH₂). 2.05-2.2 (m, 2H, CH₂),2.95-3.05 (m, 1H, CH), 3.05 (d, 1H, CH₂), 3.25 (d, 1H, CH₂),4.55 (d, 2H, CH₂). |
| 1.50 | 2 | F | F | Me | -CO₂Me | | 1.57 (s, 6H, Me), 3.22 (s, 2H, CH₂). 3.98 (s, 3H, Me), 4.36 (s, 3H, Me). |
| 1.51 | 1 | F | F | Me | -CO₂Me | | 1.47 (s, 3H, Me), 1.49 (s, 3H, Me), 3.09 (d, 1H, CH₂), 3.25 (d, 1 H, CH₂), 4.0 (s, 3H, Me), 4.33 (s, 3H, Me). |
| 1.52 | 2 | F | F | Me | -COMe | | 1.56 (s, 6H, Me), 2.62 (s, 3H, Me), 3.26 (s, 2H, CH₂), 4.34 (s, 3H, Me). |
| 1.54 | 2 | F | F | Me | -OCHF₂ | | 1.56 (s, 6H, Me), 3.18 (s, 2H, CH₂). 4.21 (s, 3H, Me), 6.88 (t, 1H, CH). |
| 1.56 | 1 | F | F | Me | -OCHF₂ | | 1.50 (s, 3H, Me), 1.50 (s, 3H, Me), 3.06 (d, 1H, CH₂), 3.25 (d, 1H, CH₂), 4.18 (s, 3H, Me), 6.87 (t, 1H, CH). |
| 1.57 | 2 | F | F | Et | Me | | 1.56 (s, 6H, Me), 1.58 (t, 3H, Me), 2.46 (s, 3H, Me), 3.17 (s, 2H, CH₂), 4.47 (q, 2H, CH₂). |
| 1.58 | 1 | F | F | Et | Me | | 1.49 (s, 6H, Me), 1.56 (t, 3H, Me), 2.42 (s, 3H, Me), 2.99 (d, 1H, CH₂), 3.22 (d, 1H, CH₂), 4.43 (q, 2H, CH₂). |
| 1.59 | 2 | F | F | Me | -CF₂Br | oil | 1.55 (s, 6H, Me), 3.19 (s, 2H, CH₂), 4.45 (s, 3H, Me). |
| 1.61 | 2 | F | F | Me | -CHF₂ | oil | 1.45 (s, 6H, Me), 3.20 (s, 2H, CH₂), 4.32 (s, 3H, Me), 6.90 (t, 1H. CH). |
| 1.63 | 2 | F | F | Me | -CHFC H₃ | | 1.56 (s, 6H, Me), 1.78 (dd, 3H, Me), 3.3 (d, 1H, CH₂), 3.4 (d, 1H, CH₂), 4.29 (s, 3H, Me), 5.28 (dq, 1H, CH). |
| 1.65 | 2 | F | F | Ph | Me | 144-146 | 1.55 (s, 6H, Me), 2.57 (s, 3H, Me), 3.18 (s, 2H, CH₂), 7.4-7.45 (m, 1H, CH), 7.45-7.5 (m, 2H, CH), 8.0-8.1 (m, 2H, CH). |
| 1.66 | 2 | F | F | Me | ^{c}Pr | | 0.96 (m, 2H, CH₂),1.03 (m, 2H, CH₂),1.56 (s, 6H, Me), 2.10 (m, 1H, CH), 3.18 (s, 2H, CH₂), 4.16 (s, 3H, Me). |
| 1.68 | 1 | F | F | Me | ^{c}Pr | | 0.96 (m, 2H, CH₂), 1.O2 (m, 2H, CH₂),1.49 (s, 3H, Me), 1.49 (s, 3H, Me), 2.01 (m, 1H, CH), 3.02 (d, 1H, CH₂), 3.24 (d, 1H, CH₂),4.12 (s, 3H, Me). |

| | | | | | | | |
|---|---|---|---|---|---|---|---|
| Key: Me = methyl; Et = ethyl; ⁱPr = *iso-*propyl; ^{c}Pr = cyclopropyl; ^{c}Bu = cyclobutyl; ^{c}Pe = cyclopentyl; s = singlet; d = doublet; t = triplet; q = quartet; dd = double doublet; dq = double quartet; sept = septet; m = multiplet. | | | | | | | |

**Table 2: Compounds of formula I.2**

| | | | | | | | |
|---|---|---|---|---|---|---|---|
| 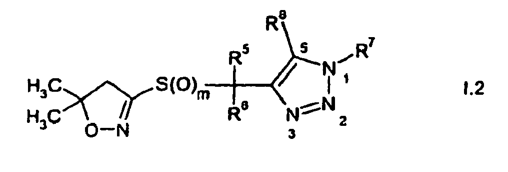 | | | | | | | |

| **No.** | **m** | **R⁵** | **R⁶** | **R⁷** | **R⁸** | **M.p. [°C]** | **¹H-NMR (400 MHz, CDCl₃)** |
|---|---|---|---|---|---|---|---|
| 2.02 | 2 | F | F | ^{t}Bu | H | | 1.59 (s, 6H, Me), 1.76 (s, 9H, Me), 3.23 (s, 2H, CH₂), 8.11 (s, 1H, CH). |
| 2.04 | 1 | F | F | ^{t}Bu | H | | 1.48 (s, 3H; Me), 1.51 (s, 3H, Me), 1.72 (s, 9H, Me), 2.97 (d, 1H, CH₂), 3.12 (d, 1H, CH₂), 7.98 (s, 1H, CH). |
| 2.06 | 2 | F | F | Me | Me | | 1.56 (s, 6H, Me), 2.49 (t, 3H, Me), 3.19 (s, 2H, CH₂), 4.03 (s, 3H, Me). |
| 2.07 | 1 | F | F | Me | Me | | 1.48 (s, 3H, Me), 1.52 (s, 3H, Me), 2.46 (t, 3H, Me), 3.02 (d, 1H, CH₂), 3.13 (d, 1H, CH₂), 4.01 (s, 3H, Me). |
| 2.08 | 2 | F | F | Me | ^{c}Pr | | 0.97 (m, 2H, CH₂), 1.17 (m, 2H, CH₂), 1.57 (s, 6H, Me), 1.78 (m, 1H, CH), 3.22 (s, 2H, CH₂), 4.11 (s, 3H, Me). |
| 2.10 | 1 | F | F | Me | ^{c}Pr | | 0.91 (m, 2H, CH₂), 1.18 (m, 2H, CH₂),1.48 (s, 3H, Me), 1.50 (s, 3H, Me), 1.74 (m, 1H, CH), 3.13 (d, 1H, CH₂), 3.20 (d, 1H, CH₂), 4.10 (s, 3 H, Me). |

| | | | | | | | |
|---|---|---|---|---|---|---|---|
| Key: Me = methyl; ^{c}Pr = cyclopropyl; ^{t}Bu = *tert-*butyl; s = singlet; d = doublet; t = triplet; m = multiplet. | | | | | | | |

**Table 3: Compounds of formula I.3**

| | | | | | | | |
|---|---|---|---|---|---|---|---|
| 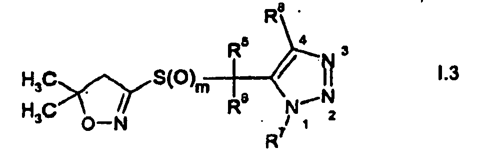 | | | | | | | |

| **No.** | **m** | **R⁵** | **R⁶** | **R⁷** | **R⁸** | **M.p. [°C]** | **¹H-NMR (400 MBz, CDCl₃)** |
|---|---|---|---|---|---|---|---|
| 3.02 | 2 | F | F | Me | -CF₃ | | 1.58 (s, 6H, Me), 3.18 (s, 2H, CH₂), 4.39 (s, 3H, Me). |
| 3.03 | 1 | F | F | Me | -CF₃ | | 1.5 (s, 3H, Me), 1.51 (s, 3H, Me), 2.9 (d, 1H, CH₂), 3.2 (d, 1H, CH₂), 4.35 (s, 3H. Me). |
| 3.04 | 2 | F | F | Me | Br | 91-93 | 1.58 (s, 6H, Me), 3.17 (s, 2H, CH₂), 4.32 (t, 3H, Me). |
| 3.06 | 1 | F | F | Me | Br | 70-72 | 1.49 (s, 3H, Me), 1.53 (s, 3H, Me), 2.76 (d, 1H, CH₂), 3.14 (d, 1H, CH₂), 4.29 (t, 3H, Me). |
| 3.07 | 2 | F | F | ¹Pr | Me | | 1.57 (s, 6H, Me), 1.65 (d, 6H, Me), 2.49 (s, 3H, Me), 3.15 (s, 2H, CH₂), 4.92 (sept, 1H, CH). |
| 3.08 | 2 | F | F | Me | ^{c}Pr | | 1.03 (m, 2H, CH₂), 1.17 (m, 2H, CH₂), 1.57 (s, 6H, Me), 2.05 (m, 1H, CH), 3.15 (s, 2H, CH₂), 4.21 (s, 3H, Me). |
| 3.10 | 1 | F | F | Me | ^{c}Pr | | 1.00-1.09 (m, 3H, CH₂), 1.25 (m, 1H, CH₂), 1.48 (s, 3H, Me), 1.49 (s, 3H, Me), 1.95 (m, 1H, CH), 2.64 (dd, 1H, CH₂), 3.14 (d, 1H, CH₂), 4.19 (t, 3H, Me). |

| | | | | | | | |
|---|---|---|---|---|---|---|---|
| Key: Me = methyl; ⁱPr = *iso-*propyl; ^{c}Pr = cyclopropyl; s = singlet; d = doublet; t = triplet; dd = double douplet; m = multiplet. | | | | | | | |

**Table 4: Compounds of formula I.4**

| | | | | | | | |
|---|---|---|---|---|---|---|---|
| 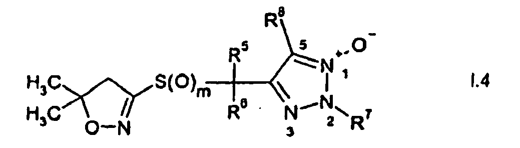 | | | | | | | |

| **No.** | **m** | **R⁵** | **R⁶** | **R⁷** | **R⁸** | **M.p. [°C]** | **¹H-NMR (400 MHz, CDCl₃)** |
|---|---|---|---|---|---|---|---|
| 4.01 | 2 | F | F | Me | Me | 73-75 | 1.57 (s, 6H, Me), 2.42 (s, 3H, Me), 3.24 (s, 2H, CH₂), 3.93 (s, 3H, Me). |

| | | | | | | | |
|---|---|---|---|---|---|---|---|
| Key: Me = methyl; s = singlet. | | | | | | | |

### Biological Examples

### Example B1: Herbicidal action

Monocotyledonous and dicotyledonous test plants were sown in sterilised standard soil in seed trays each having 96 cells. After one day (pre-emergence) or after 8 to 9 days cultivation (post-emergence) under controlled conditions in a climatic chamber (cultivation at 23/17°C, daylight ; 13 hours light; 50-60% humidity; after application at 24/19°C, day/night), the plants were treated with an aqueous spray solution of 1000 mg/l of the active ingredient used (incl. 10% DMSO as solvent). The plants were grown on in the climatic chamber until the test was evaluated (10 = total damage to plant, 0 = no damage to plant) after 9 or 13 days.

**Table B1a: Application pre-emergence**

| Comp | Rate | DIGSA | AGSTE | SETIT | POATR | AMARE | SETFA |
|---|---|---|---|---|---|---|---|
| 1.01 | 1000 | 10 | 10 | 10 | 10 | 10 | - |
| 1.03 | 1000 | 10 | 10 | 9 | 10 | 8 | - |
| 1.05 | 1000 | 10 | 10 | 10 | 10 | 10 | - |
| 1.07 | 1000 | 10 | 10 | 9 | 10 | 9 | - |
| 1.08 | 1000 | 10 | 10 | - | 10 | 10 | 9 |
| 1.09 | 1000 | 10 | 10 | 10 | 10 | 10 | - |
| 1.10 | 1000 | 10 | 10 | 10 | 10 | 10 | - |
| 1.15 | 1000 | 10 | 10 | - | 10 | 10 | 10 |
| 1.16 | 1000 | 10 | 10 | - | 10 | 10 | 9 |
| 1.17 | 1000 | 9 | 10 | - | 10 | 10 | 10 |
| 1.18 | 1000 | 10 | 10 | - | 10 | 10 | 9 |
| 1.20 | 1000 | 10 | 10 | - | 10 | 10 | 9 |
| 1.22 | 1000 | 10 | 10 | - | 10 | 10 | 9 |
| 1.23 | 1000 | 10 | 10 | - | 10 | 10 | 10 |
| 1.25 | 1000 | 10 | 10 | - | 10 | 10 | 9 |
| 1.26 | 1000 | 10 | 10 | - | 10 | 10 | 10 |
| 1.27 | 1000 | 10 | 10 | - | 10 | 10 | 9 |
| 1.29 | 1000 | 9 | 10 | - | 6 | 8 | 9 |
| 1.31 | 1000 | 9 | 10 | - | 7 | 0 | 9 |
| 1.32 | 1000 | 10 | 10 | - | 10 | 10 | 10 |
| 1.34 | 1000 | 10 | 10 | - | 10 | 9 | 10 |
| 1.35 | 1000 | 9 | 10 | - | 10 | 10 | 9 |
| 1.36 | 1000 | 10 | 10 | - | 10 | 9 | 9 |
| 1.38 | 1000 | 9 | 10 | - | 10 | 10 | 9 |
| 1.40 | 1000 | 9 | 10 | - | 10 | 10 | 9 |
| 1.41 | 1000 | 10 | 10 | - | 10 | 10 | 9 |
| 1.43 | 1000 | 10 | 10 | - | 0 | 10 | 10 |
| 1.44 | 1000 | 10 | 10 | - | 0 | 10 | 9 |
| 1.46 | 1000 | 10 | 10 | - | 10 | 10 | 9 |
| 1.47 | 1000 | 9 | 10 | - | 10 | 10 | 9 |
| 1.48 | 1000 | 10 | 10 | - | 6 | 9 | 9 |
| 1.49 | 1000 | 10 | 10 | - | 10 | 9 | 9 |
| 2.01 | 1000 | 9 | 10 | - | 0 | 3 | 9 |
| 2.02 | 1000 | 9 | 9 | - | 9 | 3 | 8 |
| 2.03 | 1000 | 9 | 8 | - | 4 | 2 | 5 |
| 2.04 | 1000 | 2 | 6 | - | 0 | 0 | 6 |
| 3.02 | 1000 | 10 | 10 | 10 | 10 | 10 | - |
| 3.03 | 1000 | 9 | 10 | - | 10 | 10 | 8 |
| 3.04 | 1000 | 9 | 10 | - | 10 | 10 | 9 |
| 3.06 | 1000 | 9 | 10 | - | 10 | 10 | 9 |

| | | | | | | | |
|---|---|---|---|---|---|---|---|
| DIGSA = Digitaria sanguinalis; AGRTE = Agrostis tenius; SETIT = Setaria italica; SETFA = Setaria faberi; POATR = Poa trivialis; AMARE = Amaranthus retroflexus. | | | | | | | |

## Claims

1. A compound of formula 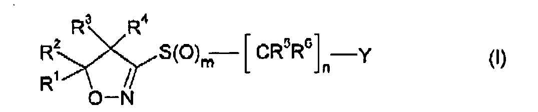 wherein
R¹ and R² are each independently of the other hydrogen, C₁-C₁₀alkyl, C₁-C₁₀haloalkyl, C₃-C₈cycloalkyl or C₃-C₈cycloalkyl-C₁-C₃alkyl, or
R¹ and R² together with the carbon atom to which they are bonded form a C₃-C₇ring,
R³ and R⁴ are each independently of the other hydrogen, C₁-C₁₀alkyl, C₁-C₁₀haloalkyl, C₃-C₈cycloalkyl, C₃-C₈cycloalkyl-C₁-C₁₀alkyl or C₁-C₆alkoxy-C₁-C₁₀alkyl, or
R³ and R⁴ together with the carbon atom to which they are bonded form a C₃-C₇ring, or
R¹ with R³ or R⁴ and together with the carbon atoms to which they are bonded form a C₅-C₈ring, or
R² with R³ or R⁴ and together with the carbon atoms to which they are bonded form a C₅-C₈ring;
R⁵ is halogen;
R⁶ is halogen;
m is 0, 1 or 2;
n is 1,2 or 3; and
Y is one of the following groups 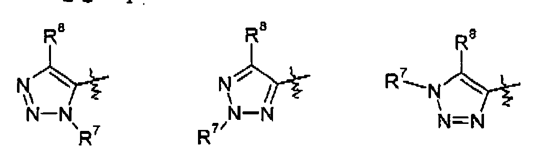 wherein
R⁷ is hydrogen, C₁-C₁₀alkyl, C₁-C₁₀alkylcarbonyl, formyl, C₁-C₁₀haloalkylcaxbonyl, C₁-C₁₀alkoxycarbonyl, C₁-C₁₀haloalkyl, cyano, C₂-C₁₀alkenyl, C₁-C₁₀alkynyl, C₃-C₁₀cycloalkyl, C₃-C10cycloalkyl-C₁-C10alkyl, C₁-C₁₀alkylcarbonyl-C₁-C₁₀alkyl. C₁-C₁₀alkylsulfonyl, C₁-C₁₀haloalkylsulfonyl, C₁-C₁₀alkoxy-C₁-C₁₀alkyl or phenyl which is optionally substituted by one to five substituents selected from cyano, C₁-C₁₀alkyl, C₁-C₁₀haloalkyl or halogen; or
R⁷ is -CONR¹³R¹⁴ or -SO₂NR¹³R¹⁴ wherein R¹³ and R¹⁴ are independently of each other hydrogen, C₁₋C₆alkyl, C₁-C₆haloalkyl, C₁-C₆cycloalkyl, C₁-C₆alkylcarbonyl, C₁-C₆halo-alkylcarbonyl, C₁-C₆alkylsulfonyl, C₁-C₆haloalkylsulfonyl, or R¹³ and R¹⁴ together form a C₃-C₈alkylene group which optionally contains one oxygen, sulfur, amino or C₁-C₆alkylamino group;
R⁸ is hydrogen, C₁-C₁₀alkyl, C₁-C₁₀alkylcarbonyl, formyl, C₁-C₁₀haloalkylcarbonyl, C₁-C₁₀alkoxycarbonyl, C₁-C₁₀haloalkyl, C₂-C₁₀alkenyl, C₁-C₁₀alkynyl, C₃-C10cycloalkyl, C₃-C₁₀cycloalkyl-C₁-C₁₀alkyl, halogen, cyano, C₁-C₁₀alkoxy or C₁-C₁₀haloalkoxy, or
R⁸ is -CONR¹⁵R¹⁶, -SO₂NR¹⁵R¹⁶ or -NR¹⁵R¹⁶ wherein R¹⁵ and R¹⁶ are independently of each other hydrogen, C₁-C₆alkyl, C₁-C₆haloalkyl, C₁-C₆cycloalkyl, C₁-C₆alkylcarbonyl, C₁-C₆haloalkylcarbonyl, C₁-C₆alkylsulfonyl, C₁-C₆haloalkylsulfonyl, or R¹⁵ and R¹⁶ together form a C₃-C₈alkylene group which optionally contains one oxygen, sulfur, amino or C₁-C₆alkylamino group;
and to N-oxides, salts and optical isomers of compounds of formula I.

2. A compound according to claim 1 in which R¹ and R² are independently C₁-C₁₀alkyl or C₁-C₁₀haloalkyl.

3. A compound according to claim 1 or claim 2 in which R¹ and R² are both methyl.

4. A compound according to any one of claims 1 to 3 in which R³ and R⁴ are independently hydrogen, C₁-C₁₀alkyl or C₁-C₁₀haloalkyl.

5. A compound according to any one of claims 1 to 4 in which R³ and R⁴ are both hydrogen.

6. A compound according to any one of claims 1 to 5 in which R⁵ is fluoro.

7. A compound according to any one of claims 1 to 6 in which R⁶ is fluoro.

8. A compound according to any one of claims 1 to 7 in which m is 1 or 2.

9. A compound according to any one of claims 1 to 8 in which m is 2.

10. A compound according to any one of claims 1 to 9 in which n is 1.

11. A compound according to any one of claims 1 to 10 in which Y is one of the following groups

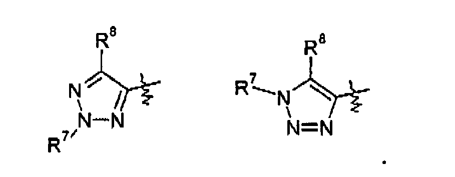

12. A compound according to any one of claims 1 to 11 in which R⁷ is hydrogen, C₁-C₁₀alkyl, C₁-C₁₀haloalkyl, C₂-C₁₀alkenyl, C₂-C₁₀alkynyl, C₃-C₁₀cycloalkyl, C₃-C₁₀cycloalkyl-C₁-C₁₀alkyl, C₁-C₁₀alkoxy-C₁-C₁₀alkyl or phenyl which is optionally substituted by one to five substituents selected from cyano, C₁-C₁₀alkyl, C₁-C₁₀haloalkyl or halogen.

13. A compound according to any one of claims 1 to 12 in which R⁷ is hydrogen, methyl, ethyl, *iso*-propyl, *tert*-butyl, monofluoromethyl, difluoromethyl, trifluoromethyl, allyl, propargyl, cyclopropyl, cyclopentyl, cyclopropylrnethyl, cyclobutylmethyl, methoxymethyl, 2-methoxy-ethyl or phenyl.

14. A compound according to any one of claims 1 to 13 in which R⁸ is hydrogen, C₁-C₁₀alkyl, C₁-C₁₀alkylcarbonyl, formyl, C₁-C₁₀alkoxycarbonyl, C₁-C₁₀haloalkyl, C₃-C₁₀cycloalkyl, halogen, cyano, C₁-C₁₀alkoxy or C₁-C₁₀haloalkoxy.

15. A compound according to any one of claims 1 to 14 in which R⁸ is hydrogen, methyl, ethyl, acetyl, formyl, methoxycarbonyl, monofluoromethyl, difluoromethyl, trifluoromethyl, bromodifluoromethyl, 1-fluoro-ethyl, cyclopropyl, fluoro, chloro, bromo, cyano, methoxy, difluoromethoxy, trifluoromethoxy or 2,2,2-trifluoroethoxy.

16. A process in which a compound of formula (Id) is formed 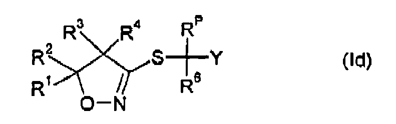 by reacting a compound of formula VIII wherein R⁶ and Y are as defined in claim 1, R^{P} is hydrogen or halogen and M^{R} is selected from the group comprising MgCl, MgBr, ZnBr and Li, 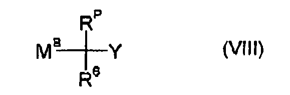 with a compound of formula IX, 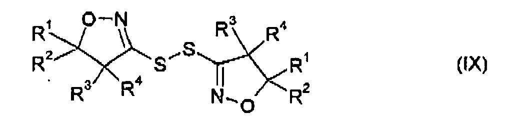 wherein R¹, R², R³ and R⁴ are as defined in claim 1, optionally in the presence of a diluent.

17. A process in which a compound of formula (Id) is formed 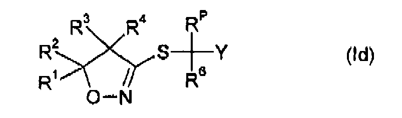 by reacting a compound of formula IIa wherein R⁶ and Y are as defined in claims 1, R^{P} is hydrogen or halogen and X^{c} is functional group that may be cleaved as a radical, 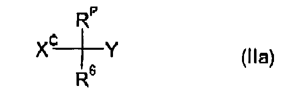 with a compound of formula IX, 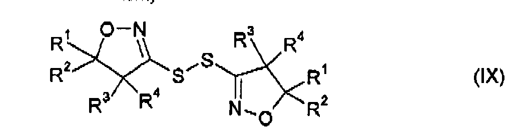 wherein R¹, R², ,R³ and R⁴ are as defined in claim 1 with a radical initiator or a precursor thereof, optionally in the presence of a base and optionally in the presence of a diluent.

18. A process in which a compound of formula (Id) is formed 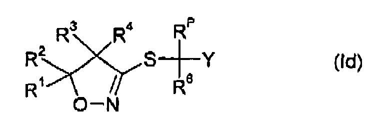 by reacting a compound of formula II wherein R⁶ and Y are as defined in claim 1, R^{p} is hydrogen or halogen, and X^{A} is a leaving group selected from the group comprising halogen, an alkylsulfonate, an arylsulfonate, or a haloalkylsulfonate, 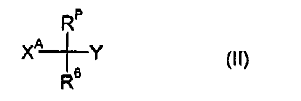 with a compound of formula IX 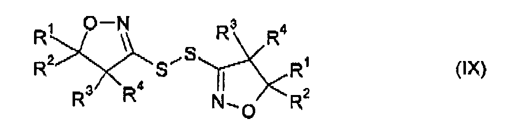 wherein R¹, R², R³ and R⁴ are as defined in claim 1 with in the presence of a reducing agent, optionally in the presence of a base and optionally in the presence of a diluent.

19. A compound of formula IX 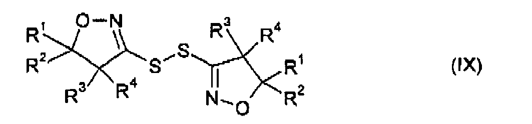 wherein R¹, R², R³ and R⁴ are as defined in claim 1.

20. A herbicidal composition which comprises a herbicidally effective amount of a compound of formula I as defined in any one of claims 1 to 15 in addition to formulation adjuvants.

21. A method of controlling plants which comprises applying a herbicidally effective amount of a compound of formula I as defined in any one of claims 1 to 15, or of a composition comprising such a compound, to the plants or to the locus thereof.

22. A composition according to claim 20, which comprises a further herbicide in addition to the compound of formula I.

23. A composition according to claim 20, which comprises a safener in addition to the compound or formula I.

## Patentansprüche

1. Verbindung der Formel 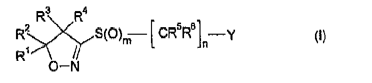 worin
R¹ und R² jeweils unabhängig vom anderen Wasserstoff, C₁-C₁₀-Alkyl, C₁-C₁₀-Haloalkyl, C₃-C₈-Cycloalkyl oder C₃-C₈-Cycloalkyl-C₁-C₃-Alkyl sind oder
R¹ und R² zusammen mit dem Kohlenstoffatom, an das sie gebunden sind, einen C₃-C₇-Ring bilden,
R³ und R⁴ jeweils unabhängig vom anderen Wasserstoff, C₁-C₁₀-Alkyl, C₁-C₁₀-Haloalkyl, C₃-C₈-Cycloalkyl, C₃-C₈-Cycloalkyl-C₁-C₁₀-Alkyl oder C₁-C₆-Alkoxy-C₁-C₁₀-Alkyl sind oder
R³ und R⁴ zusammen mit dem Kohlenstoffatom, an das sie gebunden sind, einen C₃-C₇-Ring bilden oder
R¹ mit R³ oder R⁴ und zusammen mit den Kohlenstoffatomen, an die sie gebunden sind, einen C₅-C₈-Ring bilden, oder
R² mit R³ oder R⁴ und zusammen mit den Kohlenstoffatomen, an die sie gebunden sind, einen C₅-C₈-Ring bilden;
R⁵ Halogen ist;
R⁶ Halogen ist;
m 0, 1 oder 2 ist;
n 1, 2 oder 3 ist; und
Y eine der folgenden Gruppen ist: 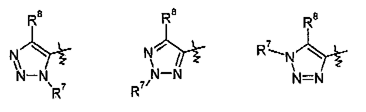 worin
R⁷ Wasserstoff, C₁-C₁₀-Alkyl, C₁-C₁₀-Alkylcarbonyl, Formyl, C₁-C₁₀-Haloalkylcarbonyl, C₁-C₁₀-Alkoxycarbonyl, C₁-C₁₀-Haloalkyl, Cyano, C₂-C₁₀-Alkenyl, C₂₋C₁₀-Alkinyl, C₃,C₁₀-Cycloalkyl, C₃-C₁₀-Cycloalkyl-C₁-C₁₀-Alkyl, C₁-C₁₀-Alkylcarbonyl-C₁-C₁₀-Alkyl, C₁-C₁₀-Alkylsulfonyl, C₁-C₁₀-Haloalkylsulfonyl, C₁-C₁₀-Alkyloxy-C₁-C₁₀-Alkyl oder Phenyl, das wahlweise substituiert ist mit ein bis fünf Substituenten, ausgewählt aus Cyano, C₁-C₁₀-Alkyl, C₁-C₁₀-Haloalkyl oder Halogen, ist; oder
R⁷ -CONR¹³R¹⁴ oder -SO₂NR¹³R¹⁴ ist, worin R¹³ und R¹⁴ unabhängig voneinander Wasserstoff, C₁-C₆-Alkyl, C₁-C₆-Haloalkyl, C₁-C₆-Cycloalkyl, C₁-C₆-Alkylcarbonyl, C₁-C₆-Haloalkylcarbonyl, C₁-C₆-Alkylsulfonyl oder C₁-C₆-Haloalkylsulfonyl sind oder R¹³ und R¹⁴ zusammen eine C₃-C₈-Alkylengruppe bilden, die wahlweise eine Sauerstoff-, Schwefel-, Amino- oder C₁-C₆-Alkylaminogruppe enthält;
R⁸ Wasserstoff, C₁-C₁₀-Alkyl. C₁-C₁₀-Alkylcarbonyl, Formyl, C₁-C₁₀-Haloalkylcarbonyl, C₁-C₁₀-Alkoxycarbonyl, C₁-C₁₀-Haloalkyl, C₂-C₁₀-Alkenyl, C₁-C₁₀-Alkinyl, C₁-C₁₀-Cycloalkyl, C₁-C₁₀-Cycloalkyl-C₁-C₁₀-Alkyl, Halogen, Cyano, C₁-C₁₀-Alkyloxy oder C₁-C₁₀-Haloalkoxy ist oder
R⁸ -CONR¹⁵R¹⁶, -SO₂NR¹⁵R¹⁶ oder -NR¹⁵R¹⁶ ist, worin R¹⁵ und R¹⁶ unabhängig voneinander Wasserstoff C₁-C₆-Alkyl, C₁-C₆-Haloalkyl, C₁-C₆-Cycloalkyl, C₁-C₆-Alkylcarbonyl, C₁-C₆-Haloalkylcarbonyl, C₁-C₆-Alkylsulfonyl, oder C₁-C₆-Haloalkylsulfonyl sind oder R¹⁵ und R¹⁶ zusammen eine C₃-C₈-Alkylengruppe bilden, die wahlweise eine Sauerstoff-, Schwefel-, Amino- oder C₁-C₆-Alkylaminogruppe enthält;
und die N-Oxide, Salze und optischen Isomere der Verbindungen der Formel I.

2. Verbindung gemäß Anspruch 1, worin R¹ und R² unabhängig C₁-C₁₀-Alkyl oder C₁-C₁₀-Haloalkyl sind.

3. Verbindung gemäß Anspruch 1 oder Anspruch 2, worin R¹ und R² beide Methyl sind.

4. Verbindung gemäß einem der Ansprüche 1 bis 3, worin R³ und R⁴ unabhängig Wasserstoff, C₁-C₁₀-Alkyl oder C₁-C₁₀-Haloalkyl sind.

5. Verbindung gemäß einem der Ansprüche 1 bis 4, worin R³ und R⁴ beide Wasserstoff sind.

6. Verbindung gemäß einem der Ansprüche 1 bis 5, worin R⁵ Fluor ist.

7. Verbindung gemäß einem der Ansprüche 1 bis 6, worin R⁶ Fluor ist.

8. Verbindung gemäß einem der Ansprüche 1 bis 7, worin m 1 oder 2 ist.

9. Verbindung gemäß einem der Ansprüche 1 bis 8, worin m 2 ist.

10. Verbindung gemäß einem der Ansprüche 1 bis 9, worin n 1 ist.

11. Verbindung gemäß einem der Ansprüche 1 bis 10, worin Y eine der folgenden Gruppe ist:

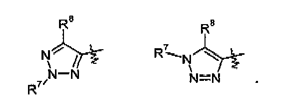

12. Verbindung gemäß einem der Ansprüche 1 bis 11, worin R⁷ Wasserstoff, C₁-C₁₀-Alkyl, C₁-C₁₀-Haloalkyl, C₁-C₁₀-Alkenyl, C₂-C₁₀-Alkinyl, C₃-C₁₀-Cycloalkyl, C₃-C₁₀-Cycloalkyl-C₁-C₁₀-alkyl, C₁-C₁₀-Alkoxy-C₁-C₁₀-alkyl oder Phenyl, das wahlweise substituiert ist mit ein bis fünf Substituenten, ausgewählt aus Cyano, C₁-C₁₀-Alkyl, C₁-C₁₀-Haloalkyl oder Halogen, ist.

13. Verbindung gemäß einem der Ansprüche 1 bis 12, worin R⁷ Wasserstoff, Methyl, Ethyl, iso-Propyl, tert-Butyl, Monofluormethyl, Difluormethyl, Trifluormethyl, Allyl, Propargyl, Cyclopropyl, Cyclopentyl, Cyclopropylmethyl, Cyclobutylmethyl, Methoxymethyl, 2-Methoxyethyl oder Phenyl ist.

14. Verbindung gemäß einem der Ansprüche 1 bis 13, worin R⁸ Wasserstoff, C₁-C₁₀-Alkyl, C₁-C₁₀-Alkylcarbonyl, Formyl, C₁-C₁₀-Alkoxycarbonyl, C₁-C₁₀-Haloalkyl, C₃-C₁₀-Cycloalkyl, Halogen, Cyano, C₁-C₁₀-Alkoxy oder C₁-C₁₀-Haloalkoxy ist.

15. Verbindung gemäß einem der Ansprüche 1 bis 14, worin R⁸ Wasserstoff, Methyl, Ethyl, Acetyl, Formyl, Methoxycarbonyl. Monofluormethyl, Difluormethyl, Trifluormethyl, Bromdifluormethyl, 1-Fluorethyl, cyclopropyl, Fluor, Chlor, Brom, Cyano, Methoxy, Difluormethoxy, Trifluormethoxy oder 2,2,2-Trifluorothoxy ist.

16. Verfahren, worin eine Verbindung der Formel (Id) 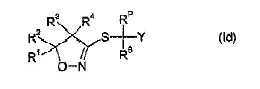 gebildet wird durch Umsetzung einer Verbindung der Formel VIII, worin R⁶ und Y wie in Anspruch 1 definiert sind, R^{P} Wasserstoff oder Halogen ist und M^{B} ausgewählt ist aus der Gruppe, umfassend MgCl, MgBr, ZnBr und Li, 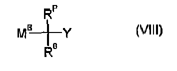 mit einer Verbindung der Formel IX 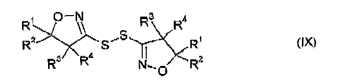 worin R¹, R², R³ und R⁴ wie in Anspruch 1 definiert sind, wahlweise in Gegenwart eines Verdünnungsmittels.

17. Verfahren, worin eine Verbindung der Formel (Id) 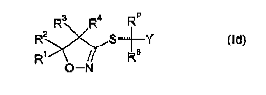 gebildet wird durch Umsetzung einer Verbindung der Formel IIa, worin R⁶ und Y wie in Anspruch 1 definiert sind, R^{P} Wasserstoff oder Halogen ist und X^{C} eine funktionale Gruppe ist, die als Radikal gespalten werden kann, 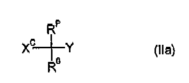 mit einer Verbindung der Formel IX 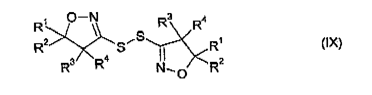 worin R¹, R², R³ und R⁴ wie in Anspruch 1 definiert sind, mit einem Radikalstarter oder einem Vorläufer davon, wahlweise in Gegenwart einer Base und wahlweise in Gegenwart eines Verdünnungsmittels.

18. Verfahren, worin eine Verbindung der Formel (Id) 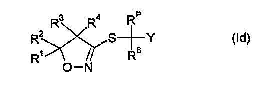 gebildet wird durch Umsetzung einer Verbindung der Formel 11, worin R⁶ und Y wie in Anspruch 1 definiert sind, R^{P} Wasserstoff oder Halogen ist und X^{A} eine Abgangsgruppe ist, auswählt aus der Gruppe umfassend Halogen, ein Alkylsulfonat, ein Arylsulfonat oder ein Haloalkylsulfonat, 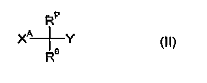 mit einer Verbindung der Formel IX 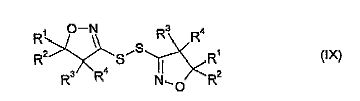 worin R¹, R², R³ und R⁴ wie in Anspruch 1 definiert sind, in Gegenwart eines Reduktionsmittels, wahlweise in Gegenwart einer Base und wahlweise in Gegenwart eines Verdünnungsmittels.

19. Verbindung der Formel IX 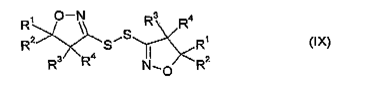 worin R¹, R², R³ und R⁴ wie in Anspruch 1 definiert sind.

20. Herbizidzusammensetzung, die zusätzlich zu den Formulierungshilfsstoffen eine herbizid wirksame Menge einer wie in einem der Ansprüche 1 bis 15 definierten Verbindung der Formel I umfasst.

21. Verfahren zur Überwachung von Pflanzen, das die Anwendung einer herbizid wirksamen Menge einer wie in einem der Ansprüche 1 bis 15 definierten Verbindung der Formel I oder einer zusammensetzung, umfassend solch eine Verbindung, an den Pflanzen oder an deren Standort umfasst.

22. Zusammensetzung gemäß Anspruch 20, die zusätzlich zu der Verbindung der Formel I ein weiteres Herbizid umfasst.

23. Zusammensetzung gemäß Anspruch 20, die zusätzlich zu der Verbindung der Formel I einen Safener umfasst.

## Revendications

1. Composé de la formule 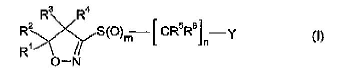 dans laquelle
R¹ et R² sont chacun Indépendamment l'hydrogène, un groupe alkyle en C₁-C₁₀, haloalkyle en C₁-C₁₀, cycloalkyle en C₃-C₈ ou (cycloalkyle en C₃-C₈)-(alkyle en C₁-C₃), ou
R¹ et R², avec l'atome de carbone auquel ils sont liés forment un cycle en C₃-C₇,
R³ et R⁴ sont chacun indépendamment l'hydrogène, un groupe alkyle en C₁-C₁₀, haloalkyle en C₁-C₁₀, cycloalkyle en C₃-C₈, (cycloalkyle en C₃-C₈)-(alkyle en C₁-C₁₀) ou (alkoxy en C₁-C₆)-(alkyle en C₁-C₁₀), ou
R³ et R⁴ avec l'atome de carbone auquel ils sont fixés forment un cycle en C₃-C₇, ou
R¹ avec R³ ou R⁴ et avec les atomes de carbone auxquels ils sont liés forment un cycle en C₅-C₈, ou
R² avec R³ ou R⁴ et avec les atomes de carbone auxquels ils sont liés forment un cycle en C₅-C₈;
R⁵ est un halogène;
R⁶ est un halogène;
m est égal à 0, 1 ou 2;
n est égal à 1, 2 ou 3; et
Y est un des groupes suivants 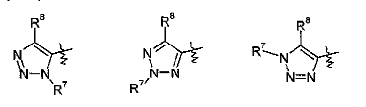 dans lesquels
R⁷ est l'hydrogène, un groupe alkyle en C₁-C₁₀, alkylcarbonyle en C₁-C₁₀, formyle, haloalkylcarbonyle en C₁-C₁₀, alkoxycarbonyle en C₁-C₁₀, haloalkyle en C₁-C₁₀, cyano, alcényle en C₂-C₁₀, alcynyle en C₂-C₁₀, cycloalkyle en C₃-C₁₀, (cycloalkyle en C₃-C₁₀)-(alkyle en C₁-C₁₀), (alkylcarbonyle en C₁-C₁₀)-(alkyle en C₁-C₁₀), alkylsulfonyle en C₁-C₁₀, haloalkylsulfonyle en C₁-C₁₀, (alkoxy en C₁-C₁₀)-(alkyle en C₁-C₁₀) ou phényle qui est éventuellement substitué par de un à cinq substituants choisis parmi un groupe cyano, alkyle en C₁-C₁₀, haloalkyle en C₁-C₁₀ ou un halogéné; ou
R⁷ est -CONR¹³R¹⁴ ou -SO₂NR¹³R¹⁴, dans lesquels R¹³ et R¹⁴ sont indépendamment l'hydrogène, un groupe alkyle en C₁-C₆, haloalkyle en C₁-C₆, cycloalkyle en C₁-C₆, alkylcarbonyle en C₁-C₆, haloalkylcarbonyle en C₁-C₆, alkylsulfonyle en C₁-C₆, haloalkylsulfonyle en C₁-C₆, ou R¹³ et R¹⁴ forment ensemble un groupe alkylène en C₃-C₈ qui contient éventuellement un parmi l'oxygène, le soufre, un groupe amino ou alkylamino en C₁-C₆;
R⁸ est l'hydrogène, un groupe alkyle en C₁-C₁₀, alkylcarbonyle en C₁-C₁₀, formyle, haloalkylcarbonyle en C₁-C₁₀, alkoxycarbonyle en C₁-C₁₀, haloalkyle en C₁-C₁₀, alcényle en C₂-C₁₀, alcynyle en C₂-C₁₀, cycloalkyle en C₃-C₁₀, (cycloalkyle en C₃-C₁₀)-(alkyle en C₁-C₁₀), un halogène, un groupe cyano, alkoxy en C₁-C₁₀ ou haloalkoxy en C₁-C₁₀, ou
R⁸ est -CONR¹⁵R¹⁶, -SO₂NR¹⁵R¹⁶ ou -NR¹⁵R¹⁶, dans lesquels R¹⁵ et R¹⁶ are indépendamment l'hydrogène, un groupe alkyle en C₁-C₆, haloalkyle en C₁-C₆, cycloalkyle en C₁-C₆, alkylcarbonyle en C₁-C₆, haloalkylcarbonyle en C₁-C₆, alkylsulfonyle en C₁-C₆, haloalkylsulfonyle en C₁-C₆, ou R¹⁵ et R¹⁶ forment ensemble un groupe alkylène en C₃-C₈ qui contient éventuellement un atome d'oxygène, de soufre, un groupe amino ou alkylamino en C₁-C₆;
et les N-oxydes, sels et isomères optiques des composés de la formule I.

2. Composé selon la revendication 1, dans lequel R¹ et R² sont indépendamment un groupe alkyle en C₁-C₁₀ ou haloalkyle en C₁-C₁₀.

3. Composé selon la revendication 1 ou 2, dans lequel R¹ et R² sont tous deux le groupe méthyle.

4. Composé selon l'une quelconque des revendications 1 à 3, dans lequel R³ et R⁴ sont indépendamment l'hydrogène, un groupe alkyle en C₁-C₁₀ ou haloalkyle en C₁-C₁₀.

5. Composé selon l'une quelconque des revendications 1 à 4, dans lequel R³ et R⁴ sont tous deux l'hydrogène.

6. Composé selon l'une quelconque des revendications 1 à 5, dans lequel R⁵ est le groupe fluoro.

7. Composé selon l'une quelconque des revendications 1 à 6, dans lequel R⁶ est le groupe fluoro.

8. Composé selon l'une quelconque des revendications 1 à 7, dans lequel m est égal à 1 ou 2.

9. Composé selon l'une quelconque des revendications 1 à 8, dans lequel m est égal à 2.

10. Composé selon l'une quelconque des revendications 1 à 9, dans lequel n est égal à 1.

11. Composé selon l'une quelconque des revendications 1 à 10, dans lequel Y est un des groupes suivants

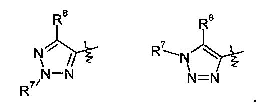

12. Composé selon l'une quelconque des revendications 1 à 11, dans lequel R⁷ est l'hydrogène, un groupe alkyle en C₁-C₁₀, haloalkyle en C₁-C₁₀, alcényle en C₂-C₁₀, alcynyle en C₂-C₁₀, cycloalkyle en C₃-C₁₀, (cycloalkyle en C₃-C₁₀)-(alkyle en C₁-C₁₀), (alkoxy en C₁-C₁₀)-(alkyle en C₁-C₁₀) ou phényle qui est éventuellement substitué par de un à cinq substituants choisis parmi un groupe cyano, alkyle en C₁-C₁₀, haloalkyle en C₁-C₁₀ ou un halogène.

13. Composé selon l'une quelconque des revendications 1 à 12, dans lequel R⁷ est l'hydrogène, le groupe méthyle, éthyle, *iso*-propyle, *tert-*butyle, monofluorométhyle, difluorométhyle, trifluorométhyle, allyle, propargyle, cyclopropyle, cyclopentyle, cyclopropylméthyle, cyclobutylméthyle, méthoxyméthyle, 2-méthoxyéthyle ou phényle.

14. Composé selon l'une quelconque des revendications 1 à 13, dans lequel R⁸ est l'hydrogène, un groupe alkyle en C₁-C₁₀, alkylcarbonyle en C₁-C₁₀, formyle, alkoxycarbonyle en C₁-C₁₀, haloalkyle en C₁-C₁₀, cycloalkyle en C₃-C₁₀, un halogène, un groupe cyano, alkoxy en C₁-C₁₀ ou haloalkoxy en C₁-C₁₀.

15. Composé selon l'une quelconque des revendications 1 à 14, dans lequel R⁸ est l'hydrogène, le groupe méthyle, éthyle, acétyle, formyle, méthoxycarbonyle, monofluorométhyle, difluorométhyle, trifluorométhyle, bromodifluorométhyle, 1-fluoro-éthyle, cyclopropyle, fluoro, chloro, bromo, cyano, méthoxy, difluorométhoxy, trifluorométhoxy ou 2,2,2-trifluoroéthoxy.

16. Procédé dans lequel est formé un composé de la formule (Id) 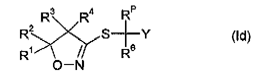 par réaction d'un composé de la formule VIII, dans laquelle R⁶ et Y sont comme définis dans la revendication, R^{p} est hydrogène ou un halogène et M^{B} est choisi dans le groupe comprenant MgCl, MgBr, ZnBr et Li, 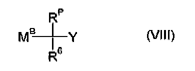 avec un composé de la formule IX, 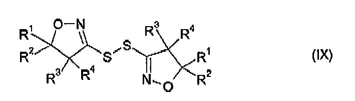 dans laquelle R¹, R², R³ et R⁴ sont comme définis dans la revendication 1, éventuellement en présence d'un diluant.

17. Procédé dans lequel est formé un composé de la formule (Id) 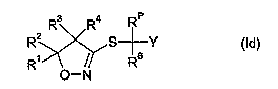 par réaction d'un composé de la formule IIa, dans laquelle R⁶ et Y sont comme définis dans la revendication 1, R^{p} est l'hydrogène ou un halogène et X^{c} est un groupe fonctionnel qui peut être dissocié comme un radical, 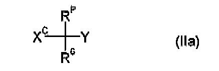 avec un composé de la formule IX, 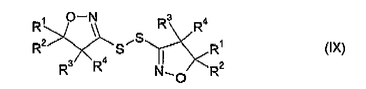 dans laquelle R¹, R², R³ et R⁴ sont comme définis dans la revendication 1 avec un initiateur de radical ou un précurseur de celui-ci, éventuellement en présence d'une base et éventuellement en présence d'un diluant.

18. Procédé dans lequel est formé un composé de la formule (Id) 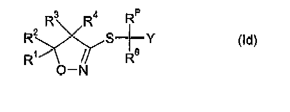 par réaction d'un composé de la formule II, dans laquelle R⁶ et Y sont comme définis dans la revendication 1, R^{p} est l'hydrogène ou un halogène, et X^{A} est un groupe qui s'éloigne choisi dans le groupe comprenant un halogène, un sulfonate d'alkyle, un sulfonate d'aryle, ou un sulfonate d'haloalkyle, 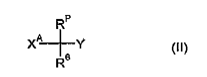 avec un composé de la formule IX 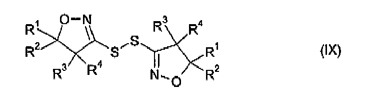 dans laquelle R¹, R², R³ et R⁴ sont comme définis dans la revendication 1 en présence d'un agent réducteur, éventuellement en présence d'une base et éventuellement en présence d'un diluant.

19. Composé de la formule IX 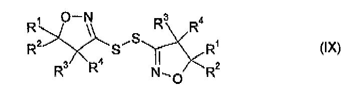 dans laquelle R¹, R², R³ et R⁴ sont comme définis dans la revendication 1.

20. Composition herbicide qui comprend une quantité efficace d'un point de vue herbicide d'un composé de la formule I comme défini dans l'une quelconque des revendications 1 à 15 en plus d'adjuvants de formulation.

21. Procédé de contrôle des plantes qui comprend l'application d'une quantité efficace d'un point de vue herbicide d'un composé de la formule I comme défini selon l'une quelconque des revendications 1 à 15, ou d'une composition comprenant un tel composé, aux plantes ou à leur locus.

22. Composition selon la revendication 20, laquelle comprend un autre herbicide en plus du composé de la formule I.

23. Composition selon la revendication 20, laquelle comprend un phytoprotecteur en plus du composé de la formule I.
